# EUROPÄISCHE PATENTSCHRIFT

(11) **EP 2 344 607 B1**
(45) Veröffentlichungstag und Bekanntmachung des Hinweises auf die Patenterteilung: **10.04.2013**
(21) Anmeldenummer: 09736571.2
(22) Anmeldetag: 15.10.2009
(51) Int. Cl.: C09K 11/06

(54) **ORGANISCHE ELEKTROLUMINESZENZVORRICHTUNGEN**
ORGANIC ELECTROLUMINESCENT DEVICES
DISPOSITIFS ÉLECTROLUMINESCENTS ORGANIQUES

(30) Priorität: 11.11.2008 DE 102008056688; 27.05.2009 DE 102009022858
(43) Veröffentlichungstag der Anmeldung: 20.07.2011
(73) Patentinhaber: Merck Patent GmbH, 64293 Darmstadt (DE)
(72) Erfinder: STOESSEL, Philipp, 60487 Frankfurt am Main (DE); HEIL, Holger, 60389 Frankfurt am Main (DE); JOOSTEN, Dominik, 60487 Frankfurt am Main (DE); PFLUMM, Christof, 60316 Frankfurt am Main (DE); GERHARD, Anja, 63329 Egelsbach (DE); BREUNING, Esther, 64372 Ober-Ramstadt (DE); PARHAM, Amir, Hossain, 65929 Frankfurt am Main (DE)
(86) Internationale Anmeldenummer: PCT/EP2009/007406
(87) Internationale Veröffentlichungsnummer: WO 2010/054730

(56) Entgegenhaltungen:
- JP-A- 2007 329 495
- CARLO ANCHISI: "Studies on the Synthesis of Heterocyclic Compounds" JOURNAL OF HETEROCYCLIC CHEMISTRY, Bd. 16, 1979, Seiten 1439-1441, XP002571058
- OGAWA SATOSHI ET AL: "Preparation and Multi-Nuclear NMR Study of New Benzodichalcogenaphospholes" HETEROCYCLES, ELSEVIER SCIENCE PUBLISHERS B.V. AMSTERDAM, NL, Bd. 41, Nr. 5, 1. Januar 1995 (1995-01-01) , Seiten 889-892, XP008119713 ISSN: 0385-5414
- ANISIMOVA ET AL: "Diaminophosphenium ions in the mass spectra of 2,3-dihydro-1H-1,3,2-benzodiazaphospholes" JOURNAL OF GENERAL CHEMISTRY USSR, CONSULTANTS BUREAU, NEW YORK, NY, US, Bd. 46, 1. Januar 1976 (1976-01-01), Seiten 807-811, XP008119714 ISSN: 0022-1279
- LISTER J H ET AL: "Synthesis and properties of 1,3-diaza-1,3-dihydro-2-phospholo[4,5- d]pyrimidine 2-oxides" JOURNAL OF THE CHEMICAL SOCIETY, SECTION C: ORGANIC CHEMISTRY, CHEMICAL SOCIETY. LETCHWORTH, GB, Nr. 14, 1. Januar 1966 (1966-01-01), Seiten 1242-1244, XP008119708 ISSN: 0022-4952

## Beschreibung

Die vorliegende Erfindung betrifft organische Elektrolumineszenzvorrichtungen sowie Materialien für die Verwendung in organischen Elektrolumineszenzvorrichtungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Steigerung der Energie- und Leistungseffizienz möglich. Generell gibt es bei OLEDs, insbesondere auch bei OLEDs, die Triplettemission zeigen, jedoch immer noch Verbesserungsbedarf, insbesndere im Hinblick auf Effizienz, Betriebsspannung und Lebensdauer. Dies gilt insbesondere für OLEDs, welche im kürzerwelligen Bereich, also grün und insbesondere blau, emittieren. So sind bislang keine Devices mit blau emittierenden Triplettemittern bekannt, welche die technischen Anforderungen für eine industrielle Anwendung erfüllen.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden unter anderem Ketone (z. B. gemäß WO 04/093207 oder gemäß der nicht offen gelegten Anmeldung DE 102008033943.1) oder Phosphinoxide (z. B. gemäß WO 05/003253) als Matrixmaterialien für phosphoreszierende Emitter verwendet.

Allerdings besteht bei Verwendung dieser Matrixmaterialien ebenso wie bei anderen Matrixmaterialien noch Verbesserungsbedarf, insbesondere in Bezug auf die Effizienz und die Lebensdauer der Vorrichtung.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Lochtransport-/Elektronenblockiermaterial bzw. Exzitonenblockiermaterial oder als Elektronentransport- bzw. Lochblockiermaterial. Insbesondere ist es die Aufgabe der vorliegenden Erfindung, Matrixmaterialien bereitzustellen, welche sich auch für blau und grün phosphoreszierende OLEDs eignen.

Überraschend wurde gefunden, dass bestimmte, unten näher beschriebene Verbindungen diese Aufgabe lösen und zu deutlichen Verbesserungen der organischen Elektrolumineszenzvorrichtung führen, insbesondere hinsichtlich der Lebensdauer, der Effizienz und der Betriebsspannung. Dies gilt insbesondere für blau und grün phosphoreszierende Elektrolumineszenzvorrichtungen, vor allem bei Einsatz der erfindungsgemäßen Verbindungen als Matrixmaterial. Organische Elektrolumineszenzvorrichtungen, welche derartige Verbindungen enthalten, sowie die entsprechenden Verbindungen sind daher der Gegenstand der vorliegenden Erfindung.

C. Anchisi et al. (Journal of Heterocyclic Chemistry 1979, 16, 1439-1441) offenbaren verschiedene cyclische Phosphor- und Arsenverbindungen, die jeweils ein trivalentes Phosphor- bzw. Arsenatom aufweisen. Entsprechende cyclische Verbindungen, die statt des trivalentes Phosphor- bzw. Arsenatoms ein Phosphinoxid bzw. Arsenoxid aufweisen, sind nicht offenbart. Ein Bezug zu organischen Elektrolumineszenzvorrichtungen ist nicht zu erkennen.

In der JP 2007/329495 werden verschiedene Phosphinoxidverbindungen für die Verwendung in OLEDs offenbart. Phosphinoxidverbindungen, in denen zwei Reste am Phosphor miteinander einen Ring bilden, sind nicht offenbart.

S. Ogawa et al. (Heterocycles 1995, 41 (5), 889-892) offenbaren verschiedene cyclische Phosphorverbindungen, die jeweils ein trivalentes Phosphoratom aufweisen. Entsprechende cyclische Verbindungen, die statt des trivalentes Phosphoratoms ein Phosphinoxid aufweisen, sind nicht offenbart.

Ein Bezug zu organischen Elektrolumineszenzvorrichtungen ist nicht zu erkennen.

O. S. Anisimova et al. (Journal of General Chemistry USSR, 1976, 46, 807-811) offenbaren verschiedene cyclische Phosphorverbindungen, die entweder ein trivalentes Phosphoratom oder eine Gruppe P(=S) aufweisen. Verbindungen, die statt dessen ein Phosphinoxid enthalten, sind nicht offenbart. Ein Bezug zu organischen Elektrolumineszenzvorrichtungen ist nicht zu erkennen.

J. H. Lister et al. (Journal of the Chemical Society, Section C: Organic Chemistry 1966, 14, 2142-1244) offenbaren Diazaphospholverbindungen, wobei an mindestens einen der Stickstoffatome ein Wasserstoff gebunden ist. Verbindungen, in denen beide Stickstoffatome des Diazaphosphol substituiert sind, sind nicht offenbart. Ein Bezug zu organischen Elektrolumineszenzvorrichtungen ist nicht zu erkennen.

Gegenstand der vorliegenden Erfindung ist eine elektronische Vorrichtung enthaltend mindestens eine Verbindung gemäß der folgenden Formel (1), 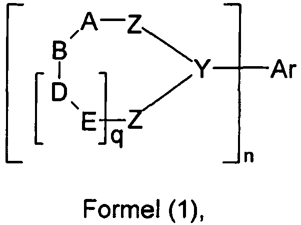 wobei für die verwendeten Symbole und Indizes gilt:
- A-B und D-E: ist jeweils gleich oder verschieden bei jedem Auftreten eine Einheit der folgenden Formel (2), (3), (4), (5) oder (6), 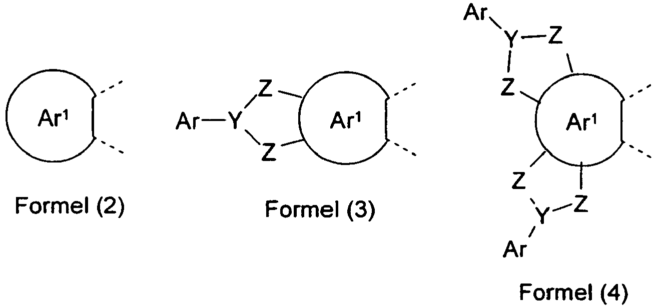 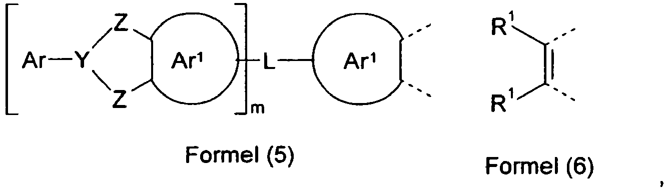 wobei die gestrichelte Bindung jeweils die Verknüpfung mit Z darstellt;
und
- Z: ist gleich oder verschieden bei jedem Auftreten N-R², O oder S;
oder
- A-Z und B-Z: ist jeweils gleich oder verschieden bei jedem Auftreten eine Einheit der folgenden Formel (7) und q = 0, 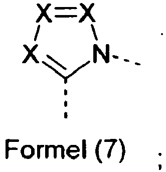 dabei stellt die gestrichelte Bindung in Formel (7) jeweils die Verknüpfung dieser Einheit in der Verbindung der Formel (1) dar, wobei der Stickstoff mit der Gruppe Y verknüpft ist;
- Y: ist bei jedem Auftreten gleich oder verschieden P(=O), P(=S), P, As(=O), As(=S), As, Sb(=O), Sb(=S), Sb, Bi(=O), Bi(=S) oder Bi;
- Ar: ist bei jedem Auftreten gleich oder verschieden ein aromatisches oder heteroaromatisches Ringsystem mit 5 bis 60 aromatischen Ringatomen, welches durch einen oder mehrere Reste R¹ substituiert sein kann;
- Ar¹: ist bei jedem Auftreten gleich oder verschieden eine Aryl- oder Heteroarylgruppe mit 5 bis 18 aromatischen Ringatomen, welche durch einen oder mehrere Reste R¹ substituiert sein kann;
- X: ist bei jedem Auftreten gleich oder verschieden CR¹ oder N;
- L: ist eine Einfachbindung oder eine bivalente, trivalente oder tetravalente Gruppe;
- R¹: ist bei jedem Auftreten gleich oder verschieden ausgewählt aus der Gruppe bestehend aus H, D, F, Cl, Br, I, CN, NO₂, N(R³)₂, C(=O)R³, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C=C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
- R²: ist bei jedem Auftreten gleich oder verschieden ausgewählt aus der Gruppe bestehend aus einer geradkettigen Alkylgruppe mit 1 bis 40 C-Atomen oder einer verzweigten oder cyclischen Alkylgruppe mit 3 bis 40 C-Atomen, die jeweils mit einem oder mehreren Resten R³ substituiert sein kann, wobei eine oder mehrere nicht-benachbarte CH₂-Gruppen durch R³C=CR³, C=C oder C=O ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, oder einer Kombination dieser Systeme; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein;
- R³: ist ausgewählt aus der Gruppe bestehend aus H, D, F, CN, aliphatischem Kohlenwasserstoffrest mit 1 bis 20 C-Atomen, aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches, aromatisches oder heteroaromatisches Ringsystem bilden können;
- n: ist 1 bis 10, bevorzugt 1, 2, 3, 4, 5 oder 6;
- m: ist 1, wenn L eine Einfachbindung oder eine bivalente Gruppe ist, oder ist 2, wenn L eine trivalente Gruppe ist, oder ist 3, wenn L eine tetravalente Gruppe ist;
- q: ist bei jedem Auftreten gleich oder verschieden 0 oder 1.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, N-oder O-Atom, unterbrochen sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe unterbrochen sind.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch R³C=CR³, C=C, Si(R³)₂, Ge(R³)₂, Sn(R³)₂, C=O, C=S, C=Se, C=NR³, P(=O)(R³), SO, SO₂, NR³, O, S oder CONR³ ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R³ oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Wenn die Verbindung der Formel (1) eine Einheit gemäß einer oder mehrerer der Formeln (2) bis (5) enthält, so steht Ar¹ bevorzugt gleich oder verschieden bei jedem Auftreten für eine Aryl- oder Heteroarylgruppe mit 5 bis 14 aromatischen Ringatomen, besonders bevorzugt mit 5 bis 10 aromatischen Ringatomen, ganz besonders bevorzugt mit 6 aromatischen Ringatomen. Besonders geeignete Aryl- und Heteroarylgruppen Ar¹ sind gleich oder verschieden bei jedem Auftreten ausgewählt aus der Gruppe bestehend aus Benzol, Pyridin, Pyrimidin, Pyridazin, Pyrazin, Triazin, Furan, Thiophen, Pyrrol, Naphthalin, Phenanthren, Chinolin, Isochinolin, Chinoxalin, Indol, Benzofuran, Benzothiophen und Carbazol.

Bevorzugte Ausführungsformen der Verbindungen gemäß der oben aufgeführten Formel (1) sind die Verbindungen der Formeln (8) bis (18), 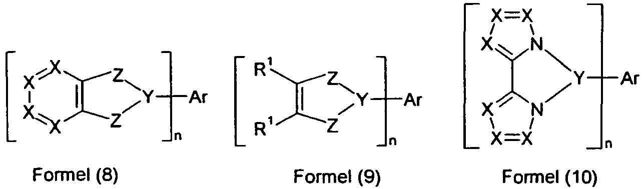 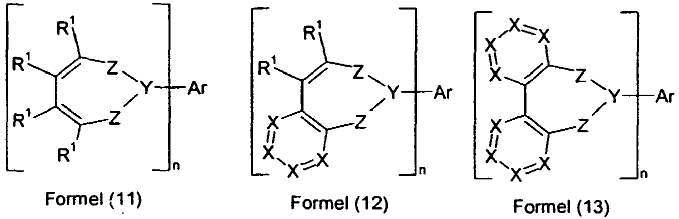 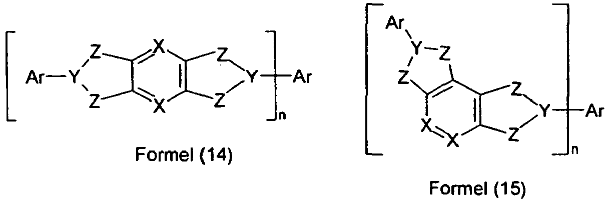 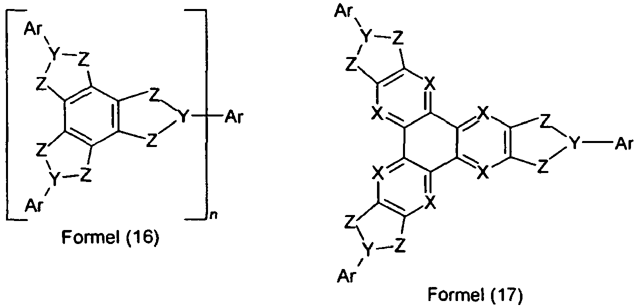 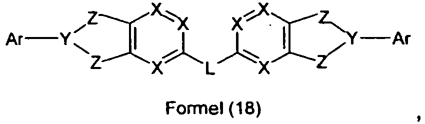 wobei die verwendeten Symbole und Indizes die oben genannten Bedeutungen aufweisen.

In einer bevorzugten Ausführungsform der Verbindungen gemäß Formel (1) bzw. Formel (8) bis (18) steht das Symbol Y bei jedem Auftreten gleich oder verschieden für P(=O), P(=S) oder P. Besonders bevorzugt steht das Symbol Y für P(=O) oder P(=S), ganz besonders bevorzugt für P(=O).

In einer weiteren bevorzugten Ausführungsform der Verbindungen gemäß Formel (1) bzw. Formel (8) bis (18) steht das Symbol Z, wenn es nicht mit A bzw. B einen Ring gemäß Formel (7) bildet, gleich oder verschieden bei jedem Auftreten für N-R².

In einer besonders bevorzugten Ausführungsform der Verbindungen gemäß Formel (1) bzw. Formel (8) bis (18) steht das Symbol Y für P(=O), und das Symbol Z steht bei jedem Auftreten gleich oder verschieden für N-R².

In einer weiteren bevorzugten Ausführungsform der Verbindungen gemäß Formel (1) bzw. Formel (18) steht das Symbol L für eine Einfachbindung, O, S, NR², eine Alkylengruppe mit 1 bis 10 C-Atomen, welche mit einem oder mehreren Resten R³ substituiert sein kann, oder ein aromatisches oder heteroaromatisches Ringsystem mit 5 bis 30 aromatischen Ringatomen, welches mit einem oder mehreren Resten R³ substituiert sein kann.

In einer weiteren bevorzugten Ausführungsform der Erfindung stehen maximal zwei Symbole X in jedem Cyclus für N und die anderen Symbole X stehen gleich oder verschieden bei jedem Auftreten für CR¹. Besonders bevorzugt steht maximal ein Symbol X in jedem Cyclus für N und die anderen Symbole X stehen gleich oder verschieden bei jedem Auftreten für CR¹. Ganz besonders bevorzugt stehen alle Symbole X gleich oder verschieden bei jedem Auftreten für CR¹.

In einer weiteren bevorzugten Ausführungsform der Erfindung steht die Gruppe Ar für ein aromatisches oder heteroaromatisches Ringsystem mit 5 bis 30 aromatischen Ringatomen, besonders bevorzugt mit 5 bis 24 aromatischen Ringatomen. Dabei enthält bevorzugt keine der Aryl- bzw. Heteroarylgruppen des aromatischen oder heteroaromatischen Ringsystems mehr als 10 aromatische Ringatome. Bevorzugte Gruppen Ar sind daher aufgebaut aus jeweils einer oder mehreren der Gruppen Benzol, Pyridin, Pyrimidin, Pyridazin, Pyrazin, Triazin, Pyrrol, Thiophen, Furan, Naphthalin, Chinolin, Isochinolin, Chinoxalin, Indol, Benzothiophen oder Benzofuran. Besonders bevorzugte Gruppen Ar sind aufgebaut aus jeweils einer oder mehreren Gruppen Benzol, Pyridin, Pyrimidin, Pyridazin, Pyrazin oder Triazin, insbesondere Benzol. Eine weitere bevorzugte Gruppe Ar ist Triphenylen.

In einer besonders bevorzugten Ausführungsform der Erfindung ist Ar ausgewählt aus der Gruppe bestehend aus den Einheiten der folgenden Formeln (19) bis (36), wobei die gestrichelte Bindung jeweils eine Verknüpfung mit der Gruppe Y andeutet:

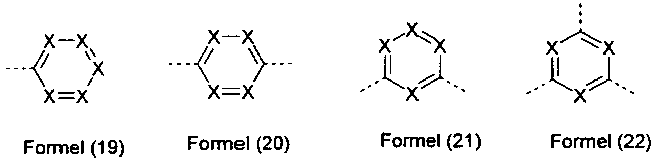

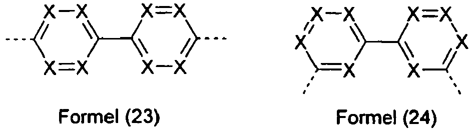

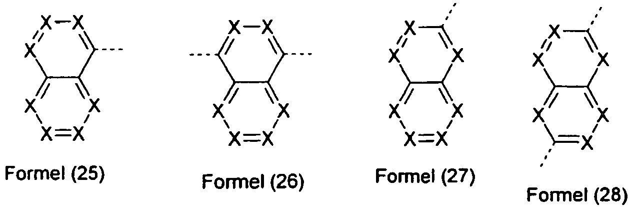

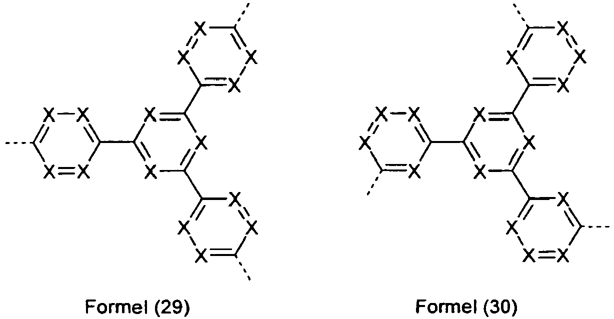

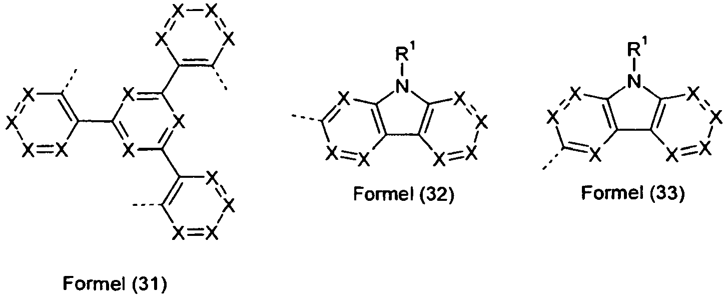

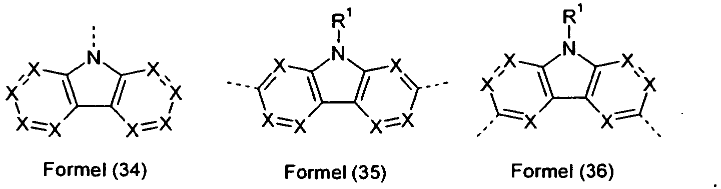

In einer weiteren bevorzugten Ausführungsform der Erfindung ist der Index q=0.

In einer weiteren bevorzugten Ausführungsform der Erfindung ist der Index n = 1, 2, 3 oder 4, besonders bevorzugt 1, 2 oder 3, ganz besonders bevorzugt 1 oder 2.

In einer weiteren bevorzugten Ausführungsform der Erfindung ist der Rest R¹ gleich oder verschieden bei jedem Auftreten ausgewählt aus der Gruppe bestehend aus H, D, F, CN, N(R³)₂, einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen oder einer Alkenylgruppe mit 2 bis 20 C-Atomen, die jeweils mit einem oder mehreren Resten R³ substituiert sein kann, wobei eine oder mehrere nicht-benachbarte CH₂-Gruppen durch R³C=CR³ oder O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R³ substituiert sein kann, einer Aryloxy- oder Heteroaryloxygruppe mit 5 bis 30 aromatischen Ringatomen, die mit einem oder mehreren Resten R³ substituiert sein können, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann. Besonders bevorzugt ist der Rest R¹ gleich oder verschieden bei jedem Auftreten ausgewählt aus der Gruppe bestehend aus H, D, CN, F, einer geradkettigen Alkylgruppe mit 1 bis 10 C-Atomen, besonders bevorzugt mit 1 bis 4 C-Atomen, oder einer verzweigten oder cyclischen Alkylgruppe mit 3 bis 10 C-Atomen, besonders bevorzugt mit 3 bis 6 C-Atomen, oder einer Alkenylgruppe mit 2 bis 10 C-Atomen, besonders bevorzugt mit 2 bis 4 C-Atomen, die jeweils mit einem oder mehreren Resten R³ substituiert sein kann, wobei ein oder mehrere H-Atome durch D ersetzt sein können, einem aromatischen oder heteroaromatischen Ringsystem mit 5 bis 12 aromatischen Ringatomen, das jeweils mit einem oder mehreren Resten R³ substituiert sein kann, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann.

In einer weiteren bevorzugten Ausführungsform der Erfindung ist R² bei jedem Auftreten gleich oder verschieden ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R³ substituiert sein kann, wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R³ substituiert sein kann, oder einer Kombination dieser Systeme; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein kann. In einer besonders bevorzugten Ausführungsform der Erfindung ist R² bei jedem Auftreten gleich oder verschieden ausgewählt aus der Gruppe bestehend aus einem aromatischen oder heteroaromatischen Ringsystem mit 5 bis 24 aromatischen Ringatomen, das jeweils mit einem oder mehreren Resten R³ substituiert sein kann. In einer ganz besonders bevorzugten Ausführungsform der Erfindung steht R² für Phenyl, Naphthyl, Biphenyl oder Terphenyl, welches jeweils mit einem oder mehreren Resten R³ substituiert sein kann, insbesondere für Phenyl oder Biphenyl, welches jeweils mit einem oder mehreren Resten R³ substituiert sein kann, jedoch bevorzugt unsubstituiert ist.

Besonders bevorzugt sind Verbindungen der oben aufgeführten Formeln (1) bzw. (8) bis (18), in denen die oben genannten Bevorzugungen gleichzeitig gelten. Besonders bevorzugt sind daher Verbindungen, in denen R³ wie vorne definiert ist und weiterhin gilt:
- Ar¹: ist gleich oder verschieden bei jedem Auftreten für eine Aryl- oder Heteroarylgruppe mit 5 bis 14 aromatischen Ringatomen, besonders bevorzugt mit 5 bis 10 aromatischen Ringatomen, ganz besonders bevorzugt mit 6 aromatischen Ringatomen;
- Y: ist bei jedem Auftreten gleich oder verschieden P(=O) oder P(=S);
- Z: ist gleich oder verschieden bei jedem Auftreten N-R², wenn es nicht mit A bzw. B einen Ring gemäß Formel (7) bildet;
- X: steht für CR¹ oder N, wobei maximal zwei Symbole X für N stehen, bevorzugt maximal ein Symbol X;
- Ar: ist gleich oder verschieden ein aromatisches oder heteroaromatisches Ringsystem mit 5 bis 30 aromatischen Ringatomen, besonders bevorzugt mit 5 bis 24 aromatischen Ringatomen; dabei enthält bevorzugt keine der Aryl- bzw. Heteroarylgruppen des aromatischen oder heteroaromatischen Ringsystems mehr als 10 aromatische Ringatome;
- q: ist 0;
- n: ist 1, 2, 3 oder 4, bevorzugt 1, 2 oder 3.
- R¹: ist gleich oder verschieden bei jedem Auftreten ausgewählt aus der Gruppe bestehend aus H, D, F, CN, N(R³)₂, einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen oder einer Alkenylgruppe mit 2 bis 20 C-Atomen, die jeweils mit einem oder mehreren Resten R³ substituiert sein kann, wobei eine oder mehrere nicht-benachbarte CH₂-Gruppen durch R³C=CR oder O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R³ substituiert sein kann, einer Aryloxy- oder Heteroaryloxygruppe mit 5 bis 30 aromatischen Ringatomen, die mit einem oder mehreren Resten R³ substituiert sein können, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
- R²: ist bei jedem Auftreten gleich oder verschieden ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R³ substituiert sein kann, wobei ein oder mehrere H-Atome durch F oder D ersetzt sein können, einem aromatischen oder heteroaromatischen Ringsystem mit 5 bis 30 aromatischen Ringatomen, das jeweils mit einem oder mehreren Resten R³ substituiert sein kann, oder einer Kombination dieser Systeme; dabei können optional solche R¹ und R², die in 1,2-Position zueinander benachbart stehen, ein monocyclisches oder polycydisches, aliphatisches, aromatisches oder heteroaromatisches Ringsystem bilden, das mit einem oder mehreren Resten R³ substituiert sein kann.

Ganz besonders bevorzugt sind Verbindungen gemäß Formel (1) bzw. Formel (8) bis (18), in denen R³ wie vorne definiert ist und weiterhin gilt:
- Ar¹: ist gleich oder verschieden bei jedem Auftreten ausgewählt aus der Gruppe bestehend aus Benzol, Pyridin, Pyrimidin, Pyridazin, Pyrazin, Triazin, Furan, Thiophen, Pyrrol, Naphthalin, Chinolin, Isochinolin, Chinoxalin, Indol, Benzofuran, Benzothiophen und Carbazol;
- Y: ist P(=O);
- Z: ist gleich oder verschieden bei jedem Auftreten N-R², wenn es nicht mit A bzw. B einen Ring gemäß Formel (7) bildet;
- X: ist gleich oder verschieden bei jedem Auftreten CR¹;
- Ar: ist ein aromatisches oder heteroaromatisches Ringsystem mit 5 bis 24 aromatischen Ringatomen, welches aufgebaut ist aus jeweils einer oder mehreren der Gruppen Benzol, Pyridin, Pyrimidin, Pyridazin, Pyrazin, Pyrrol, Thiophen, Furan, Naphthalin, Triphenylen, Chinolin, Isochinolin, Chinoxalin, Indol, Benzothiophen oder Benzofuran, bevorzugt aus jeweils einer oder mehreren Gruppen Benzol, Pyridin, Pyrimidin, Pyridazin, Pyrazin oder Triazin, insbesondere Benzol;
- q: ist 0;
- n: ist 1 oder 2;
- R¹: ist gleich oder verschieden bei jedem Auftreten ausgewählt aus der Gruppe bestehend aus H, D, F, CN, einer geradkettigen Alkylgruppe mit 1 bis 10 C-Atomen, bevorzugt mit 1 bis 4 C-Atomen, oder einer verzweigten oder cyclischen Alkylgruppe mit 3 bis 10 C-Atomen, besonders bevorzugte mit 3 bis 5 C-Atomen, oder einer Alkenylgruppe mit 2 bis 10 C-Atomen, bevorzugt mit 2 bis 4 C-Atomen, die jeweils mit einem oder mehreren Resten R³ substituiert sein kann, wobei ein oder mehrere H-Atome durch D ersetzt sein können, einem aromatischen oder heteroaromatischen Ringsystem mit 5 bis 12 aromatischen Ringatomen, das jeweils mit einem oder mehreren Resten R³ substituiert sein kann, und einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
- R²: ist bei jedem Auftreten gleich oder verschieden ausgewählt aus der Gruppe bestehend aus einem aromatischen oder heteroaromatischen Ringsystem mit 5 bis 24 aromatischen Ringatomen, das jeweils mit einem oder mehreren Resten R³ substituiert sein kann.

Für Verbindungen, die aus Lösung verarbeitet werden, eignen sich als Substituenten insbesondere auch lange Alkylgruppen, beispielsweise mit 5 bis 10 C-Atomen, oder substituierte oder unsubstituierte Oligoarylengruppen. Geeignete Oligoarylengruppen sind zum Beispiel Terphenyl, insbesondere meta-Terphenyl, verzweigtes Terphenyl, meta-Quarterphenyl oder verzweigtes Quarterphenyl.

Bevorzugt sind weiterhin Verbindungen, in welchen alle Reste R² gleich gewählt sind.

Beispiele für bevorzugte Verbindungen gemäß den oben aufgeführten Ausführungsformen bzw. Verbindungen, wie sie bevorzugt in organischen elektronischen Vorrichtungen eingesetzt werden können, sind die Verbindungen der folgenden Strukturen (1) bis (274).

| | | |
|---|---|---|
| 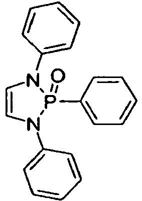 | 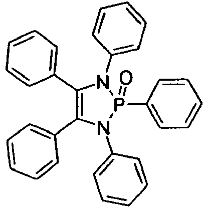 | 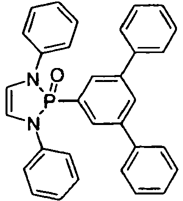 |
| (1) | (2) | (3) |
| 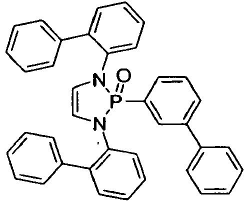 | 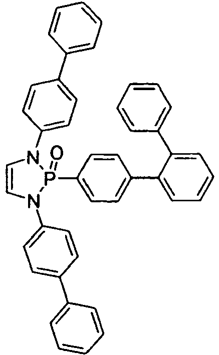 | 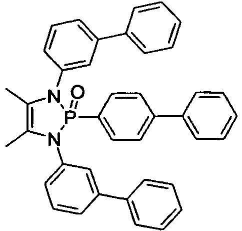 |
| (4) | (5) | (6) |
| 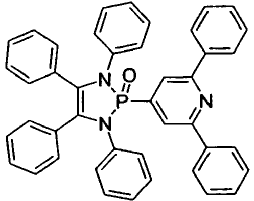 | 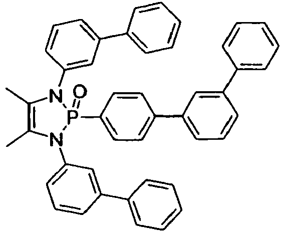 | 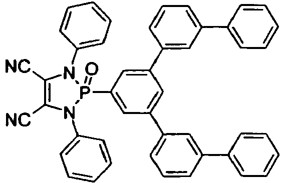 |
| (7) | (8) | (9) |
| 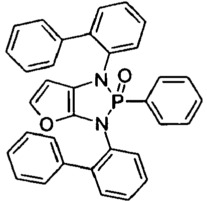 | 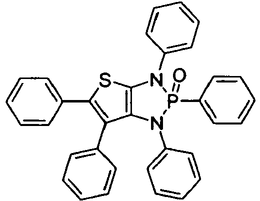 | 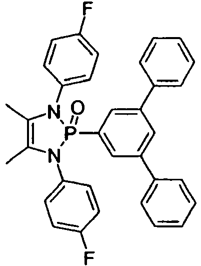 |
| (10) | (11) | (12) |
| 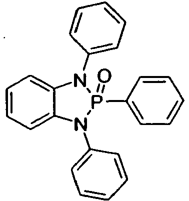 | 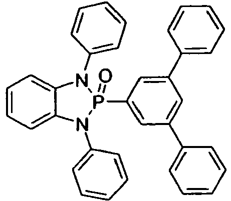 | 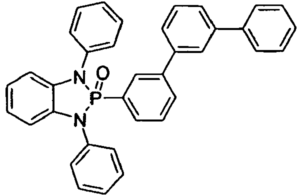 |
| (13) | (14) | (15) |
| 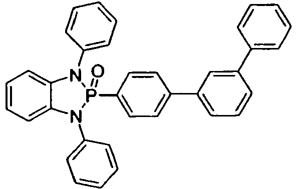 | 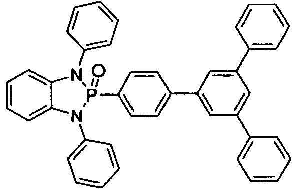 | 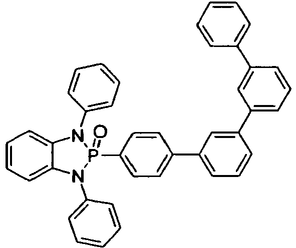 |
| (16) | (17) | (18) |
| 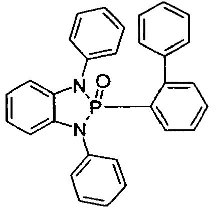 | 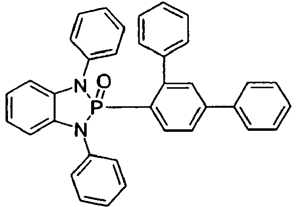 | 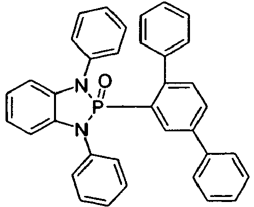 |
| (19) | (20) | (21) |
| 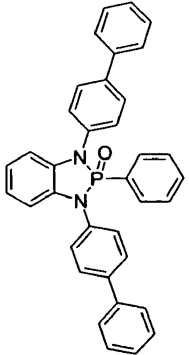 | 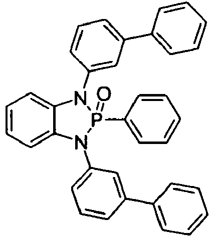 | 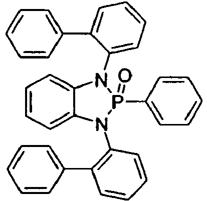 |
| (22) | (23) | (24) |
| 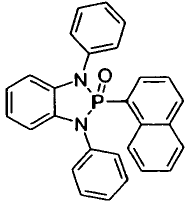 | 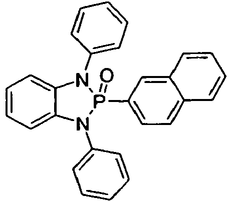 | 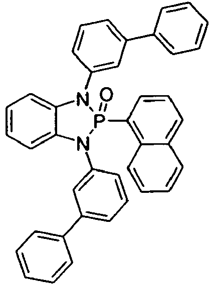 |
| (25) | (26) | (27) |
| 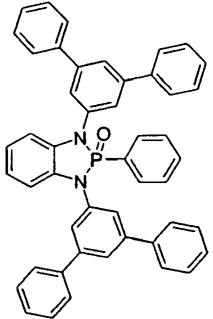 | 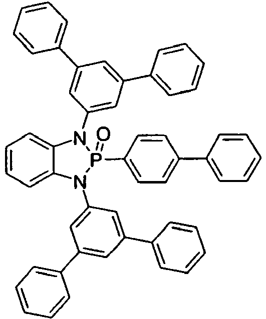 | 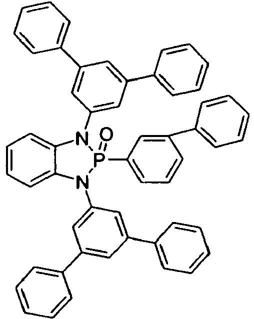 |
| (28) | (29) | (30) |
| 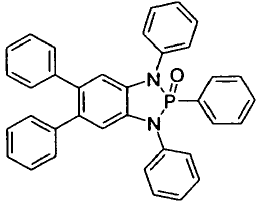 | 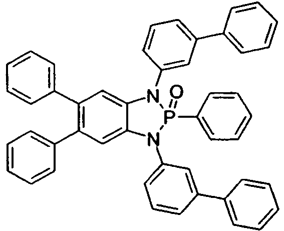 | 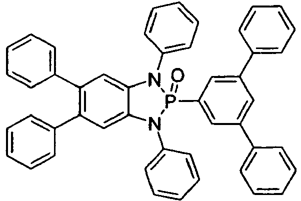 |
| (31) | (32) | (33) |
| 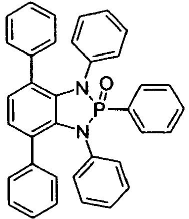 | 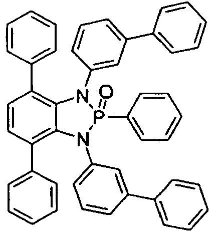 | 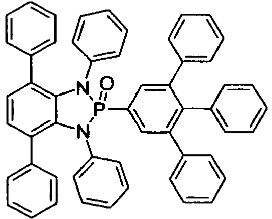 |
| (34) | (35) | (36) |
| 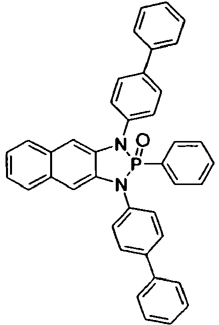 | 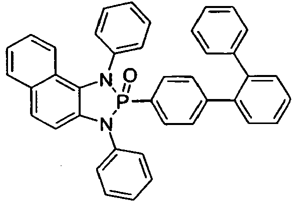 | 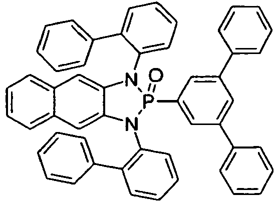 |
| (37) | (38) | (39) |
| 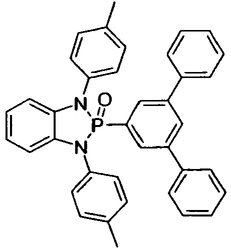 | 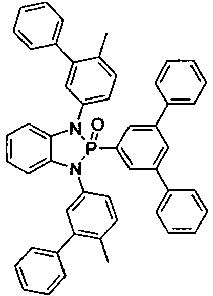 | 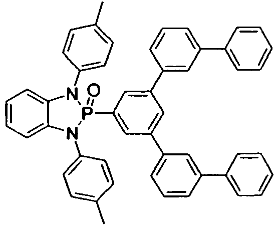 |
| (40) | (41) | (42) |
| 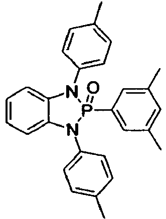 | 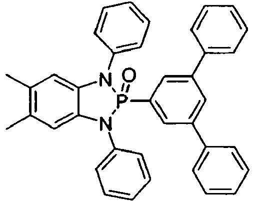 | 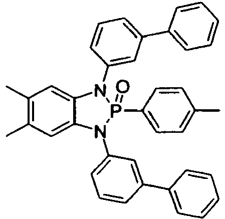 |
| (43) | (44) | (45) |
| 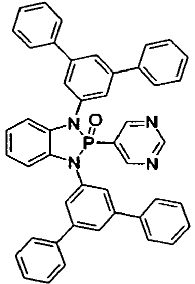 | 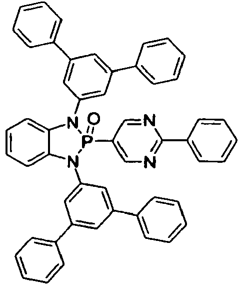 | 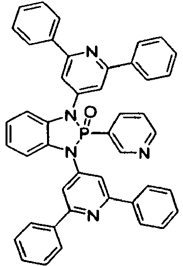 |
| (46) | (47) | (48) |
| 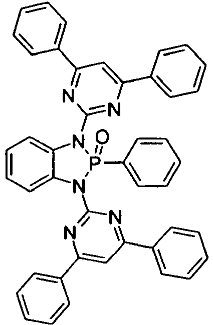 | 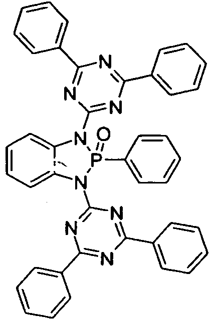 | 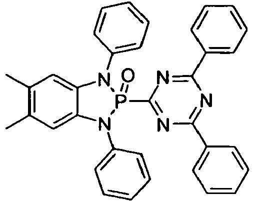 |
| (49) | (50) | (51) |
| 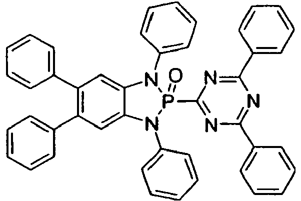 | 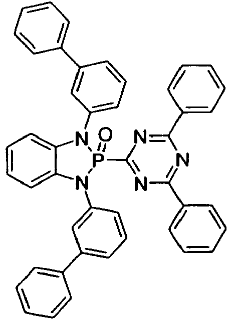 | 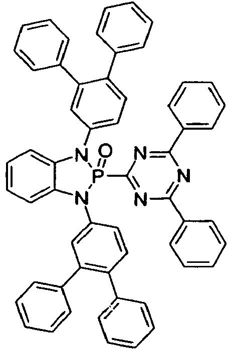 |
| (52) | (53) | (54) |
| 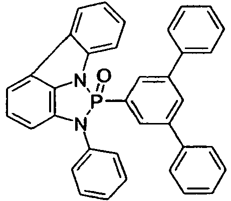 | 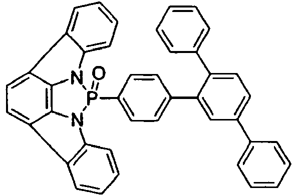 | 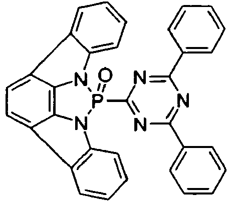 |
| (55) | (56) | (57) |
| 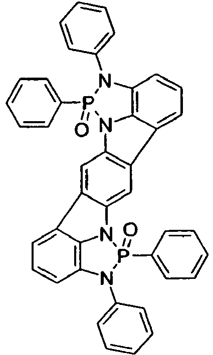 | 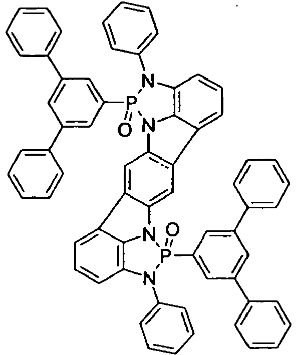 | 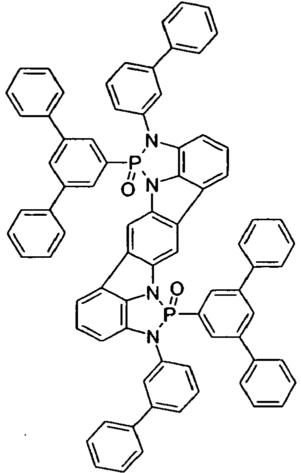 |
| (58) | (59) | (60) |
| 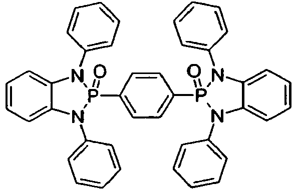 | 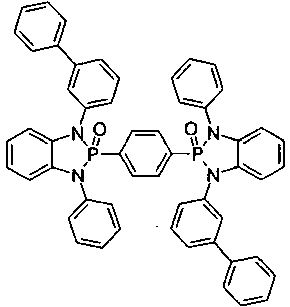 | 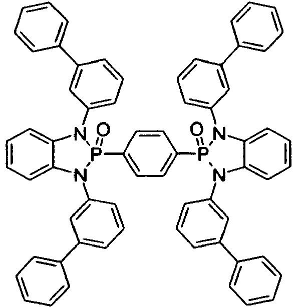 |
| (61) | (62) | (63) |
| 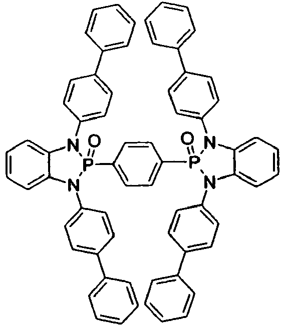 | 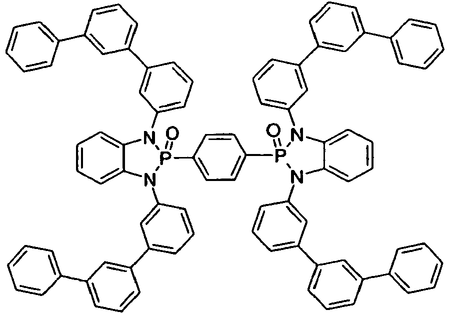 | |
| (64) | (65) | |
| 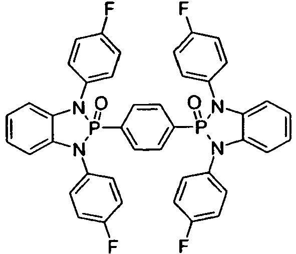 | 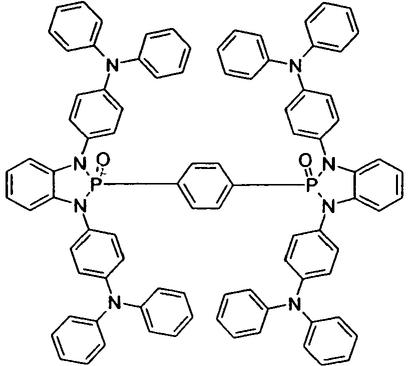 | |
| (66) | (67) | |
| 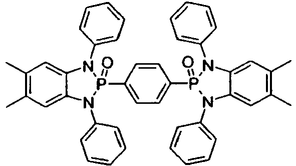 | 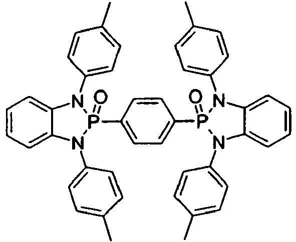 | 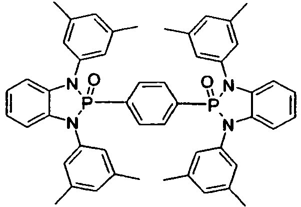 |
| (68) | (69) | (70) |
| 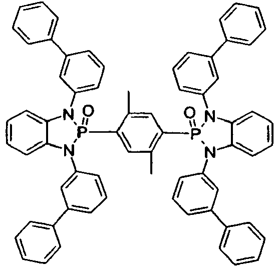 | 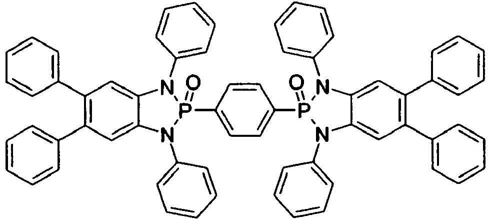 | |
| (71) | (72) | |
| 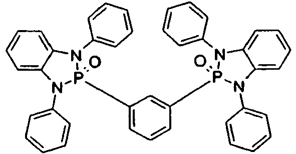 | 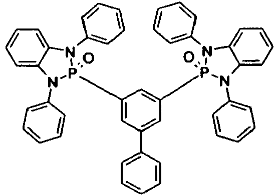 | 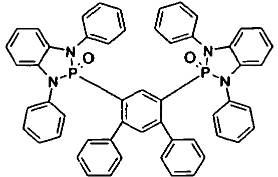 |
| (73) | (74) | (75) |
| 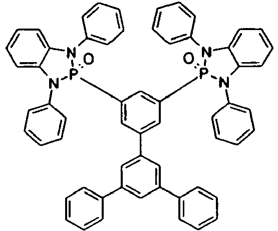 | 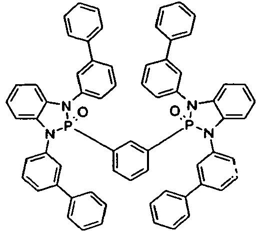 | 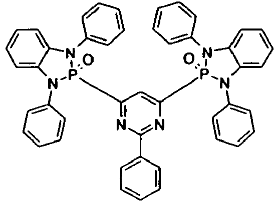 |
| (76) | (77) | (78) |
| 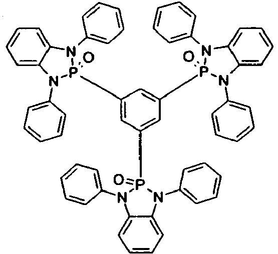 | 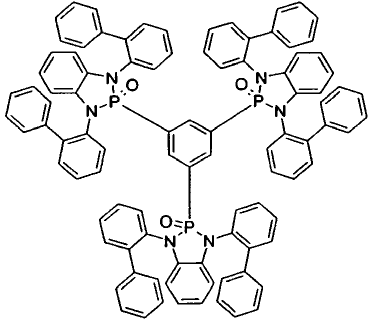 | |
| (79) | (80) | |
| 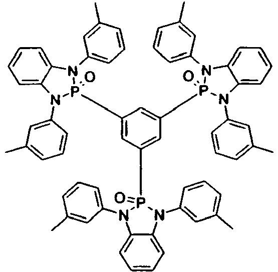 | 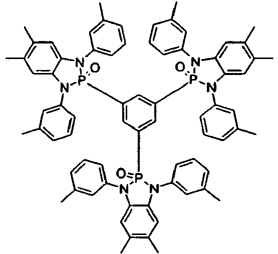 | 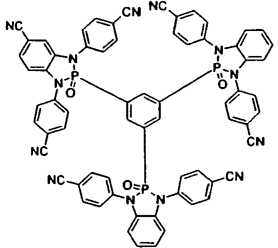 |
| (81) | (82) | (83) |
| 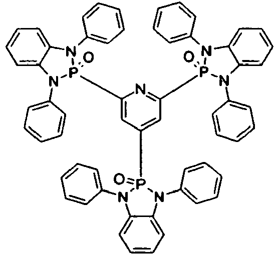 | 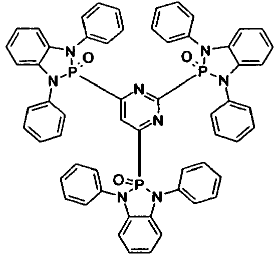 | 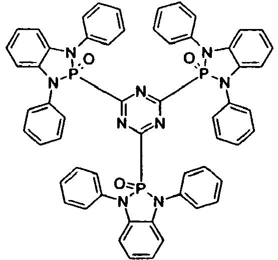 |
| (84) | (85) | (86) |
| 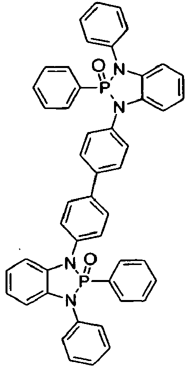 | 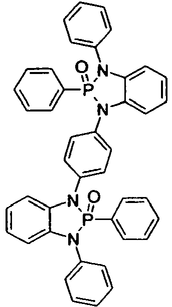 | 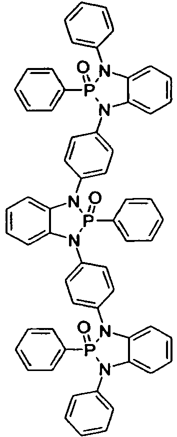 |
| (87) | (88) | (89) |
| 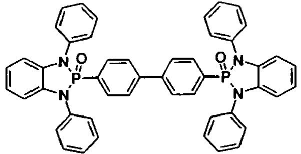 | 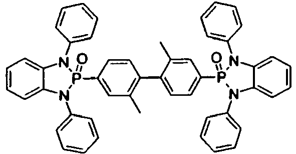 | 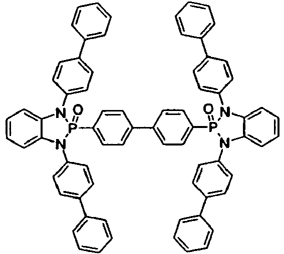 |
| (90) | (91) | (92) |
| 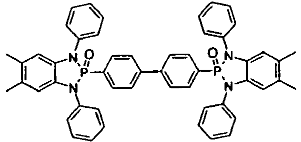 | 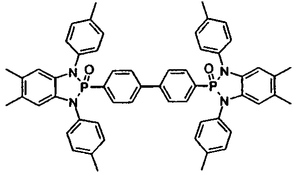 | 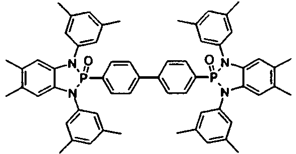 |
| (93) | (94) | (95) |
| 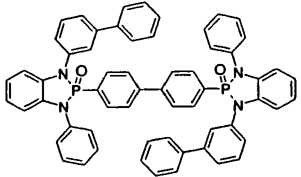 | 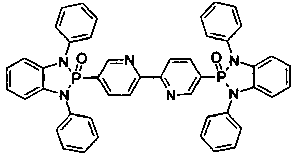 | 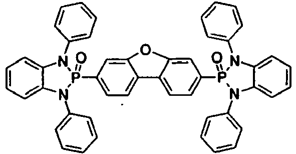 |
| (96) | (97) | (98) |
| 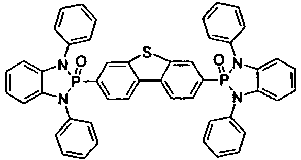 | 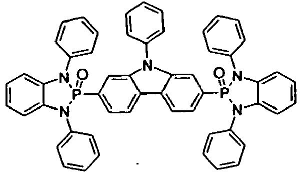 | 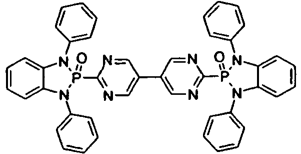 |
| (99) | (100) | (101) |
| 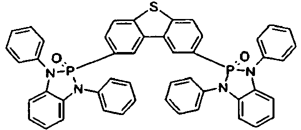 | 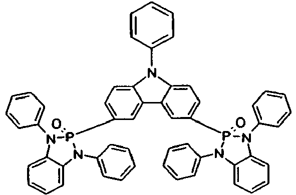 | 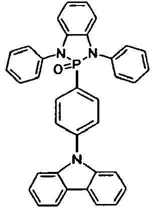 |
| (102) | (103) | (104) |
| 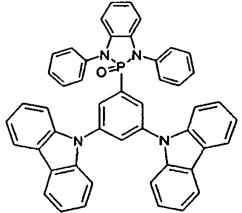 | 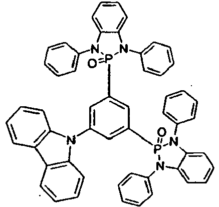 | 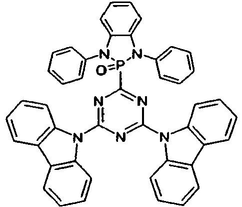 |
| (105) | (106) | (107) |
| 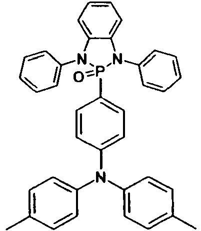 | 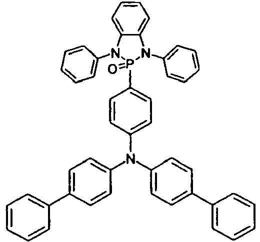 | 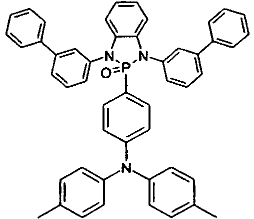 |
| (108) | (109) | (110) |
| 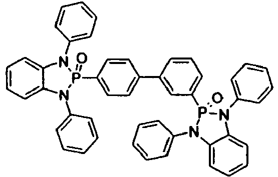 | 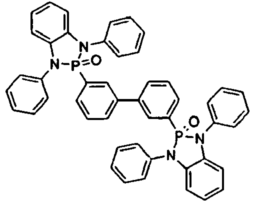 | 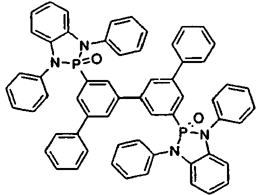 |
| (111) | (112) | (113) |
| 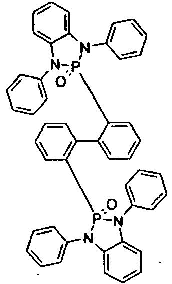 | 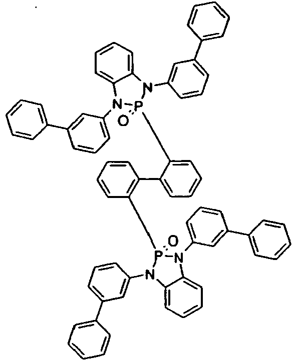 | 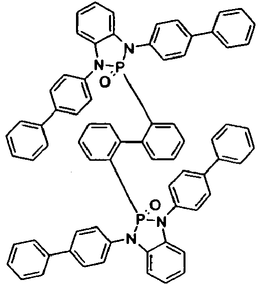 |
| (114) | (115) | (116) |
| 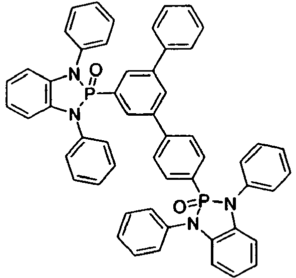 | 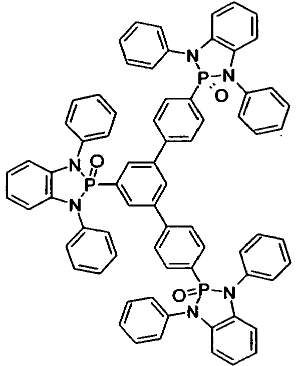 | 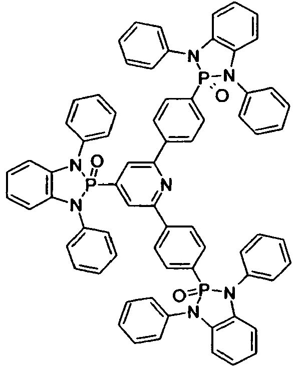 |
| (117) | (118) | (119) |
| 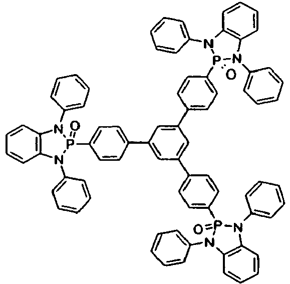 | 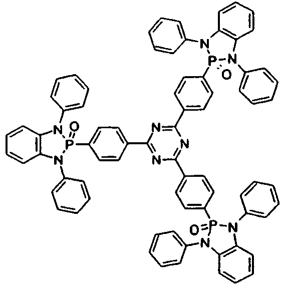 | 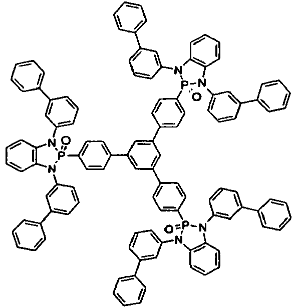 |
| (120) | (121) | (122) |
| 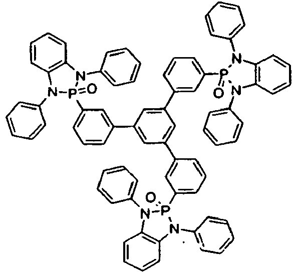 | 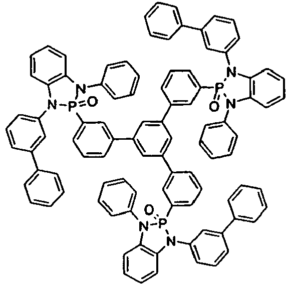 | 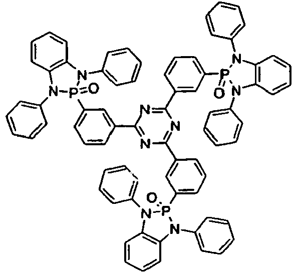 |
| (123) | (124) | (125) |
| 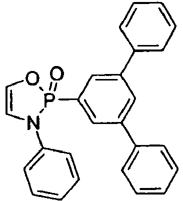 | 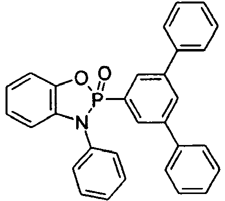 | 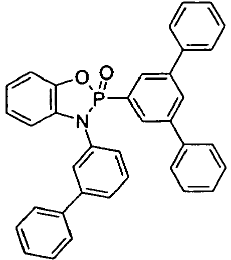 |
| (126) | (127) | (128) |
| 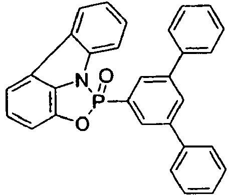 | 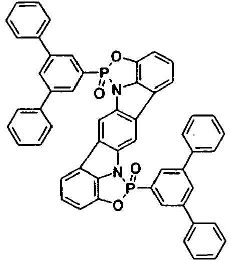 | 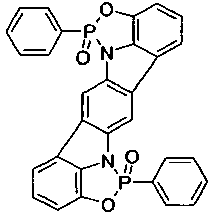 |
| (129) | (130) | (131) |
| 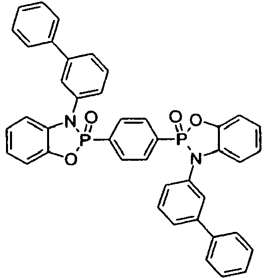 | 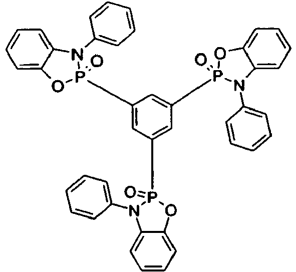 | 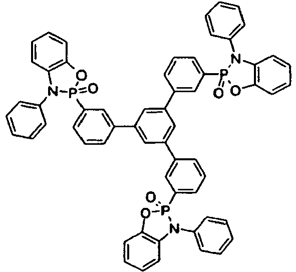 |
| (132) | (133) | (134) |
| 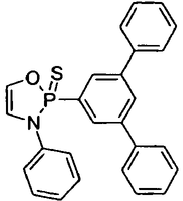 | 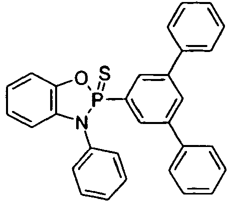 | 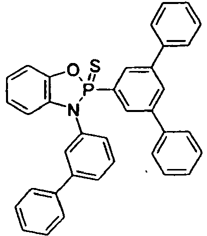 |
| (135) | (136) | (137) |
| 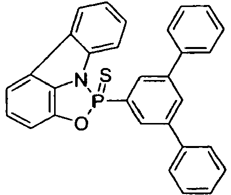 | 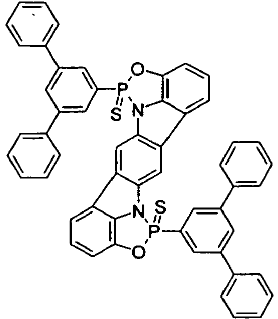 | 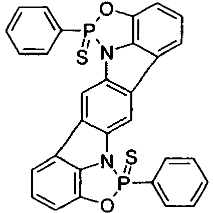 |
| (138) | (139) | (140) |
| 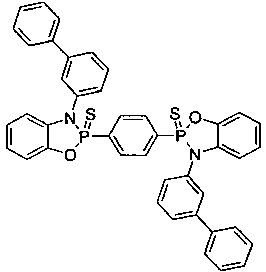 | 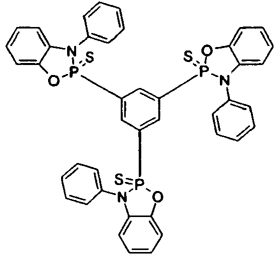 | 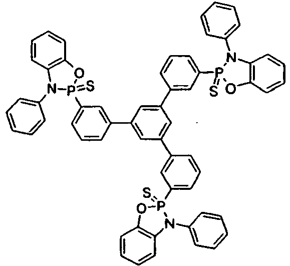 |
| (141) | (142) | (143) |
| 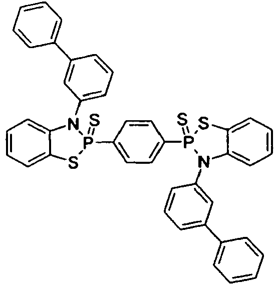 | 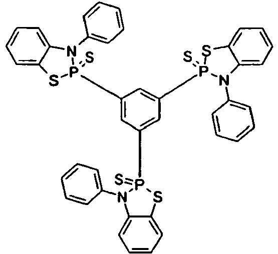 | 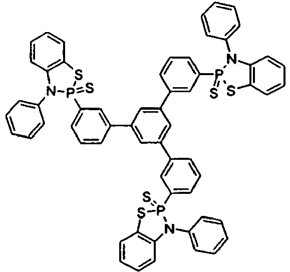 |
| (144) | (145) | (146) |
| 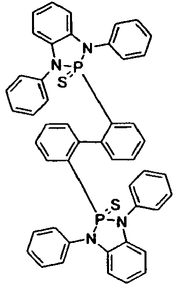 | 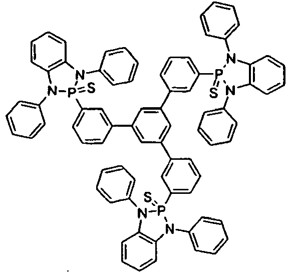 | 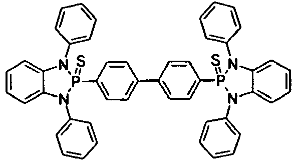 |
| (147) | (148) | (149) |
| 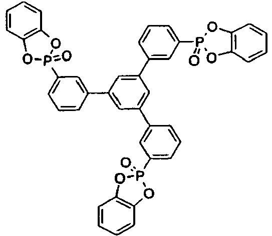 | 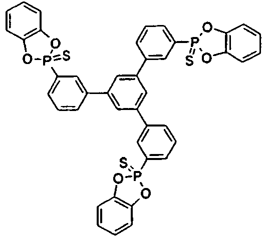 | 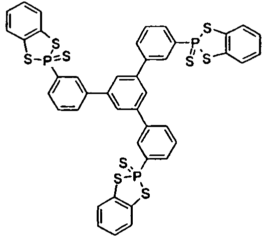 |
| (150) | (151) | (152) |
| 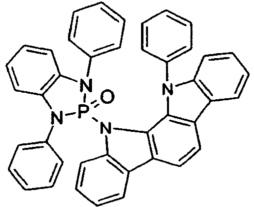 | 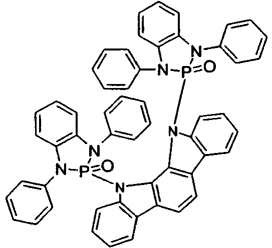 | 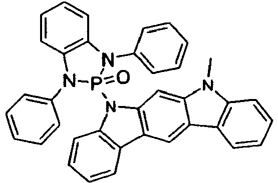 |
| (153) | (154) | (155) |
| 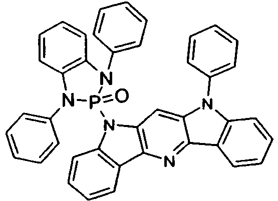 | 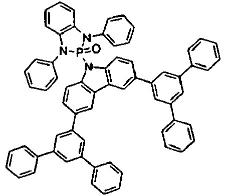 | 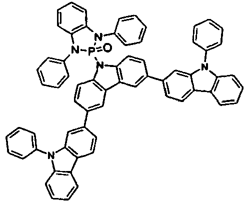 |
| (156) | (157) | (158) |
| 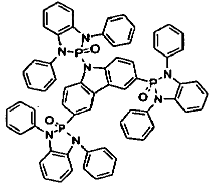 | 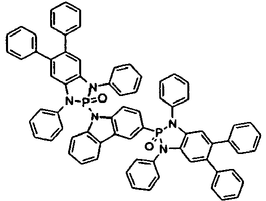 | 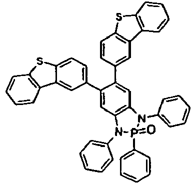 |
| (159) | (160) | (161) |
| 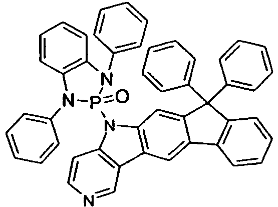 | 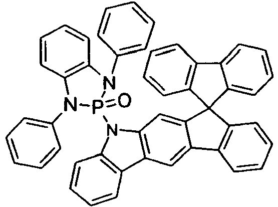 | 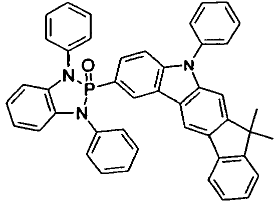 |
| (162) | (163) | (164) |
| 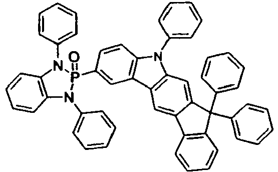 | 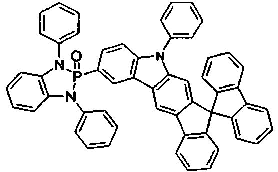 | 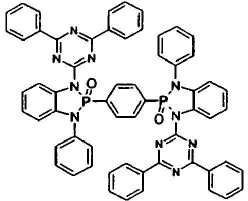 |
| (165) | (166) | (167) |
| 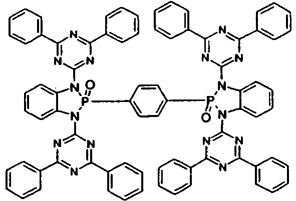 | 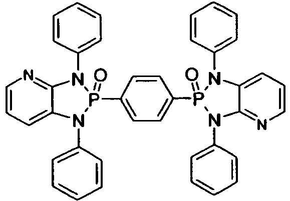 | 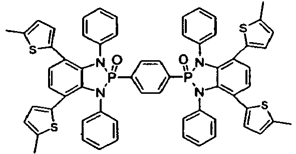 |
| (168) | (169) | (170) |
| 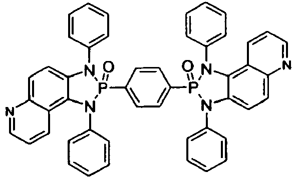 | 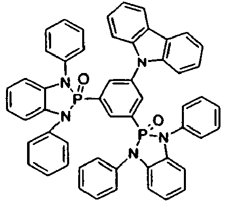 | 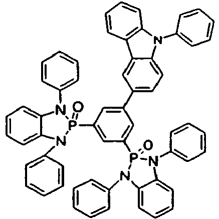 |
| (171) | (172) | (173) |
| 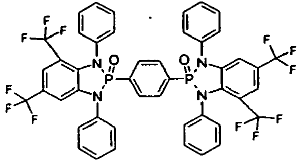 | 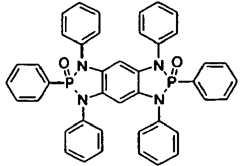 | 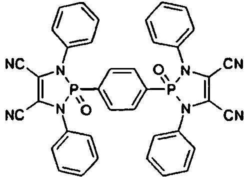 |
| (174) | (175) | (176) |
| 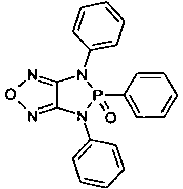 | 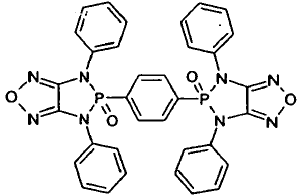 | 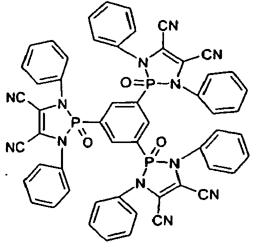 |
| (177) | (178) | (179) |
| 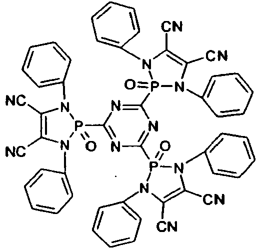 | 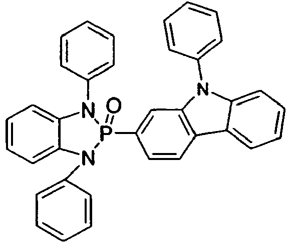 | 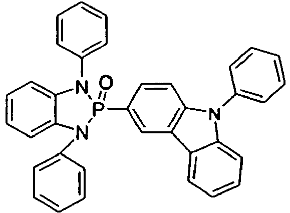 |
| (180) | (181) | (182) |
| 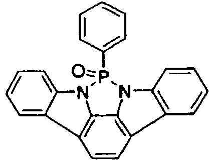 | 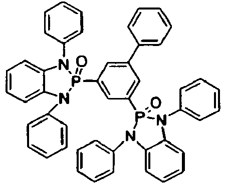 | 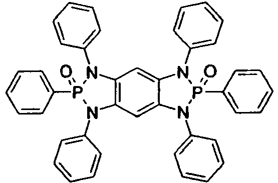 |
| (182) | (183) | (184) |
| 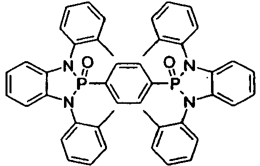 | 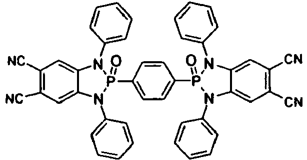 | 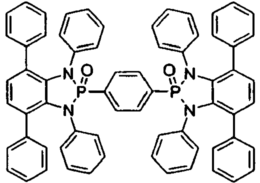 |
| (185) | (186) | (187) |
| 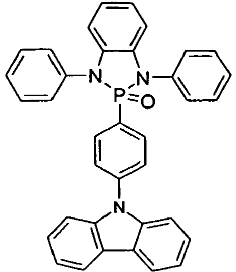 | 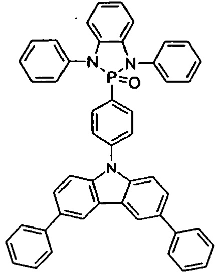 | 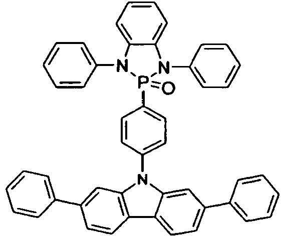 |
| (188) | (189) | (190) |
| 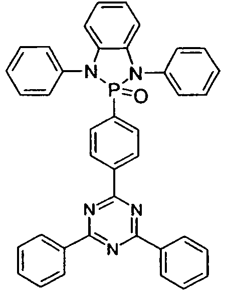 | 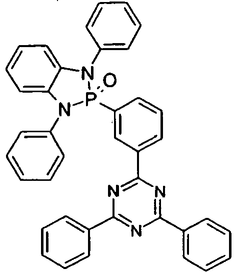 | 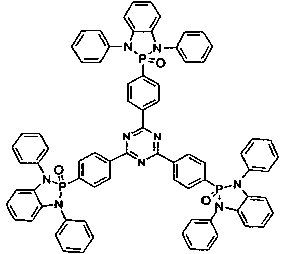 |
| (191) | (192) | (193) |
| 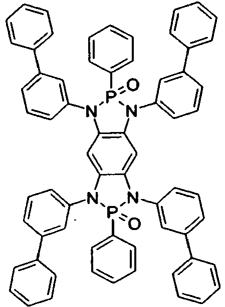 | 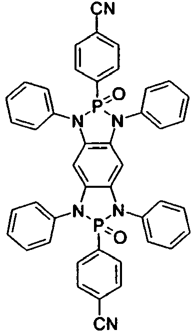 | 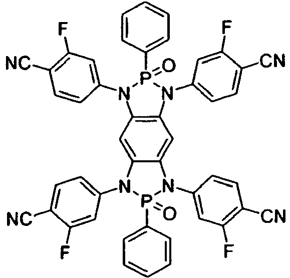 |
| (194) | (195) | (196) |
| 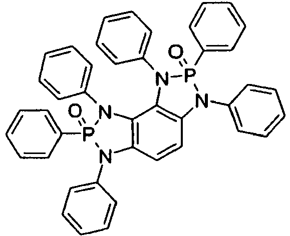 | 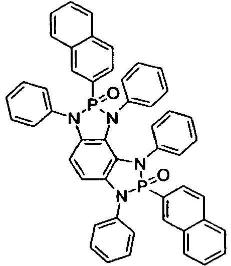 | 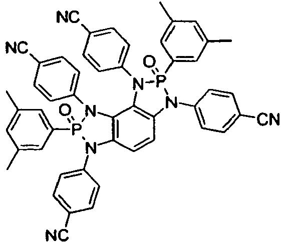 |
| (197) | (198) | (199) |
| 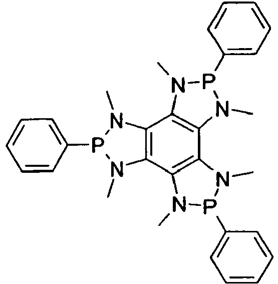 | 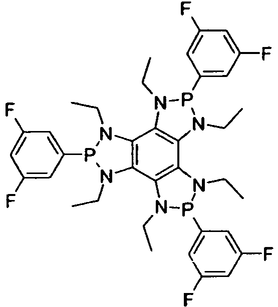 | 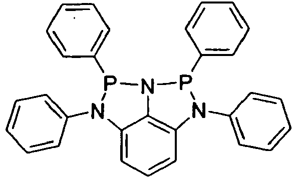 |
| (200) | (201) | (202) |
| 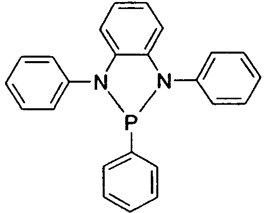 | 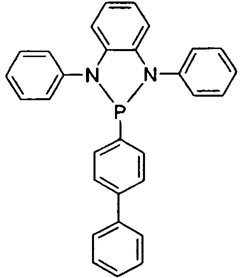 | 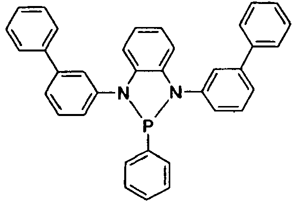 |
| (203) | (204) | (205) |
| 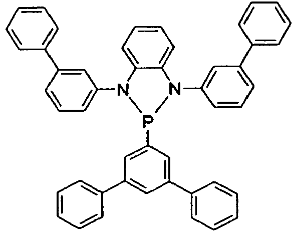 | 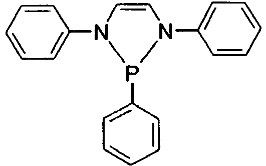 | 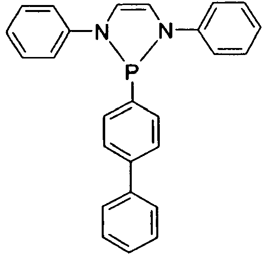 |
| (206) | (207) | (208) |
| 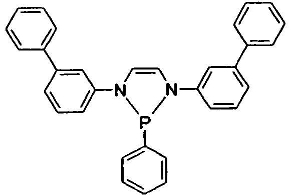 | 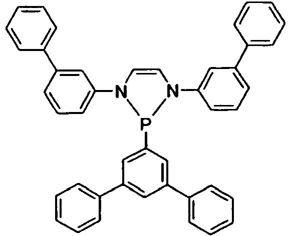 | 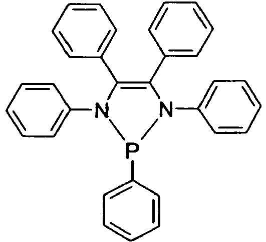 |
| (209) | (210) | (211) |
| 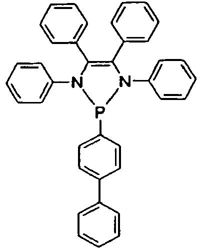 | 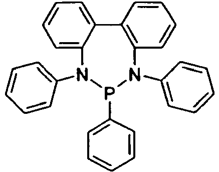 | 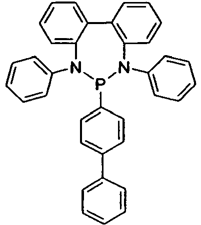 |
| (212) | (213) | (214) |
| 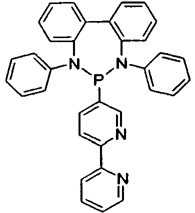 | 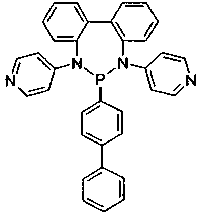 | 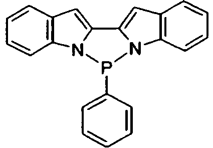 |
| (215) | (216) | (217) |
| 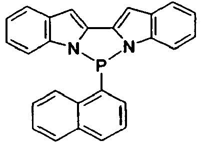 | 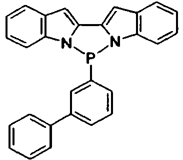 | 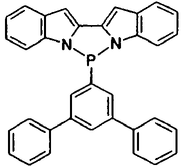 |
| (218) | (219) | (220) |
| 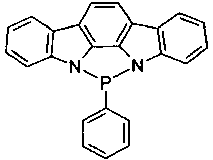 | 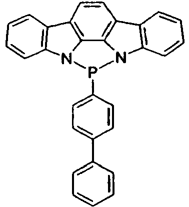 | 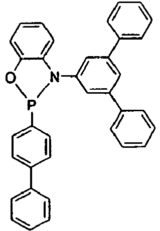 |
| (221) | (222) | (223) |
| 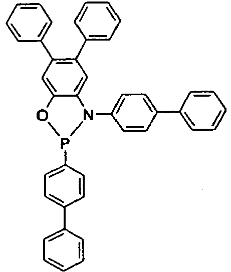 | 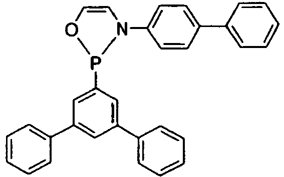 | 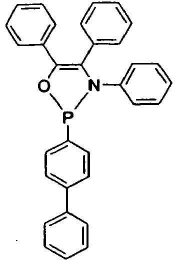 |
| (224) | (225) | (226) |
| 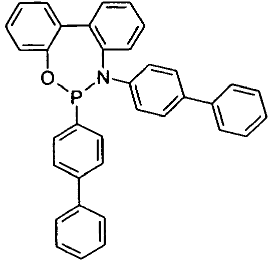 | 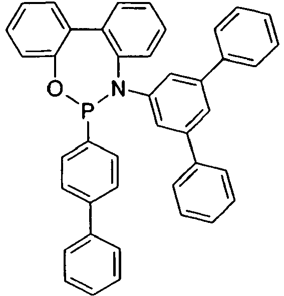 | 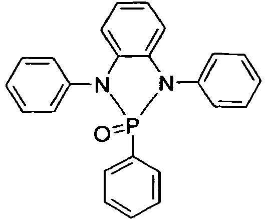 |
| (227) | (228) | (229) |
| 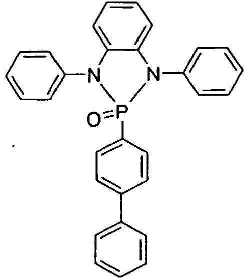 | 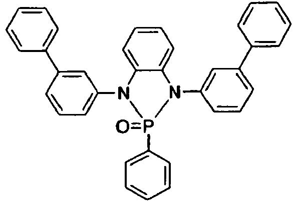 | 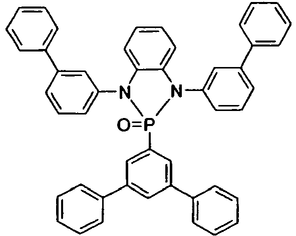 |
| (230) | (231) | (232) |
| 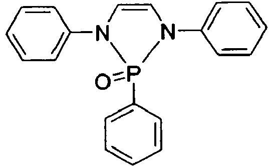 | 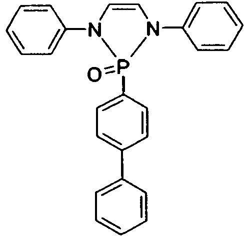 | 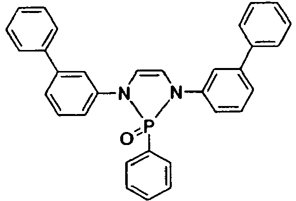 |
| (233) | (234) | (235) |
| 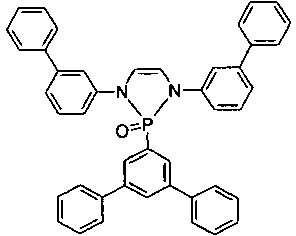 | 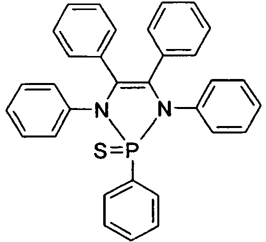 | 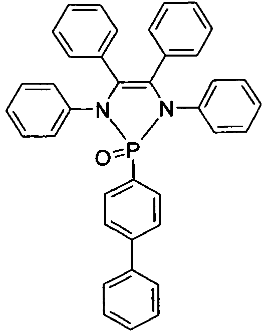 |
| (236) | (237) | (238) |
| 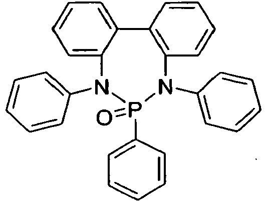 | 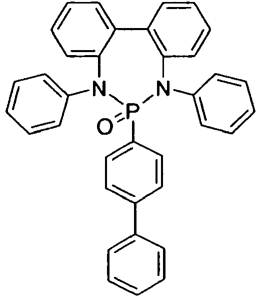 | 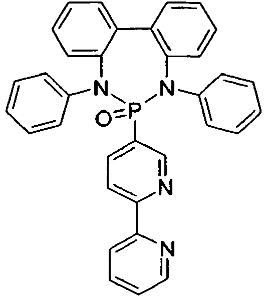 |
| (239) | (240) | (241) |
| 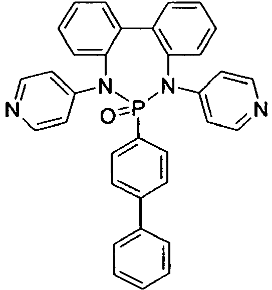 | 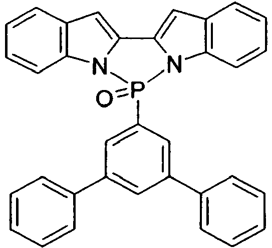 | 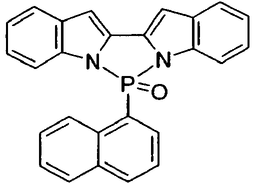 |
| (242) | (243) | (244) |
| 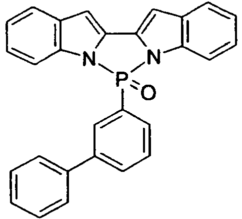 | 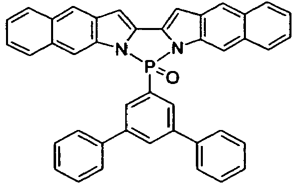 | 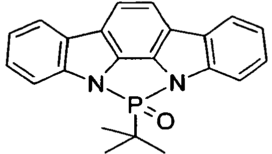 |
| (245) | (246) | (247) |
| 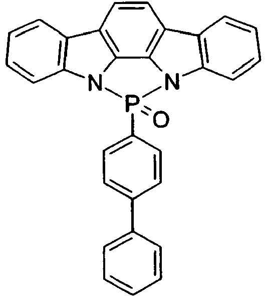 | 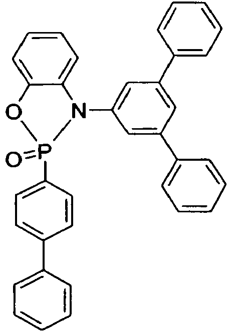 | 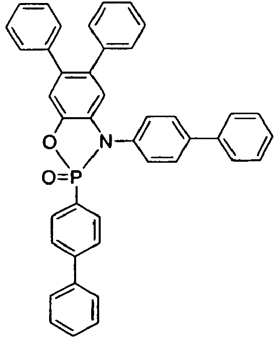 |
| (248) | (249) | (250) |
| 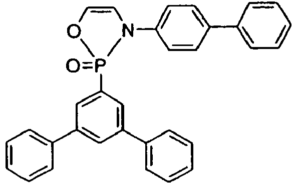 | 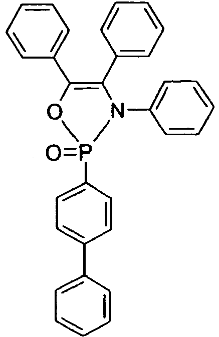 | 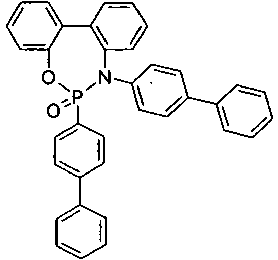 |
| (251) | (252) | (253) |
| 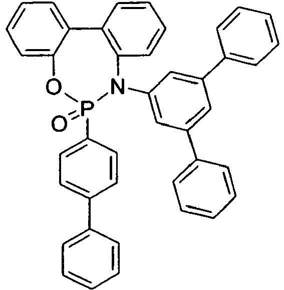 | 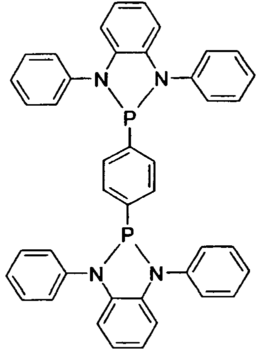 | 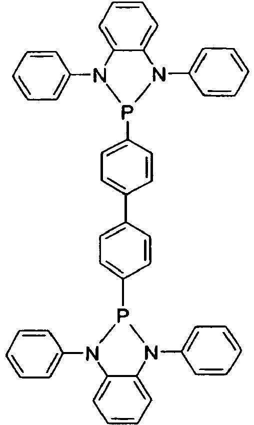 |
| (254) | (255) | (256) |
| 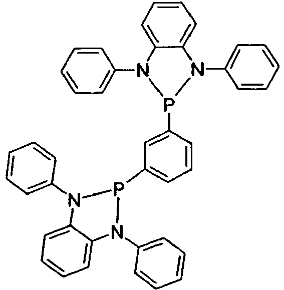 | 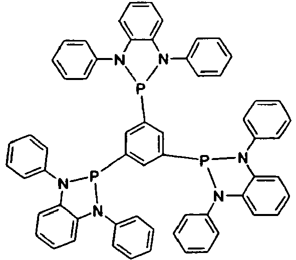 | 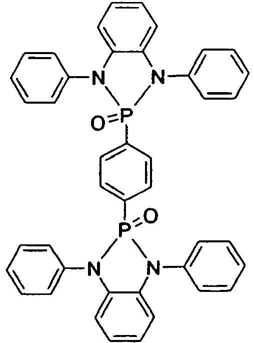 |
| (257) | (258) | (259) |
| 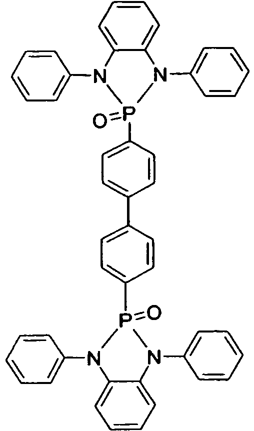 | 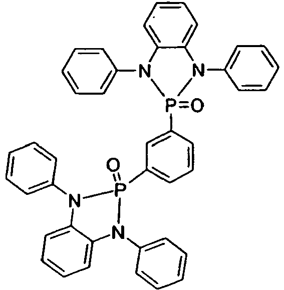 | 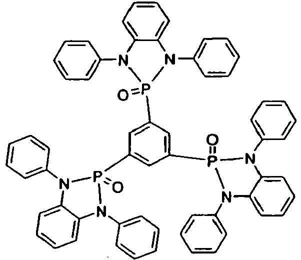 |
| (260) | (261) | (262) |
| 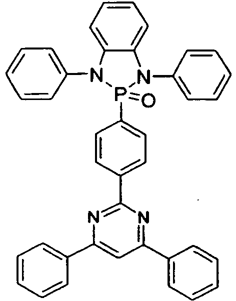 | 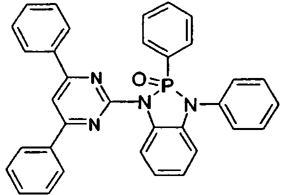 | 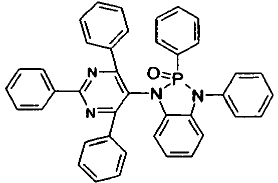 |
| (263) | (264) | (265) |
| 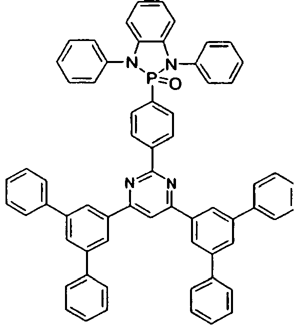 | 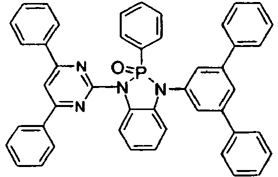 | 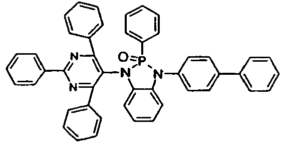 |
| (266) | (267) | (268) |
| 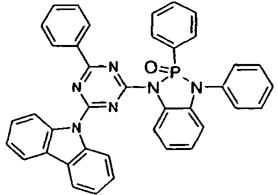 | 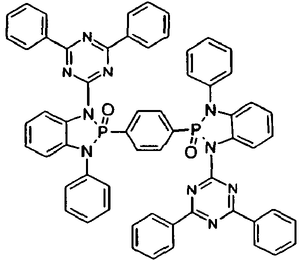 | 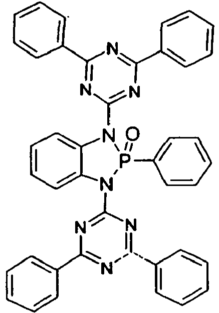 |
| (269) | (270) | (271) |
| 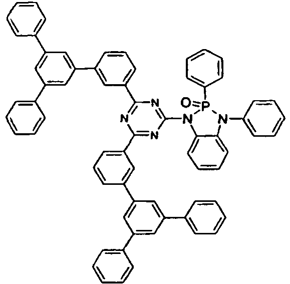 | 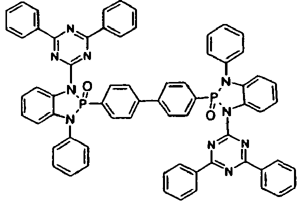 | 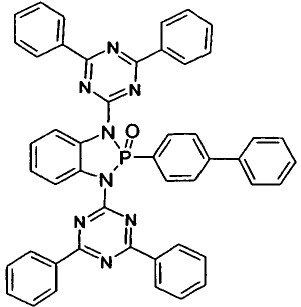 |
| (272) | (273) | (274) |

Wie oben aufgeführt, werden die Verbindungen gemäß Formel (1) in einer elektronischen Vorrichtung verwendet. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 05/011013).

Die Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. Formel (8) bis (18) als Matrixmaterial für fluoreszierende oder phosphoreszierende Emitter, insbesondere für phosphoreszierende Emitter, und/oder in einer Lochblockierschicht und/oder in einer Elektronentransportschicht und/oder in einer elektronenblockierenden bzw. exzitonenblockierenden Schicht und/oder in einer Lochtransportschicht. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. Formel (8) bis (18) als Matrixmaterial für eine fluoreszierende oder phosphoreszierende Verbindung in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. Formel (8) bis (18) als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Metallen der zweiten und dritten Übergangsmetallreihe, insbesondere alle Iridium- und Platinkomplexe als phosphoreszierende Verbindungen angesehen werden.

Die Mischung aus der Verbindung gemäß Formel (1) bzw. (8) bis (18) und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. (8) bis (18) bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1) bzw. Formel (8) bis (18) als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. Formel (8) bis (18) eingesetzt werden können, sind aromatische Ketone, aromatische Phosphinoxide oder aromatische Sulfoxide oder Sulfone, z. B. gemäß WO 04/013080, WO 04/093207, WO 06/005627 oder der nicht offen gelegten Anmeldung DE 102008033943.1, Triarylamine, Carbazolderivate, z. B. CBP (N,N-Biscarbazolylbiphenyl) oder die in WO 05/039246, US 2005/0069729, JP 2004/288381, EP 1205527 oder WO 08/086851 offenbarten Carbazolderivate, Indolocarbazolderivate, z. B. gemäß WO 07/063754 oder WO 08/056746, Azacarbazolderivate, z. B. gemäß EP 1617710, EP 1617711, EP 1731584, JP 2005/347160, bipolare Matrixmaterialien, z. B. gemäß WO 07/137725, Silane, z. B. gemäß WO 05/111172, Azaborole oder Boronester, z. B. gemäß WO 06/117052, Triazinderivate, z. B. gemäß der nicht offen gelegten Anmeldung DE 102008036982.9, WO 07/063754 oder WO 08/056746, Zinkkomplexe, z. B. gemäß EP 652273 oder WO 09/062578, oder Diazasilol- bzw. Tetraazasilol-Derivate, z. B. gemäß der nicht offen gelegten Anmeldung DE 102008056688.8.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 05/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 09/030981 beschrieben.

In einer weiteren bevorzugten Ausführungsform der Erfindung wird die Verbindung gemäß Formel (1) bzw. (8) bis (18) als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. Liq (Lithiumhydroxychinolinat).

In nochmals einer weiteren bevorzugten Ausführungsform der Erfindung wird die Verbindung gemäß Formel (1) bzw. (8) bis (18) in einer Lochblockierschicht eingesetzt. Unter einer Lochblockierschicht wird eine Schicht verstanden, die auf Kathodenseite direkt an eine emittierende Schicht angrenzt.

In nochmals einer weiteren Ausführungsform der Erfindung wird die Verbindung gemäß Formel (1) bzw. (8) bis (18) in einer Lochtransportschicht bzw. in einer Elektronenblockierschicht bzw. Exzitonenblockierschicht eingesetzt.

Es ist weiterhin möglich, die Verbindung gemäß Formel (1) bzw. (8) bis (18) sowohl in einer Lochblockierschicht bzw. Elektronentransportschicht als auch als Matrix in einer emittierenden Schicht und/oder sowohl in einer Lochtransportschicht bzw. Exzitonenblockierschicht als auch als Matrix in einer emittierenden Schicht zu verwenden.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. Formel (8) bis (18) einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light "Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die oben als bevorzugt genannten Verbindungen der Formel (1) sind neu und sind somit ebenfalls ein Gegenstand der vorliegenden Erfindung.

Gegenstand der Erfindung sind daher Verbindungen gemäß Formel (1'), 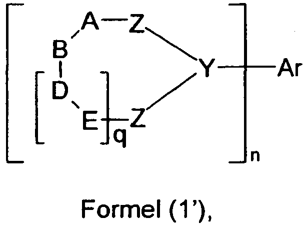 wobei für die verwendeten Symbole und Indizes gilt:
- A-B und D-E: ist jeweils gleich oder verschieden bei jedem Auftreten eine Einheit der folgenden Formel (2), (3), (4), (5) oder (6), 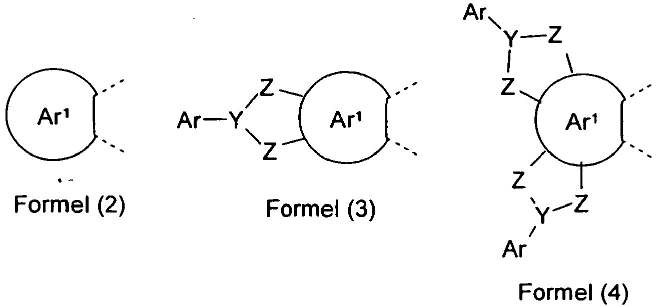 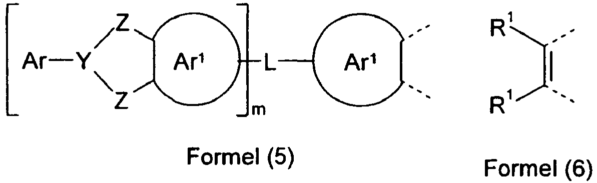 dabei stellt die gestrichelte Bindung jeweils die Bindung zu Z dar;
und
- Z: ist gleich oder verschieden bei jedem Auftreten N-R², O oder S, mit der Maßgabe, dass nicht beide Gruppen Z, die an dieselbe Gruppe Y binden, für O stehen;
oder
- A-Z und B-Z: ist jeweils gleich oder verschieden bei jedem Auftreten eine Einheit der folgenden Formel (7) und q = 0, 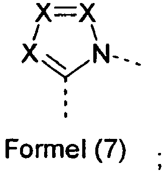 dabei stellt die gestrichelte Bindung in Formel (7) die Verknüpfung dieser Einheit in der Verbindung der Formel (1) dar, wobei der Stickstoff mit der Gruppe Y verknüpft ist;
- Y: ist bei jedem Auftreten gleich oder verschieden P(=O), As(=O), As(=S), Sb(=O), Sb(=S), Bi(=O) oder Bi(=S);
- Ar: ist bei jedem Auftreten gleich oder verschieden ein aromatisches oder heteroaromatisches Ringsystem mit 5 bis 60 aromatischen Ringatomen, welches durch einen oder mehrere Reste R¹ substituiert sein kann;
- Ar¹: ist bei jedem Auftreten gleich oder verschieden eine Aryl- oder Heteroarylgruppe mit 5 bis 16 aromatischen Ringatomen, welche durch einen oder mehrere Reste R¹ substituiert sein kann;
- X: ist bei jedem Auftreten gleich oder verschieden CR¹ oder N;
- L: ist eine Einfachbindung oder eine bivalente, trivalente oder tetravalente Gruppe;
- R¹: ist bei jedem Auftreten gleich oder verschieden ausgewählt aus der Gruppe bestehend aus H, D, F, Cl, Br, I, CN, NO₂, N(R³)₂, C(=O)R³, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C=C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
- R²: ist bei jedem Auftreten gleich oder verschieden ausgewählt aus der Gruppe bestehend aus einer geradkettigen Alkylgruppe mit 1 bis 40 C-Atomen oder einer verzweigten oder cyclischen Alkylgruppe mit 3 bis 40 C-Atomen, die jeweils mit einem oder mehreren Resten R³ substituiert sein kann, wobei eine oder mehrere nicht-benachbarte CH₂-Gruppen durch R³C=CR³, C≡C oder C=O ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, oder einer Kombination dieser Systeme; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein;
- R³: ist ausgewählt aus der Gruppe bestehend aus H, D, F, CN, aliphatischem Kohlenwasserstoffrest mit 1 bis 20 C-Atomen, aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches, aromatisches oder heteroaromatisches Ringsystem bilden können;
- n: ist 1 bis 10, bevorzugt 1, 2, 3, 4, 5 oder 6;
- m: ist 1, wenn L eine Einfachbindung oder eine bivalente Gruppe ist, oder ist 2, wenn L eine trivalente Gruppe ist, oder ist 3, wenn L eine tetravalente Gruppe ist;
- q: ist bei jedem Auftreten gleich oder verschieden 0 oder 1;
dabei sind die folgenden Verbindungen von der Erfindung ausgeschlossen:

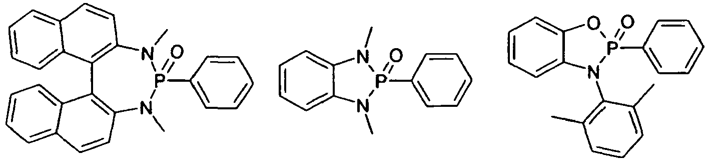

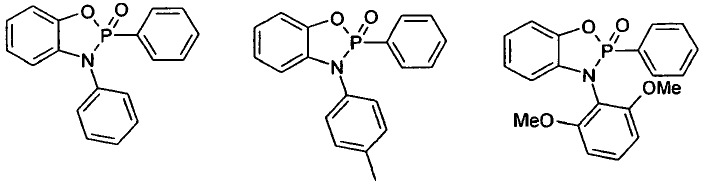

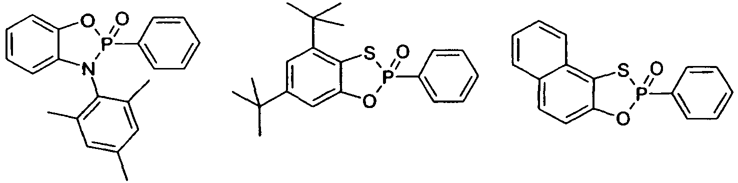

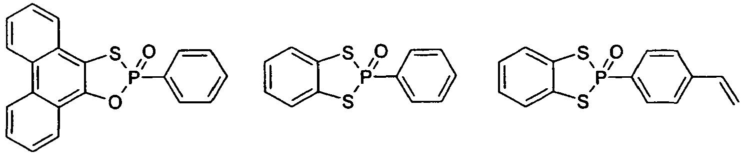

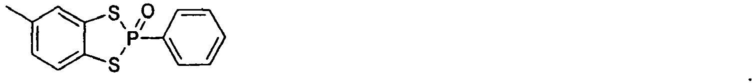

Für die erfindungsgemäßen Verbindungen gemäß Formel (1') gelten dieselben Bevorzugungen, wie oben bereits für die elektronische Vorrichtung aufgeführt.

Ein weiterer Gegenstand der Erfindung ist daher die Verwendung der erfindungsgemäßen Verbindungen in einer elektronischen Vorrichtung.

Gegenstand der Erfindung sind weiterhin Verbindungen gemäß Formel (39) sowie deren Verwendung in elektronischen Vorrichtungen und elektronische Vorrichtungen, insbesondere organische Elektrolumineszenzvorrichtungen, enthaltend diese Verbindungen: 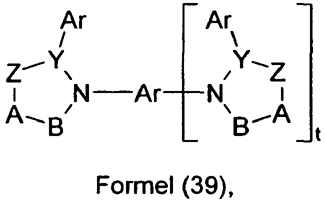 wobei die verwendeten Symbole und Indizes oben für Formel (1) genannten Bedeutungen haben und t eine ganze Zahl von 1 bis 10, bevorzugt 1, 2, 3, 4, 5 oder 6, darstellt. Dabei gelten die oben für Formel (1) aufgeführten bevorzugten Ausführungsformen ebenso für Verbindungen der Formel (39).

Zur Herstellung der Verbindungen gemäß Formel (1) bzw. der erfindungsgemäßen Verbindungen hat sich das im Folgenden beschriebene Verfahren als besonders gut geeignet herausgestellt. Dazu wird entweder ein ortho-Dibrom-substituierter Aromat mit einem primären Amin oder ein ortho-Diamino-substituierter Aromat mit einem Arylbromid in einer Hartwig-Buchwald-Kupplung umgesetzt, wie in Schema 1 als Weg A bzw. Weg B aufgeführt. Das resultierende Diamin wird mit einem aromatischen Phosphonsäurechlorid bzw. einem aromatischen Bis(phosphonsäurechlorid) zur entsprechenden Verbindung gemäß Formel (1) bzw. (1') umgesetzt. Die in Schema 1 exemplarisch gezeigte Synthese lässt sich selbstverständlich ganz analog mit anderen aromatischen Gruppen bzw. mit unterschiedlich substituierten Gruppen durchführen.

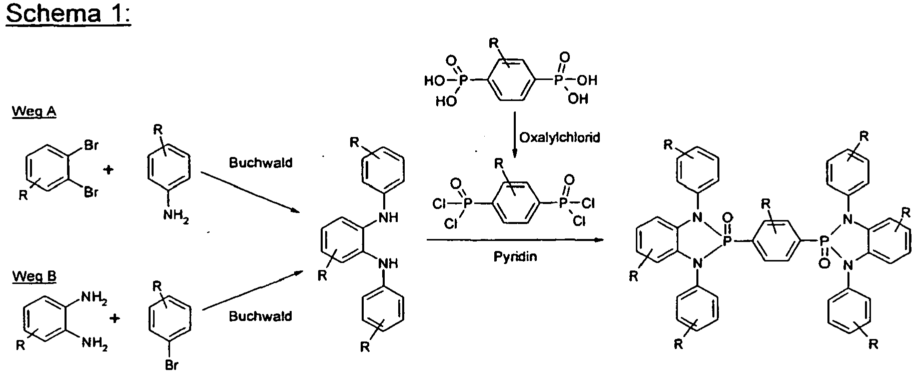

Die Synthese von Verbindungen gemäß Formel (1), welche an den beiden ortho-ständigen Stickstoffatomen unterschiedlich substituiert sind, ist in Schema 2 gezeigt. Diese Verbindungen sind zugänglich durch den Einsatz eines ortho-Diamino-substituierten Aromaten, in welchem eine der beiden Aminogruppen substituiert ist.

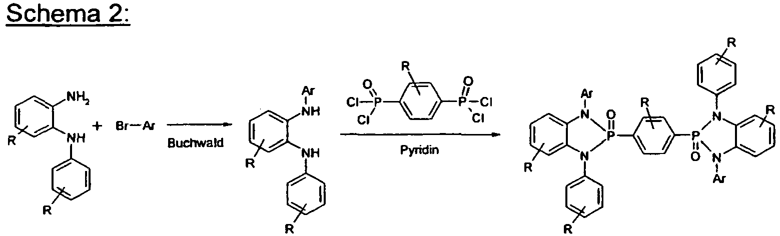

Ein weiterer Gegenstand der vorliegenden Erfindung ist ein Verfahren zur Herstellung der oben aufgeführten erfindungsgemäßen Verbindungen durch Umsetzung eines ortho-Diamino-substituierten Aromaten, wobei die Aminogruppen unsubstituiert oder bevorzugt monosubstituiert sind, mit einem aromatischen Phosphonsäurechloridderivat bzw. einem aromatischen Oligophosphonsäurechloridderivat.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei ein oder mehrere Bindungen der erfindungsgemäßen Verbindung zum Polymer, Oligomer oder Dendrimer vorhanden sind. Je nach Verknüpfung der erfindungsgemäßen Verbindung bildet diese daher eine Seitenkette des Oligomers oder Polymers oder ist in der Hauptkette verknüpft. Die Polymere, Oligomere oder Dendrimere können konjugiert, teilkonjugiert oder nicht-konjugiert sein. Die Oligomere oder Polymeren können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Homopolymere oder Copolymere, wobei die Einheiten gemäß Formel (1) bzw. (8) bis (18) bzw. (39) zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 00/22026), Spirobifluorenen (z. B. gemäß EP 707020, EP 894107 oder WO 06/061181), Para-phenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder WO 07/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten. Außerdem können die Polymere entweder einpolymerisiert oder als Blend eingemischt Triplett-Emitter enthalten. Gerade die Kombination von Einheiten gemäß den Formeln (1) bzw. (8) bis (18) mit TriplettEmittern führt zu besonders guten Ergebnissen.

Eine weitere Möglichkeit der Polymerisation besteht in der Umsetzung einer Verbindung Hal₂Y-Ar-YHal₂, wobei Hal für Cl, Br oder I steht, mit einem entsprechenden Tetramin. Dies führt zu Verbindungen, Oligomeren und Polymeren gemäß der folgenden Formel (37). Ganz analog ist die Bildung von Verbindungen, Oligomeren und Polymeren gemäß der folgenden Formel (38) möglich. 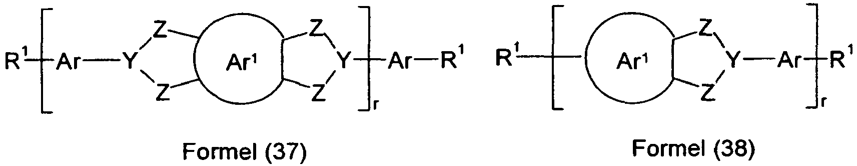 wobei die verwendeten Symbole die oben genannten Bedeutungen aufweisen und r für eine ganze Zahl zwischen 2 und 1000000 steht. Diese Verbindungen, Oligomere und Polymere sind ebenfalls Gegenstand der vorliegenden Erfindung. Dabei gelten für die in Formel (37) und (38) aufgeführten Symbole die oben genannten Bevorzugungen. Insbesondere bevorzugt ist die Gruppe Ar¹ gleich oder verschieden bei jedem Auftreten gewählt aus 1,2,4,5-verknüpftem Benzol oder 1,2,3,4-verknüpftem Benzol.

Weiterhin können die Verbindungen gemäß Formel (1) bzw. (1') auch weiter funktionalisiert werden und so zu erweiterten Strukturen umgesetzt werden. Hier ist als Beispiel die Umsetzung mit Arylboronsäuren gemäß SUZUKI oder mit primären oder sekundären Aminen gemäß HARTWIG-BUCHWALD zu nennen. So können die Verbindungen gemäß Formel (1) bzw. (1') auch direkt an phosphoreszierende Metallkomplexe oder auch an andere Metallkomplexe gebunden werden.

Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen bzw. Verbindungen gemäß Formeln (1) bzw. Formel (8) bis (18), eingesetzt als Matrixmaterial für fluoreszierende oder phosphoreszierende Emitter führen zu sehr hohen Effizienzen sowie zu langen Lebensdauern. Dies gilt insbesondere, wenn die Verbindungen als Matrixmaterial für einen phosphoreszierenden Emitter eingesetzt werden. Dabei werden wesentlich bessere Effizienzen, insbesondere Leistungseffizienzen, und Lebensdauern erhalten als bei Einsatz von strukturell ähnlichen Phosphinoxiden als Matrixmaterialien.
2. Die erfindungsgemäßen Verbindungen bzw. Verbindungen gemäß Formel (1) bzw. (8) bis (18) eignen sich nicht nur als Matrix für grün und rot phosphoreszierende Verbindungen, sondern insbesondere auch für blau phosphoreszierende Verbindungen.
3. Im Gegensatz zu vielen Verbindungen gemäß dem Stand der Technik, die der teilweisen oder vollständigen pyrolytischen Zersetzung bei Sublimation unterliegen, weisen die erfindungsgemäßen Verbindungen eine hohe thermische Stabilität auf.
4. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatzspannungen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Komplexe herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Als Ausgangsverbindung können z. B. 1,4-Bis(phosphonsäure)benzol (Inorganic Chemistry 1996, 35(17), 4942-4949), N,N'-Diphenyl-1,2-benzoldiamin (Organic Letters 2007, 9(7), 1339-1342) oder N-Phenyl-o-phenylendiamin (Indian Journal of Pharmaceutical Sciences 2003, 65(2), 135-138) dienen.

### Beispiel 1a: Synthese von 1,4-Bis(phosphonsäurechlorid)benzol

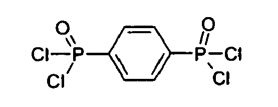

55,2 g (232 mmol) 1,4-Bis(phosphonsäure)benzol werden in 1400 ml Methylenchlorid vorgelegt und mit 10 Tropfen DMF versetzt. Bei Raumtemperatur werden 86,3 ml (1020 mmol) Oxalylchlorid in 400 ml Methylenchlorid zugetropft und bei 45 °C 5 h gerührt. Das Lösungsmittel wird im Vakuum entzogen und das Produkt unter Schutzgas in Hexan umkristallisiert. Ausbeute: 70 g (227 mmol), 98 %.

Analog können folgende Verbindungen erhalten werden:

| **Bsp.** | **Edukt** | **Produkt** | **Ausbeute** |
|---|---|---|---|
| 1b | 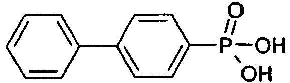 | 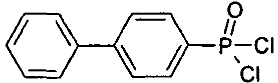 | 89 % |
| 1c | 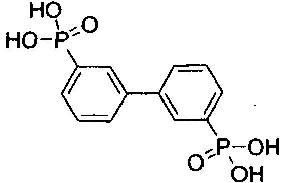 | 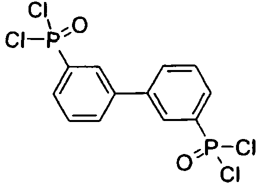 | 95 % |
| 1d | 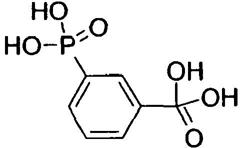 | 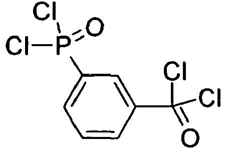 | 84 % |

### Beispiel 2: Allgemeine Synthese von N,N'-Diaryl-1,2-benzotdiamin

In 660 ml entgastes Toluol wird 1,06 g (4,75 mmol) Pd(OAc)₂ und 14,46 ml (14,46 mmol) Tri-tert-butylphosphin (1M Lösung in Toluol) zugegeben und 5 min. gerührt. Dann wird die Lösung mit 240 mmol des 1,2-Dibrombenzolderivats, 505 mmol des Arylamins und 67,22 g (700 mmol) Natrium-*tert-*butylat versetzt, nachentgast und 10 h bei 140°C unter Schutzgasatmosphäre gerührt. Nach dem Abkühlen wird die Lösung mit 600 ml NH₄Cl-Lösung und 150 ml Essigsäureethylester versetzt, Phasen getrennt, mit Wasser gewaschen, über MgSO₄ getrocknet und eingeengt. Der Feststoff wird in Toluol gelöst und über Celite abfiltriert. Das Rohprodukt wird mit Heptan heiß ausgerührt.

### Beispiel 3: Synthese von 4,5-Dimethyl-N,N'-diphenyl-1,2-benzoldiamin

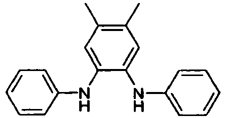

Die Synthese erfolgt nach der allgemeinen Vorschrift gemäß Beispiel 2 aus 63,3 g (240 mmol) 1,2-Dibrom-4,5 dimethylbenzol und 46 ml (505 mmol) Anilin. Der ausgefallene Feststoff wird aus Toluol/Acetonitril (5:1) umkristallisiert und der Rückstand mit MeOH gewaschen. Dabei erhält man 65 g (223 mmol) eines kristallinen Feststoffs. Die Gesamtausbeute beträgt 93 %.

### Beispiel 4: Synthese von N,N'-Ditolyl-1,2-benzoldiamin

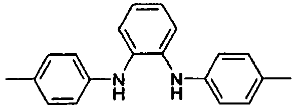

Die Synthese erfolgt nach der allgemeinen Vorschrift gemäß Beispiel 2 aus 56,6 g (240 mmol) 1,2-Dibrombenzol und 54 ml (505 mmol) p-Toluidin. Der ausgefallene Feststoff wird aus Toluol/Acetonitril (5:1) umkristallisiert und der Rückstand mit MeOH gewaschen. Dabei erhält man 75 g (262 mmol) eines kristallinen Feststoffs. Die Gesamtausbeute beträgt 98.%.

### Beispiel 5: Synthese von N,N'-Di-o-tolyl-1,2-benzolamin

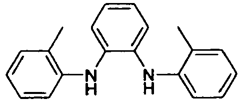

Die Synthese erfolgt nach der allgemeinen Vorschrift gemäß Beispiel 2 aus 56,6 g (240 mmol) 1,2-Dibrombenzol und 54 ml (505 mmol) o-Toluidin. Der ausgefallene Feststoff wird aus Toluol/Acetonitril (5:1) umkristallisiert und der Rückstand mit MeOH gewaschen. Dabei erhält man 68 g (237 mmol) eines kristallinen Feststoffs. Die Gesamtausbeute beträgt 90 %.

### Beispiel 6: Synthese von 4,5-Dimethyl-N,N'-di-p-tolyl-1,2-benzoldiamin

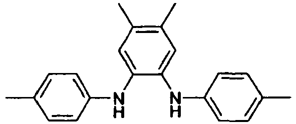

Die Synthese erfolgt nach der allgemeinen Vorschrift gemäß Beispiel 2 aus 63,3 g (240 mmol) 1,2-Dibrom-4,5 dimethylbenzol und 54 ml (505 mmol) p-Toluidin. Der ausgefallene Feststoff wird aus Toluol/Acetonitril (5:1) umkristallisiert und der Rückstand mit MeOH gewaschen. Dabei erhält man 69 g (218 mmol) eines kristallinen Feststoffs. Die Gesamtausbeute beträgt 91 %.

### Beispiel 7: Synthese von N,N'-Bis(biphenyl-4-yl)-1,2-benzol-diamin

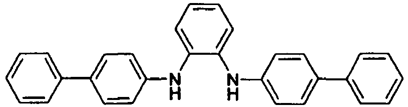

Die Synthese erfolgt nach der allgemeinen Vorschrift gemäß Beispiel 2 aus 56,6 g (240 mmol) 1,2-Dibrombenzol und 85,4g (505 mmol) 4-Aminobiphenyl. Der ausgefallene Feststoff wird aus Toluol/Acetonitril (5:1) umkristallisiert und der Rückstand mit MeOH gewaschen. Dabei erhält man 78 g (189 mmol) eines kristallinen Feststoffs. Die Gesamtausbeute beträgt 80 %.

### Beispiel 8: Synthese von 4,5-Dimethyl-N,N'- bis(biphenyl-4-yl)-1,2-benzoldiamin

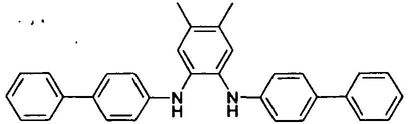

Die Synthese erfolgt nach der allgemeinen Vorschrift gemäß Beispiel 2 aus 63,3 g (240 mmol) 1,2-Dibrom-4,5 dimethylbenzol und 85,4 g (505 mmol) 4-Aminobiphenyl. Der ausgefallene Feststoff wird aus Toluol/Acetonitril (5:1) umkristallisiert und der Rückstand mit MeOH gewaschen. Dabei erhält man 80,3 g (182 mmol) eines kristallinen Feststoffs. Die Gesamtausbeute beträgt 76 %.

### Beispiel 9: Synthese von N-Biphenyl-4-yl-N'-phenyl-1,2-benzoldiamin

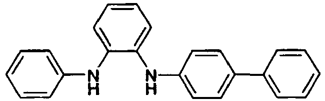

In 660 ml entgastes Toluol wird 0,35 g (1,58 mmol) Pd(OAc)₂ und 4,8 ml (4,86 mmol) Tri-*tert*-butylphosphin (1 M Lösung in Toluol) zugegeben und 5 min. gerührt. Dann wird die Lösung mit 37,2 g (160 mmol) 4-Brombiphenyl, 29,4g (160 mmol) N-Phenyl-o-phenylendiamin und 22,4 g (233 mmol) Natrium-*tert*-butyiat versetzt, nachentgast und 10 h bei 140 °C unter Schutzgasatmosphäre gerührt. Nach dem Abkühlen wird die Lösung mit 200 ml NH₄Cl-Lösung und 50 ml Essigsäureethylester versetzt, Phasen getrennt, mit Wasser gewaschen, über MgSO₄ getrocknet und eingeengt. Der Feststoff wird in Toluol gelöst und über Celite abfiltriert. Das Rohprodukt wird mit Heptan heiß ausgerührt und mit MeOH gewaschen. Dabei erhält man 47 g (140 mmol) eines kristallinen Feststoffs. Die Gesamtausbeute beträgt 80 %.

### Beispiel 10: Allgemeine Synthese der Diazaphosphole

158 mmol des N,N'-Diaryl-1,2-benzoldiamins werden in 500 ml Pyridin gelöst und auf 0 °C gekühlt. Zu dieser Lösung werden bei 0 °C 74 mmol des 1,4-Bis(phosphonsäurechlorid)benzols, gelöst in 1000 ml Toluol, unter gutem Rühren tropfenweise zugetropft, 1 h gerührt und dann 24 h unter Rückfluss erhitzt. Das Lösungsmittel wird im Vakuum abdestilliert, der Feststoff in Essigsäureethylester ausgekocht, abgesaugt, einmal mit 100 ml Essigsäureester gewaschen und anschließend aus Dioxan umkristallisiert.

### Beispiel 11: Synthese von H3

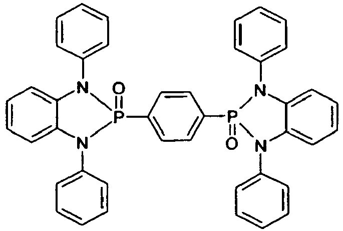

Die Synthese erfolgt nach der allgemeinen Vorschrift gemäß Beispiel 10 aus 42 g (158 mmol) N,N'-diphenyl-1,2-benzoldiamin und 23 g (74 mmol) 1,4-Bis(phosphonsäurechlorid)benzol. Der erhaltene Feststoff wird aus Dioxan mehrfach umkristallisiert. Ausbeute: 34.4 g (50 mmol), 68 %, Reinheit 99.9 %ig (HPLC).

### Beispiel 12: Synthese von H4

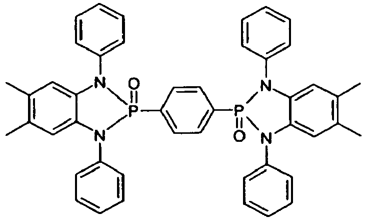

Die Synthese erfolgt nach der allgemeinen Vorschrift gemäß Beispiel 10 aus 45,5 g (158 mmol) 4,5-Dimethyl-N,N'-diphenyl-1,2-benzoldiamin und 23 g (74 mmol) 1,4-Bis(phosphonsäurechlorid)benzol. Der erhaltene Feststoff wird aus Dioxan mehrfach umkristallisiert. Ausbeute: 35,7 g (48 mmol), 65 %, Reinheit 99.9 % ig (HPLC).

### Beispiel 13: Synthese von H5

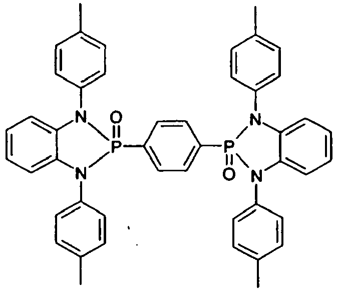

Die Synthese erfolgt nach der allgemeinen Vorschrift gemäß Beispiel 10 aus 45,5 g (158 mmol) N,N'-Ditolyl-1,2-benzoldiamin und 23 g (74 mmol) 1,4-Bis(phosphonsäurechlorid)benzol. Der erhaltene Feststoff wird aus Dioxan mehrfach umkristallisiert. Ausbeute: 37,9 g (51 mmol), 69 %, Reinheit 99.9 % ig (HPLC).

### Beispiel 14: Synthese von H6

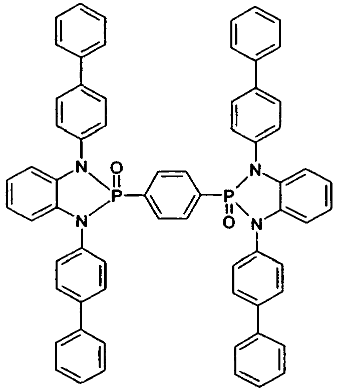

Die Synthese erfolgt nach der allgemeinen Vorschrift gemäß Beispiel 10 aus 65 g (158 mmol) N,N'-Bis(biphenyl-4-yl)-1,2-benzoldiamin und 23 g (74 mmol) 1,4-Bis(phosphonsäurechlorid)benzol. Der erhaltene Feststoff wird aus Dioxan mehrfach umkristallisiert. Ausbeute: 43,9 g (44,3 mmol), 60 %, Reinheit 99.9 %ig (HPLC).

### Beispiel 15: Synthese von H7

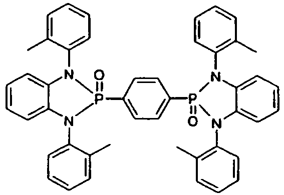

Die Synthese erfolgt nach der allgemeinen Vorschrift gemäß Beispiel 10 aus 45,5 g (158 mmol) N,N'-Di-o-tolyl-1,2-benzoldiamin und 23 g (74 mmol) 1,4-Bis(phosphonsäurechlorid)benzol. Der erhaltene Feststoff wird aus Dioxan mehrfach umkristallisiert. Ausbeute: 34,6 g (46,5 mmol), 63 %, Reinheit 99.9 % ig (HPLC).

### Beispiel 16: Synthese von H8

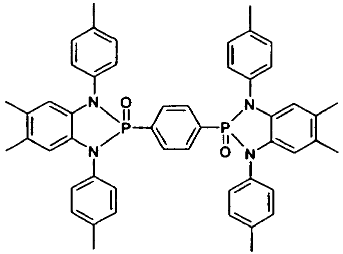

Die Synthese erfolgt nach der allgemeinen Vorschrift gemäß Beispiel 10 aus 50,6 g (158 mmol) 4,5-Dimethyl-N,N'-di-p-tolyl-1,2-benzoldiamin und 23 g (74 mmol) 1,4-Bis(phosphonsäurechlorid)benzol. Der erhaltene Feststoff wird aus Dioxan mehrfach umkristallisiert. Ausbeute: 35,4 g (44,3 mmol), 60%, Reinheit 99.9 %ig (HPLC).

### Beispiel 17: Synthese von H9

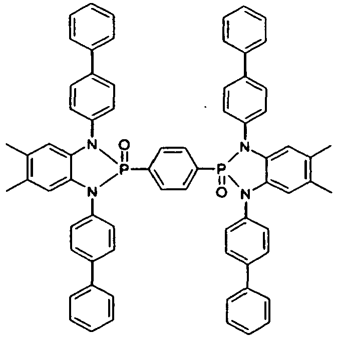

Die Synthese erfolgt nach der allgemeinen Vorschrift gemäß Beispiel 10 aus 69,6 g (158 mmol) 4,5-Dimethyl-N,N'-bis(biphenyl-4-yl)-1,2-benzoldiamin und 23 g (74 mmol) 1,4-Bis(phosphonsäurechlorid)benzol. Der erhaltene Feststoff wird aus Dioxan mehrfach umkristallisiert. Ausbeute: 48,8 g (46,6 mmol), 63 %, Reinheit 99.9 %ig (HPLC).

### Beispiel 18: Synthese von H10

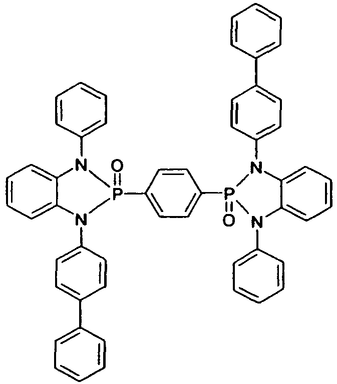

Die Synthese erfolgt nach der allgemeinen Vorschrift gemäß Beispiel 9 aus 53,1 g (158 mmol) N-Biphenyl-4-yl-N'-phenyl-1,2-benzoldiamin und 23 g (74 mmol) 1,4-Bis(phosphonsäurechlorid)benzol. Der erhaltene Feststoff wird aus Dioxan mehrfach umkristallisiert. Ausbeute: 50,2 g (42 mmol), 81 %, Reinheit 99.9 %ig (HPLC).

Analog werden weiterhin folgende Verbindungen erhalten:

| **Bsp.** | **Edukt** | **Produkt** | **Ausbeute** |
|---|---|---|---|
| 19 | 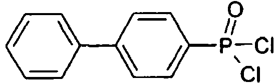 | 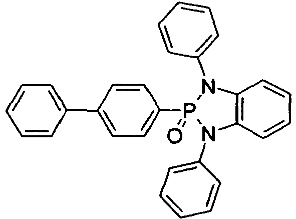 | 67 % |
| 20 | 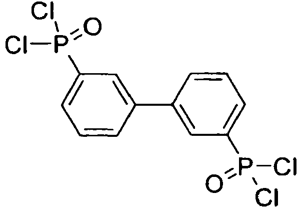 | 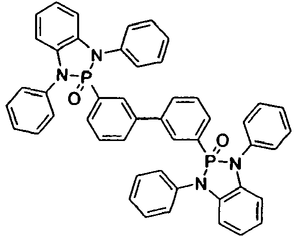 | 50 % |
| 21 | 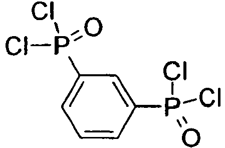 | 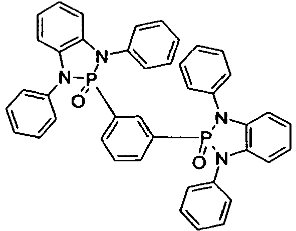 | 65 % |

Werden anstelle der Phosphonsäurechloride die entsprechenden Dichlorphosphine eingesetzt, so werden folgende Verbindungen erhalten:

| **Bsp.** | **Edukt** | **Produkt** | **Ausbeute** |
|---|---|---|---|
| 22 | 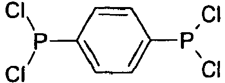 | 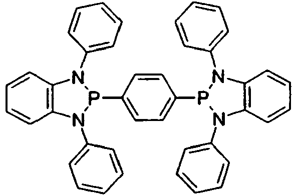 | 71% |
| 23 | 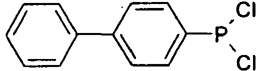 | 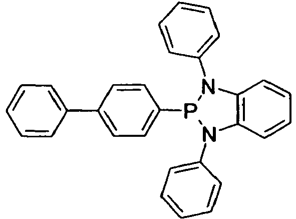 | 76% |
| 24 | 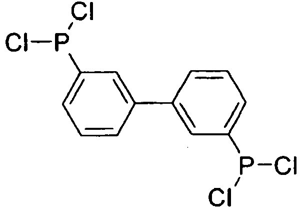 | 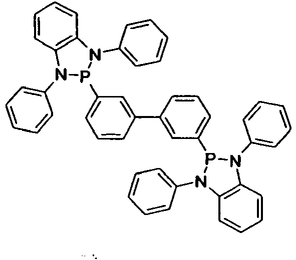 | 44 % |
| 25 | 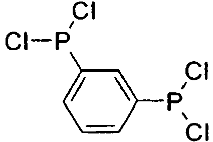 | 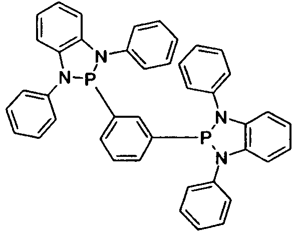 | 46% |

### Beispiel 26: Synthese N,N'-Dipyrid-4-yl-1,2-benzoldiamin

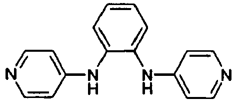

Die Synthese erfolgt nach der allgemeinen Vorschrift gemäß Beispiel 2 aus 56.6 g (240 mmol) 1,2-Dibrom-benzol und 47.5 g (505 mmol) 4-Aminopyridin. Der ausgefallene Feststoff wird aus Toluol/Acetonitril (5:1) umkristallisiert und der Rückstand mit MeOH gewaschen. Dabei erhält man 46.2 g (176 mmol) eines kristallinen Feststoffs. Die Gesamtausbeute beträgt 73 %.

### Beispiel 27: Synthese N-phenyl-N'-4,6-diphenyl-1,3,5-triazin-2-yl-1,2-benzoldiamin

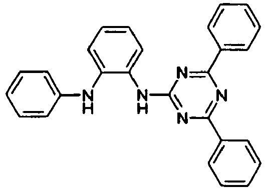

Eine Lösung von 18.4 g (100 mmol) N-Phenyl-o-phenylendiamin in 200 ml THF wird mit 51 ml (300 mmol) Ethyl-di-iso-propylamin und anschließend tropfenweise mit einer Lösung von 29.5 g (110 mmol) 2-Chlor-4,6-diphenyl-1,3,5-triazin in 100 ml THF versetzt. Nach 1 h rühren bei Raumtemperatur wird die Reaktionsmischung 6 h unter Rückfluss erhitzt. Nach Erkalten wird das THF im Vakuum entfernt, der Rückstand wird in 50 ml Dichlormethan gelöst und tropfenweise mit 300 ml Methanol versetzt. Nach 12 h Rühren wird vom Feststoff abgesaugt, dieser wird mit MeOH gewaschen und getrocknet. Die Gesamtausbeute beträgt 27.8 g (67 mmol), 67 %.

### Beispiel 28: Synthese von H11

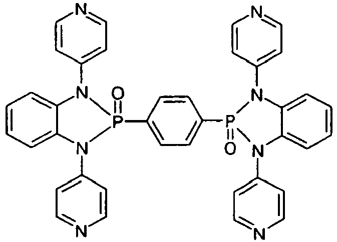

Die Synthese erfolgt nach der allgemeinen Vorschrift gemäß Beispiel 10 aus 41.4 g (158 mmol) N,N'-Dipyrid-4-yl-1,2-benzoldiamin und 23 g (74 mmol) 1,4-Bis(phosphonsäurechlorid)benzol. Der erhaltene Feststoff wird aus Dioxan mehrfach umkristallisiert. Ausbeute: 39.4 g (57 mmol), 77 %, Reinheit 99.9 %ig (HPLC).

### Beispiel 29: Synthese von H12

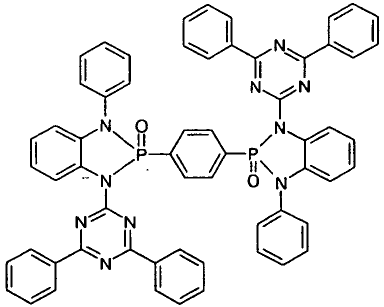

Die Synthese erfolgt nach der allgemeinen Vorschrift gemäß Beispiel 10 aus 65.6 g (158 mmol) N-phenyl-N'-4,6-diphenyl-1,3,5-triazin-2-yl-1,2-benzoldiamin und 23 g (74 mmol) 1,4-Bis(phosphonsäurechlorid)benzol. Der erhaltene Feststoff wird aus Chlorbenzol mehrfach umkristallisiert. Ausbeute: 42.9 g (43 mmol), 58 %, Reinheit 99.9 %ig (HPLC).

### Beispiel 30: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, verwendete Materialien) angepasst wird.

In den folgenden Beispielen 31 bis 50 (siehe Tabellen 1 und 2) werden die Ergebnisse verschiedener OLEDs vorgestellt. Glasplättchen, die mit strukturiertem ITO (Indium Zinn Oxid) der Dicke 150 nm beschichtet sind, werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Die OLEDs haben prinzipiell den folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs verwendeten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:CBP:TER1 (55%:35%:10%) bedeutet hierbei, dass das Material H1 in einem Volumenanteil von 55 %, CBP in einem Volumenanteil von 35 % und TER1 in einem Volumenanteil von 10 % in der Schicht vorliegt. Analog kann auch die Elektronentransportschicht aus einer Mischung zweier Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Helligkeits-Kennlinien (IUL-Kennlinien), sowie die Lebensdauer bestimmt. Als Lebensdauer wird die Zeit definiert, nach der die Leuchtdichte von einer bestimmten Startleuchtdichte I₀ auf einen gewissen Anteil abgesunken ist. Die Angabe LD80 bedeutet, dass es sich bei der genannten Lebensdauer um die Zeit handelt, bei der die Leuchtdichte auf 0.8·I₀ (auf 80 %) abgefallen ist, also von z. B. 4000 cd/m² auf 3200 cd/m².

Die erfindungsgemäßen Verbindungen lassen sich unter anderem als Matrixmaterialien (Hostmaterialien, Wirtsmaterialen) für phosphoreszierende Dotanden einsetzen. Hierbei kommen die erfindungsgemäßen Verbindungen H3, H4, H5, H6 und H7 zum Einsatz. Als Vergleich gemäß dem Stand der Technik werden die Verbindungen H1, H2 und H8 verwendet. Es werden OLEDs mit dem grün emittierenden Dotanden TEG1, dem blau emittierenden Dotanden TEB1 sowie dem rot emittierenden Dotanden TER1 gezeigt. Die Ergebnisse der OLEDs sind in Tabelle 2 zusammengefasst. Bsp. 31-38 zeigen OLEDs mit Materialien gemäß dem Stand der Technik und dienen als Vergleichsbeispiele. Die erfindungsgemäßen OLEDs 39-50 zeigen die Vorteile beim Einsatz von Verbindungen gemäß Formel (1).

Durch den Einsatz von erfindungsgemäßen Verbindungen lassen sich, verglichen mit dem Stand der Technik, Verbesserungen in allen relevanten Parametern, allen voran Lebensdauer und Leistungseffizienz, erzielen. Gerade die Verbesserung der Leistungseffizienz ist von großer Bedeutung, da die Laufzeit mobiler Geräte entscheidend vom Energieverbrauch abhängt. Hier sind bereits Steigerungen von 10% als wesentlicher Fortschritt anzusehen.

Gegenüber dem phosphinoxidhaltigen Matrixmaterial H2 zeigen die erfindungsgemäßen Verbindungen den größten Fortschritt (Vergleich der Bsp. 35-37 mit Bsp. 44-48). Man erhält durch Einsatz der erfindungsgemäßen Matrixmaterialien H3, H4, H5, H6 und H7 zwar vergleichbare Betriebsspannungen wie beim Einsatz von H2 gemäß dem Stand der Technik, allerdings lässt sich die Stromeffizienz deutlich steigern. Die Verbesserung beträgt zwischen etwa 25% (Bsp. 45 und Bsp. 36) bis zu ca. 50% (Bsp. 48 und Bsp. 37), wodurch man entsprechend verbesserte Leistungseffizienzen erhält. Dies zeigt sich vor allem beim Einsatz von H1 als Elektronentransportmaterial, hier beträgt die Verbesserung in der Leistungseffizienz gegenüber dem Stand der Technik etwa 50% (Bsp. 48 und Bsp. 37). Hierbei ist hervorzuheben, dass die Verbesserung in der Leistungseffizienz mit einer deutlichen Steigerung der Lebensdauer einhergeht. Gegenüber dem Stand der Technik steigert sich durch Einsatz von erfindungsgemäßen Verbindungen die Lebensdauer um einem Faktor 1.8 (Bsp. 45 und Bsp. 36) bis zu 2.8 (Bsp. 47 und Bsp. 36).

Auch im Vergleich zu OLEDs enthaltend H1, die bereits relativ gute Leistungsdaten zeigen, lassen sich durch Verwendung von erfindungsgemäßen Verbindungen signifikante Verbesserungen erzielen. Dies gilt für rot emittierende (Bsp. 31, 32 im Vergleich zu Bsp. 39, 40) ebenso wie für grün emittierende OLEDs (Bsp. 33, 34 und Bsp. 41-43). Bei rot emittierenden OLEDs lässt sich die Leistungseffizienz um etwas mehr als 15%, vor allem aber auch die Lebensdauer signifikant steigern. Die Verbesserung in der Lebensdauer beträgt hierbei etwas über 50%. Bei grün emittierenden OLEDs führen die erfindungsgemäßen Verbindungen zwar nur zu einer geringen bzw. keiner Steigerung der Leistungseffizienz, allerdings erhält man durch den Einsatz von H3, H4 und H5 als Matrixmaterialien eine sehr deutliche Verbesserung in der Lebensdauer. Die Steigerung beträgt bis zu 55% (Bsp. 41 und Bsp. 33). Bei blau emittierenden OLEDs sinkt die Spannung um 1 V und die Leistungseffizienz nimmt um 33% bei der Verwendung von H3 als Hostmaterial zu (Bsp. 49 und Bsp. 38).

Einen sehr deutlichen Vorteil bezüglich der Betriebsspannung und damit vor allem auch der Leistungseffizienz lässt sich durch Einsatz der Triazin substituierten Verbindung H9 erhalten. Diese zeigt signifikant verbesserte Betriebsspannung, Leistungseffizienz und auch Lebensdauer gegenüber dem Stand der Technik (Bsp. 50).

**Tabelle 1: Aufbau der OLEDs**

| Bsp. | HTL Dicke | IL Dicke | EBL Dicke | EML Dicke | HBL Dicke | ETL Dicke | EIL Dicke |
|---|---|---|---|---|---|---|---|
| 31 (Vgl.) | HTM1 20 nm | - | NPB 20 nm | H1:TER1 (85%:15%) 30 nm | - | Alq₃ 20 nm | LiF 1 nm |
| 32 (Vgl.) | HTM1 20 nm | - | NPB 20 nm | H1:CBP:TER1 (45%:45%:10%) 30 nm | H1 10 nm | Alq₃ 20 nm | LiF 1 nm |
| 33 (Vgl.) | HTM1 160 nm | HTM1 - | EBM1 20 nm | H1:TEG1 (90%:10%) 30 nm | H1 10 nm | ETM1:LiQ (50%:50%) 40 nm | - |
| 34 (Vgl.) | HTM1 160 nm | - | EBM1 20 nm | H1:TEG1 (90%:10%) 30 nm | - | ETM1:LiQ (50%:50%) 40 nm | - |
| 35 (Vgl.) | HTM1 160 nm | - | EBM1 20 nm | H2:TEG1 (90%:10%) 30 nm | H1 10 nm | Alq₃ 20 nm | LiF 1 nm |
| 36 (Vgl.) | HTM1 140 nm | HIL1 5nm | EBM1 130 nm | H2:TEG1 (85%:15%) 30 nm | - | H1:LiQ (50%:50%) 40 nm | - |
| 37 (Vgl.) | HTM1 140 nm | HIL1 5 nm | EBM1 130 nm | H2:TEG1 (85%:15%) | - | H1 40 nm | LiQ 3 nm |
| 38 (Vgl.) | HTM11 140 nm | - | NPB 5 nm/ EBM2 15 nm | H8:TEB1 (90%:10%) | | H1 30 nm | LiQ 2 nm |
| 39 | HTM1 20 nm | - | NPB 20 nm | H4:TER1 (85%:15%) 30 nm | - | Alq₃ 20 nm | LiF 1 nm |
| 40 | HTM1 20 nm | - | NPB 20 nm | H4:CBP:TER1 (45%:45%:10%) 30 nm | H1 10 nm | Alq₃ 20 nm | LiF 1 nm |
| 41 | HTM1 160 nm | - | EBM1 20 nm | H3:TEG1 (90%:10%) 30 nm | H1 10 nm | ETM1:LiQ (50%:50%) 40 nm | - |
| 42 | HTM1 160 nm | - | EBM1 20 nm | H5:TEG1 (90%:10%) 30 nm | H1 10 nm | ETMI:LiQ (50%:50%) 40 nm | - |
| 43 | HTM1 160 nm | - | EBM1 20 nm | H4:TEG1 (90%:10%) 30 nm | - | ETM1:LiQ (50%:50%) 40 nm | - |
| 44 | HTM1 160 nm | - | EBM1 20 nm | H4:TEG1 (90%:10%) 30 nm | H1 10 nm | Alq₃ 20 nm | LiF 1 nm |
| 45 | HTM1 140 nm | HIL1 5 nm | EBM1 130 nm | H3:TEG1 (85%:15%) 30 nm | - | H1:LiQ (50%:50%) 40 nm | - |
| 46 | HTM1 140 nm | HIL1 5 nm | EBM1 130 nm | H6:TEG1 (85%:15%) 30 nm | - | H1:LiQ (50%:50%) 40 nm | - |
| 47 | HTM1 140 nm | HIL1 5 nm | EBM1 130 nm | H5:TEG1 (85%:15%) | - | H1 40 nm | LiQ 3nm |
| 48 | HTM1 140 nm | HIL1 5 nm | EBM1 130 nm | H7:TEG1 (85%:15%) | - | H1 40 nm | LiQ 3nm |
| 49 | HTM1 140 nm | - | NPB 5 nm/ EBM2 15 nm | H8:H3:TEB1 (80%:10%:10%) | | H1 30 nm | LiQ 2 nm |
| 50 | HTM1 140 nm | HIL1 5 nm | EBM1 130 nm | H9:TEG1 (85%:15%) | - | H1 40 nm | LiQ 3 nm |

**Tabelle 2: Ergebnisse für die OLEDs**

| Bsp. | Spannung für | Effizienz bei | Effizienz bei | CIE x/y bei | LD80 |
|---|---|---|---|---|---|
| | 1000 cd/m2 | 1000 cd/m2 | 1000 cd/m² | 1000 cd/m² | I₀ = 4000 cd/m² |
| 31 (Vgl.) | 5.0 V | 7.2 cd/A | 4.5 In/W | 0.69/0.31 | 230 h |
| 32 (Vgl.) | 5.2 V | 8.1 cd/A | 4.9 Im/W | 0.68/0.32 | 250 h |
| 33 (Vgl.) | 4.7 V | 55 cd/A | 37 Imin | 0.36/0.61 | 440 h |
| 34 (Vgl.) | 4.6 V | 54 cd/A | 37 Im/W | 0.37/0.60 | 400 h |
| 35 (Vgl.) | 4.2 V | 42 cd/A | 31 Im/W | 0.36/0.61 | 230 h |
| 36 (Vgl.) | 4.0 V | 43 cd/A | 33 Im/W | 0.38/0.59 | 300 h |
| 37 (Vgl.) | 3.8 V | 39 cd/A | 32 Im/W | 0.38/0.60 | 210 h |
| 38 (Vgl.) | 8.2 V | 17 cd/A | 6 Im/W | 0.16/0.27 | |
| 39 | 4.4 V | 7.4 cd/A | 5.3 Im/W | 0.69/0.32 | 350 h |
| 40 | 4.5 V | 8.0 cd/A | 5.6 Im/W | 0.68/0.32 | 390 h |
| 41 | 4.6 V | 54 cd/A | 37 Im/W | 0.35/0.61 | 680 h |
| 42 | 4.4 V | 52 cd/A | 37 Im/W | 0.3610.61 | 590 h |
| 43 | 4.2 V | 57 cd/A | 43 Im/W | 0.36/0.60 | 560 h |
| 44 | 4.4 V | 54 cd/A | 39 Im/W | 0.36/0.60 | 620 h |
| 45 | 4.3 V | 53 cd/A | 38 Im/W | 0.36/0.60 | 550 h |
| 46 | 4.2 V | 58 cd/A | 43 Im/W | 0.35/0.61 | 630 h |
| 47 | 3.7 V | 55 cd/A | 47 Im/W | 0.36/0.60 | 590 h |
| 48 | 3.9 V | 59 cd/A | 48 In/W | 0.35/0.61 | 530 h |
| 49 | 7.1 | 18 cd/A | 8 In/W | 0.16/0.27 | |
| 50 | 3.3 V | 57 cd/A | 54 In/W | 0.38/0.59 | 610 h |

**Tabelle 3: Strukturformeln der verwendeten Materialien**

| | |
|---|---|
| 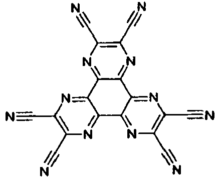 | 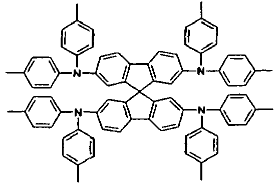 |
| HIL1 | HTM1 |
| 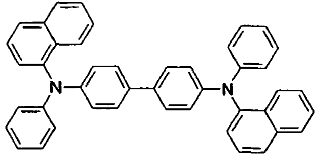 | 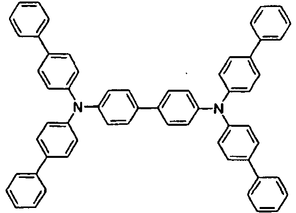 |
| NPB | EBM1 |
| 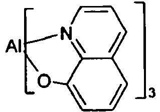 | 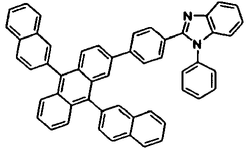 |
| Alq₃ | ETM1 |
| 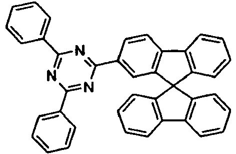 | 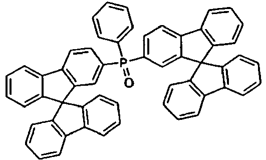 |
| H1 | H2 |
| 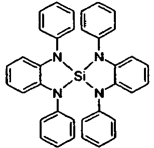 | 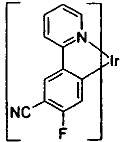 |
| EBM3 | TEB1 |
| 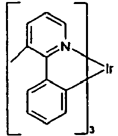 | 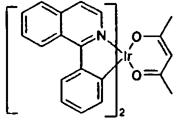 |
| TEG1 | TER1 |
| 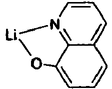 | 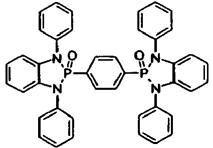 |
| LiQ | H3 |
| 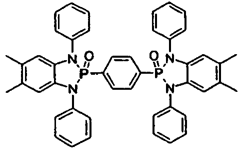 | 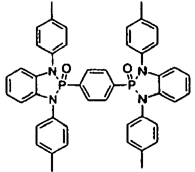 |
| H4 | H5 |
| 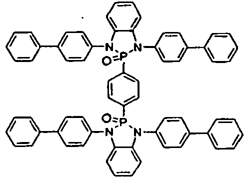 | 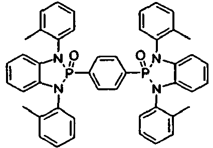 |
| H6 | H7 |
| 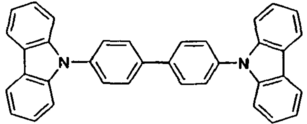 | 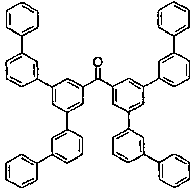 |
| CBP | H8 |
| 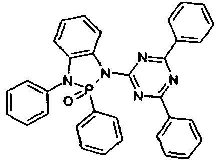 | |
| H9 | |

### Beispiel 51: Synthese von 6-[[2-(phenylamino)phenyl]amino]-2,4-diphenyl-1,3,5-triazin

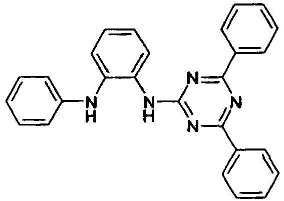

44.3 g (240 mmol) N-Phenyl-o-phenylendiamin werden in THF vorgelegt, auf 0 °C gekühlt und tropfenweise mit 136.4 ml (818 mmol) N-Ethyldiisopropylamin versetzt. Anschließend wird 1 h bei Raumtemperatur nachgerührt. Dann wird eine Lösung aus 64.4 g (240 mmol) 2-chlor-4,6-diphenyl-1,3,5-triazine gelöst in 1600 ml THF zugetropft und 96 h bei 60 °C gerührt. Nach dem Abkühlen wird die Lösung mit 400 ml Heptan, 80 ml Dichlormethan und 100 ml Ethanol gerührt. Der Feststoff wird mit Toluol gewaschen und mit Heptan heiß ausgerührt. Dabei erhält man 87.7 g (211 mmol) eines kristallinen Feststoffs. Die Gesamtausbeute beträgt 88 %.

### Beispiel 52-54: Synthese der Diamine mit Heteroaromaten

Die nachfolgend aufgeführten Diamine können aus den entsprechenden Dibrom-Aromaten durch Umsetzung mit dem entsprechenden o-Phenylendiamin analog zu Beispiel 51 dargestellt werden.

| **Bsp.** | **Aromat 1** | **Aromat 2** | **o-Phenylendiamin** | **Ausbeute** |
|---|---|---|---|---|
| 52 | 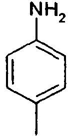 | 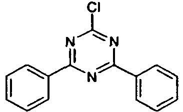 | 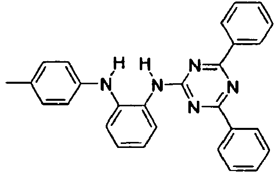 | 85.0 % |
| 53 | 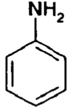 | 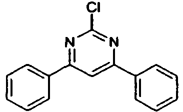 | 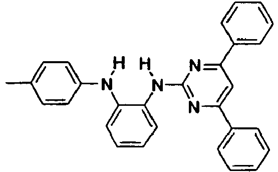 | 87.0% |
| 54 | 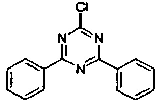 | 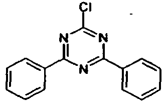 | 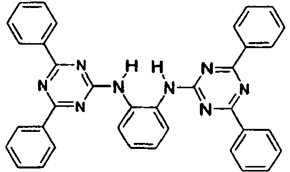 | 56.0% |

### Beispiel 55: Synthese von Diazaphospholen mit Heteroaromaten

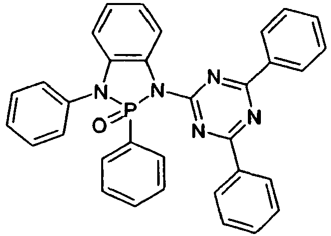

21.5 g (51.9 mmol) 6-[[2-(Phenylamino)phenyl]amino]-2,4-diphenyl-1,3,5-triazin wird in 165 ml Pyridin gelöst und auf 0 °C gekühlt. Zu dieser Lösung werden bei 0 °C 7 ml (51.9 mmol) Phenylphosphonigsäuredichlorid gelöst in 199 ml Toluol unter gutem Rühren tropfenweise zugetropft, 1 h gerührt und dann 24 h unter Rückfluss erhitzt. Das Lösungsmittel wird im Vakuum abdestilliert, der Feststoff in Essigsäureethylester ausgekocht, abgesaugt, einmal mit 100 ml Essigsäureethylester gewaschen und anschließend aus Dioxan umkristallisiert. Ausbeute: 12 g (33 mmol), 69 %, Reinheit 99.9 % ig (HPLC).

### Beispiel 56-60: Synthese der Diazaphosphole

Die nachfolgend aufgeführten Diazaphosphole können aus den entsprechenden Diamino-Aromaten durch Umsetzung mit dem entsprechenden Phosphonigsäurechlorid analog zu Beispiel 55 dargestellt werden.

| **Bsp.** | Phosphonigsäurechlorid | Phenylendiamin | **Diazaphosphole** | **Ausbeute** |
|---|---|---|---|---|
| 56 | 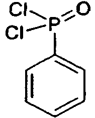 | 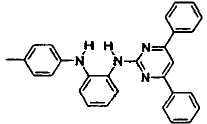 | 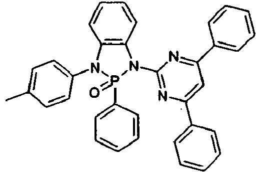 | 71.0 % |
| 57 | 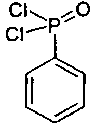 | 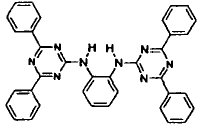 | 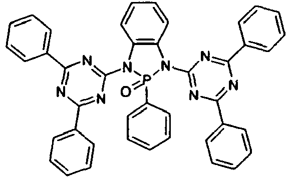 | 70% |
| 58 | 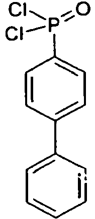 | 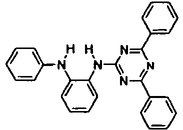 | 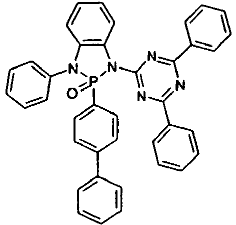 | 73.0 % |
| 59 | 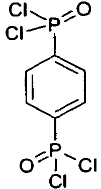 | 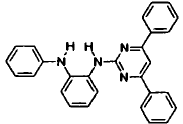 | 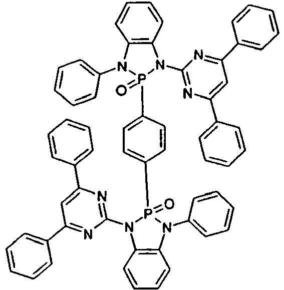 | 45.0% |
| 60 | 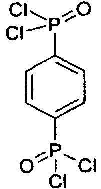 | 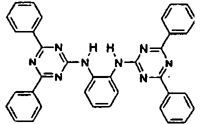 | 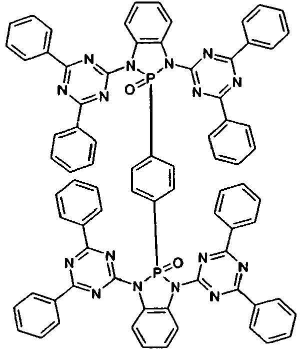 | 65.0 % |

## Patentansprüche

1. Elektronische Vorrichtung enthaltend mindestens eine Verbindung gemäß Formel (1) oder Formel (39), 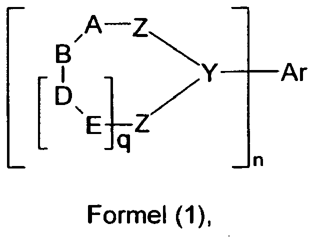 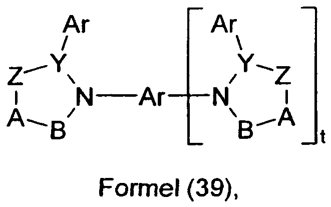 wobei für die verwendeten Symbole und Indizes gilt:
A-B und D-E ist jeweils gleich oder verschieden bei jedem Auftreten eine Einheit der folgenden Formel (2), (3), (4), (5) oder (6), 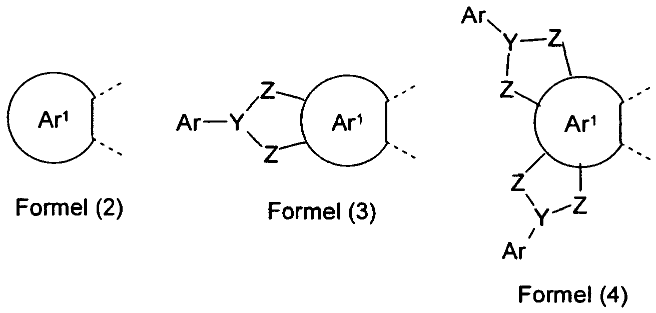 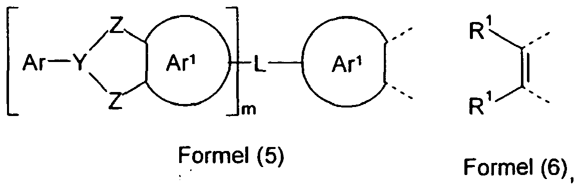 wobei die gestrichelte Bindung jeweils die Verknüpfung mit Z darstellt;
und
Z ist gleich oder verschieden bei jedem Auftreten N-R², O oder S;
oder
A-Z und B-Z ist jeweils gleich oder verschieden bei jedem Auftreten eine Einheit der folgenden Formel (7) und q = 0, 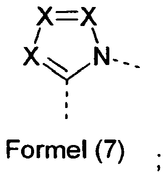 dabei stellt die gestrichelte Bindung die Verknüpfung dieser Einheit in der Verbindung der Formel (1) dar, wobei der Stickstoff mit der Gruppe Y verknüpft ist;
Y ist bei jedem Auftreten gleich oder verschieden P(=O), P(=S), P, As(=O), As(=S), As, Sb(=O), Sb(=S), Sb, Bi(=O), Bi(=S) oder Bi;
Ar ist bei jedem Auftreten gleich oder verschieden ein aromatisches oder heteroaromatisches Ringsystem mit 5 bis 60 aromatischen Ringatomen, welches durch einen oder mehrere Reste R¹ substituiert sein kann;
Ar¹ ist bei jedem Auftreten gleich oder verschieden eine Aryl- oder Heteroarylgruppe mit 5 bis 18 aromatischen Ringatomen, welche durch einen oder mehrere Reste R¹ substituiert sein kann;
X ist bei jedem Auftreten gleich oder verschieden CR¹ oder N;
L ist eine Einfachbindung oder eine bivalente, trivalente oder tetravalente Gruppe;
R¹ ist bei jedem Auftreten gleich oder verschieden ausgewählt aus der Gruppe bestehend aus H, D, F, Cl, Br, I, CN, NO₂, N(R³)₂, C(=O)R³, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C=-C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
R² ist bei jedem Auftreten gleich oder verschieden ausgewählt aus der Gruppe bestehend aus einer geradkettigen Alkylgruppe mit 1 bis 40 C-Atomen oder einer verzweigten oder cyclischen Alkylgruppe mit 3 bis 40 C-Atomen, die jeweils mit einem oder mehreren Resten R³ substituiert sein kann, wobei eine oder mehrere nicht-benachbarte CH₂-Gruppen durch R³C=CR³, C≡C oder C=O ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, oder einer Kombination dieser Systeme; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein;
R³ ist ausgewählt aus der Gruppe bestehend aus H, D, F, CN, aliphatischem Kohlenwasserstoffrest mit 1 bis 20 C-Atomen, aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches, aromatisches oder heteroaromatisches Ringsystem bilden können;
n, t ist 1 bis 10, bevorzugt 1, 2, 3, 4, 5 oder 6;
m ist 1, wenn L eine Einfachbindung oder eine bivalente Gruppe ist, oder ist 2, wenn L eine trivalente Gruppe ist, oder ist 3, wenn L eine tetravalente Gruppe ist;
q ist bei jedem Auftreten gleich oder verschieden 0 oder 1.

2. Elektronische Vorrichtung nach Anspruch 1, **dadurch gekennzeichnet, dass** Ar¹ gleich oder verschieden bei jedem Auftreten für eine Aryl- oder Heteroarylgruppe mit 5 bis 14 aromatischen Ringatomen, bevorzugt mit 5 bis 10 aromatischen Ringatomen, besonders bevorzugt für Benzol, Pyridin, Pyrimidin, Pyridazin, Pyrazin, Triazin, Furan, Thiophen, Pyrrol, Naphthalin, Phenanthren, Chinolin, Isochinolin, Chinoxalin, Indol, Benzofuran, Benzothiophen oder Carbazol steht.

3. Elektronische Vorrichtung nach Anspruch 1 oder 2, **dadurch gekennzeichnet, dass** die Verbindung gemäß Formel (1) ausgewählt ist aus Verbindungen der Formeln (8) bis (18), 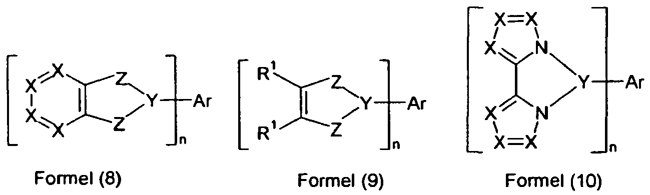 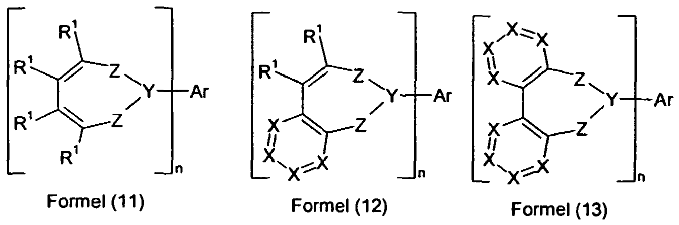 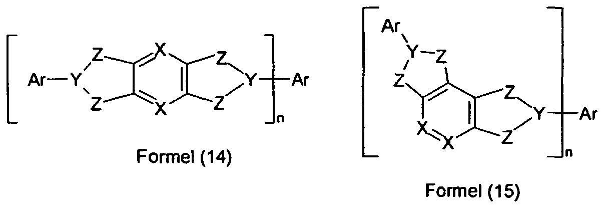 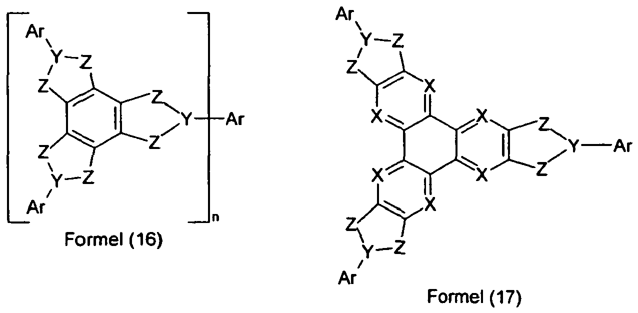 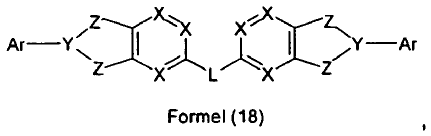 wobei die verwendeten Symbole und Indizes die in Anspruch 1 genannten Bedeutungen aufweisen.

4. Elektronische Vorrichtung nach einem oder mehreren der Ansprüche 1 bis 3, **dadurch gekennzeichnet, dass** das Symbol Y für P(=O) und das Symbol Z bei jedem Auftreten gleich oder verschieden für N-R² steht.

5. Elektronische Vorrichtung nach einem oder mehreren der Ansprüche 1 bis 4, **dadurch gekennzeichnet, dass** die Gruppe Ar für ein aromatisches oder heteroaromatisches Ringsystem mit 5 bis 30 aromatischen Ringatomen steht, bevorzugt ausgewählt aus der Gruppe bestehend aus den Einheiten der folgenden Formeln (19) bis (36), wobei die gestrichelte Bindung jeweils eine Verknüpfung mit der Gruppe Y in Formel (1) bzw. mit N in Formel (39) andeutet:

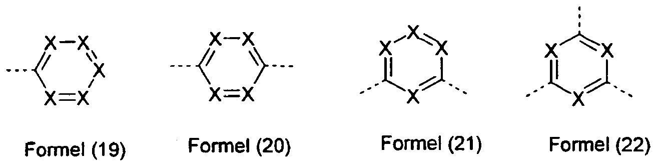

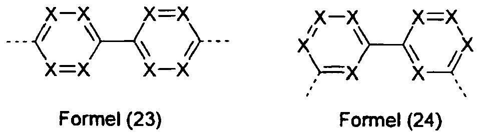

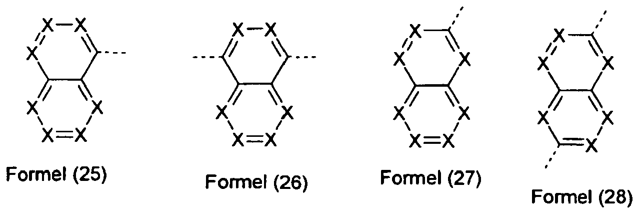

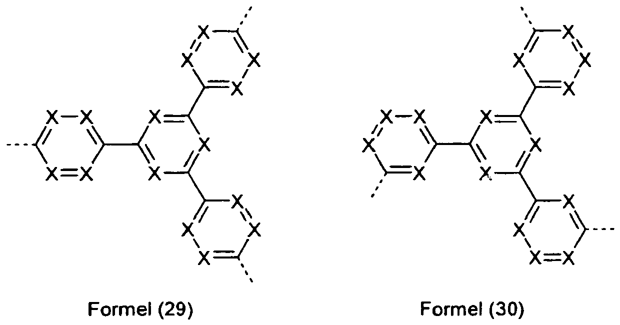

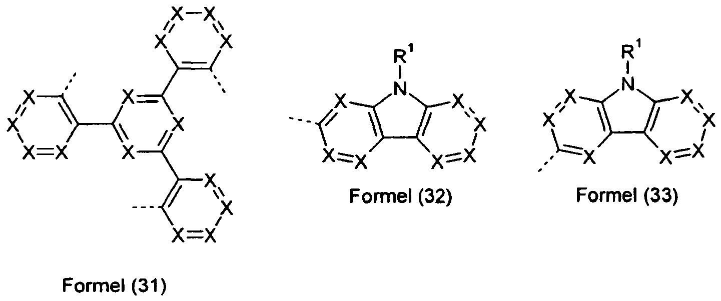

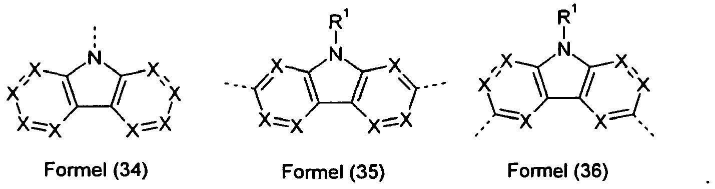

6. Elektronische Vorrichtung nach einem oder mehreren der Ansprüche 1 bis 5, **dadurch gekennzeichnet, dass** R² gleich oder verschieden bei jedem Auftreten ausgewählt ist aus der Gruppe bestehend aus einem aromatischen oder heteroaromatischen Ringsystem mit 5 bis 24 aromatischen Ringatomen, das jeweils mit einem oder mehreren Resten R³ substituiert sein kann, bevorzugt Phenyl, Naphthyl, Biphenyl oder Terphenyl, welches jeweils mit einem oder mehreren Resten R³ substituiert sein kann.

7. Elektronische Vorrichtung nach einem oder mehreren der Ansprüche 1 bis 6, **dadurch gekennzeichnet, dass** für die Symbole und Indizes gilt:
Ar¹ ist gleich oder verschieden bei jedem Auftreten für eine Aryl- oder Heteroarylgruppe mit 5 bis 14 aromatischen Ringatomen, bevorzugt mit 5 bis 10 aromatischen Ringatomen, besonders bevorzugt mit 6 aromatischen Ringatomen;
Y ist bei jedem Auftreten gleich oder verschieden P(=O) oder P(=S);
Z ist gleich oder verschieden bei jedem Auftreten N-R², wenn es nicht mit A bzw. B einen Ring gemäß Formel (7) bildet;
X steht für CR¹ oder N, wobei maximal zwei Symbole X für N stehen, bevorzugt maximal ein Symbol X;
Ar ist gleich oder verschieden ein aromatisches oder heteroaromatisches Ringsystem mit 5 bis 30 aromatischen Ringatomen, bevorzugt mit 5 bis 24 aromatischen Ringatomen; dabei enthält bevorzugt keine der Aryl- bzw. Heteroarylgruppen des aromatischen oder heteroaromatischen Ringsystems mehr als 10 aromatische Ringatome;
q ist 0;
n ist 1, 2, 3 oder 4, bevorzugt 1, 2 oder 3.
R¹ ist gleich oder verschieden bei jedem Auftreten ausgewählt aus der Gruppe bestehend aus H, D, F, CN, N(R³)₂, einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen oder einer Alkenylgruppe mit 2 bis 20 C-Atomen, die jeweils mit einem oder mehreren Resten R³ substituiert sein kann, wobei eine oder mehrere nicht-benachbarte CH₂-Gruppen durch R³C=CR³ oder O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R³ substituiert sein kann, einer Aryloxy- oder Heteroaryloxygruppe mit 5 bis 30 aromatischen Ringatomen, die mit einem oder mehreren Resten R³ substituiert sein können, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
R² ist bei jedem Auftreten gleich oder verschieden ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R³ substituiert sein kann, wobei ein oder mehrere H-Atome durch F oder D ersetzt sein können, einem aromatischen oder heteroaromatischen Ringsystem mit 5 bis 30 aromatischen Ringatomen, das jeweils mit einem oder mehreren Resten R³ substituiert sein kann, oder einer Kombination dieser Systeme; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein kann;
die weiteren Symbole und Indizes haben die in Anspruch 1 genannten Bedeutungen.

8. Elektronische Vorrichtung nach einem oder mehreren der Ansprüche 1 bis 7, **dadurch gekennzeichnet, dass** für die verwendeten Symbole und Indizes gilt:
Ar¹ ist gleich oder verschieden bei jedem Auftreten ausgewählt aus der Gruppe bestehend aus Benzol, Pyridin, Pyrimidin, Pyridazin, Pyrazin, Triazin, Furan, Thiophen, Pyrrol, Naphthalin, Chinolin, Isochinolin, Chinoxalin, Indol, Benzofuran, Benzothiophen und Carbazol;
Y ist P(=O);
Z ist gleich oder verschieden bei jedem Auftreten N-R², wenn es nicht mit A bzw. B einen Ring gemäß Formel (7) bildet;
X ist gleich oder verschieden bei jedem Auftreten CR¹;
Ar ist ein aromatisches oder heteroaromatisches Ringsystem mit 5 bis 24 aromatischen Ringatomen, welches aufgebaut ist aus jeweils einer oder mehreren der Gruppen Benzol, Pyridin, Pyrimidin, Pyridazin, Pyrazin, Pyrrol, Thiophen, Furan, Naphthalin, Triphenylen, Chinolin, Isochinolin, Chinoxalin, Indol, Benzothiophen oder Benzofuran, bevorzugt aus jeweils einer oder mehreren Gruppen Benzol, Pyridin, Pyrimidin, Pyridazin, Pyrazin oder Triazin, insbesondere Benzol;
q ist 0;
n ist 1 oder 2;
R¹ ist gleich oder verschieden bei jedem Auftreten ausgewählt aus der Gruppe bestehend aus H, D, F, CN, einer geradkettigen Alkylgruppe mit 1 bis 10 C-Atomen, bevorzugt mit 1 bis 4 C-Atomen, oder einer verzweigten oder cyclischen Alkylgruppe mit 3 bis 10 C-Atomen, besonders bevorzugt mit 3 bis 5 C-Atomen, oder einer Alkenylgruppe mit 2 bis 10 C-Atomen, bevorzugt mit 2 bis 4 C-Atomen, die jeweils mit einem oder mehreren Resten R³ substituiert sein kann, wobei ein oder mehrere H-Atome durch D ersetzt sein können, einem aromatischen oder heteroaromatischen Ringsystem mit 5 bis 12 aromatischen Ringatomen, das jeweils mit einem oder mehreren Resten R³ substituiert sein kann, und einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
R² ist bei jedem Auftreten gleich oder verschieden ausgewählt aus der Gruppe bestehend aus einem aromatischen oder heteroaromatischen Ringsystem mit 5 bis 24 aromatischen Ringatomen, das jeweils mit einem oder mehreren Resten R³ substituiert sein kann;
die weiteren verwendeten Symbole und Indizes haben die in Anspruch 1 genannten Bedeutungen.

9. Elektronische Vorrichtung nach einem oder mehreren der Ansprüche 1 bis 8, ausgewählt ist aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices".

10. Organische Elektrolumineszenzvorrichtung nach einem oder mehreren der Ansprüche 1 bis 9, **dadurch gekennzeichnet, dass** die Verbindung gemäß Formel (1) bzw. Formel (8) bis (18) als Matrixmaterial für fluoreszierende oder phosphoreszierende Emitter, insbesondere für phosphoreszierende Emitter, und/oder als Lochblockiermaterial in einer Lochblockierschicht und/oder als Elektronentransportmaterial in einer Elektronentransportschicht bzw. einer Elektroneninjektionsschicht und/oder als Elektronenblockiermaterial bzw. Exzitonenblockiermaterial in einer elektronenblockierenden bzw. exzitonenblockierenden Schicht und/oder als Lochtransportmaterial in einer Lochtransportschicht bzw. Lochinjektionsschicht eingesetzt wird.

11. Organische Elektrolumineszenzvorrichtung nach Anspruch 10,
**dadurch gekennzeichnet, dass** als phosphoreszierender Emitter eine Verbindung eingesetzt wird, die Kupfer, Molybdän, Wolfram, Rhenium, Ruthenium, Osmium, Rhodium, Iridium, Palladium, Platin, Silber, Gold oder Europium enthält, insbesondere Iridium oder Platin.

12. Verbindung gemäß Formel (1'), 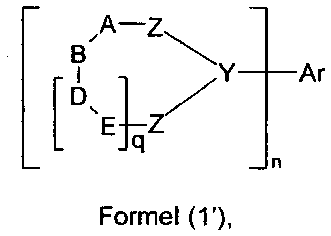 wobei für die verwendeten Symbole und Indizes gilt:
A-B und D-E ist jeweils gleich oder verschieden bei jedem Auftreten eine Einheit der folgenden Formel (2), (3), (4), (5) oder (6), 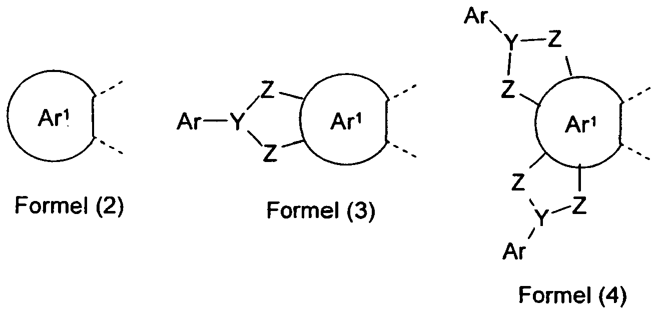 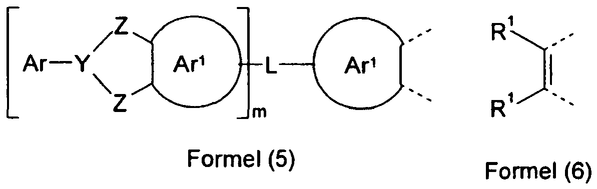 dabei stellt die gestrichelte Bindung jeweils die Bindung zu Z dar;
und
Z ist gleich oder verschieden bei jedem Auftreten N-R², O oder S, mit der Maßgabe, dass nicht beide Gruppen Z, die an dieselbe Gruppe Y binden, für O stehen;
oder
A-Z und B-Z ist jeweils gleich oder verschieden bei jedem Auftreten eine Einheit der folgenden Formel (7) und q = 0, 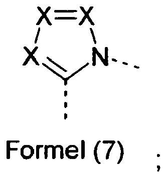 dabei stellt die gestrichelte Bindung in Formel (7) die Verknüpfung dieser Einheit in der Verbindung der Formel (1) dar, wobei der Stickstoff mit der Gruppe Y verknüpft ist;
Y ist bei jedem Auftreten gleich oder verschieden P(=O), As(=O), As(=S), Sb(=O), Sb(=S), Bi(=O) oder Bi(=S);
Ar ist bei jedem Auftreten gleich oder verschieden ein aromatisches oder heteroaromatisches Ringsystem mit 5 bis 60 aromatischen Ringatomen, welches durch einen oder mehrere Reste R¹ substituiert sein kann;
Ar¹ ist bei jedem Auftreten gleich oder verschieden eine Aryl- oder Heteroarylgruppe mit 5 bis 16 aromatischen Ringatomen, welche durch einen oder mehrere Reste R¹ substituiert sein kann;
X ist bei jedem Auftreten gleich oder verschieden CR¹ oder N;
L ist eine Einfachbindung oder eine bivalente, trivalente oder tetravalente Gruppe;
R¹ ist bei jedem Auftreten gleich oder verschieden ausgewählt aus der Gruppe bestehend aus H, D, F, Cl, Br, I, CN, NO₂, N(R³)₂, C(=O)R³, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C≡C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
R² ist bei jedem Auftreten gleich oder verschieden ausgewählt aus der Gruppe bestehend aus einer geradkettigen Alkylgruppe mit 1 bis 40 C-Atomen oder einer verzweigten oder cyclischen Alkylgruppe mit 3 bis 40 C-Atomen, die jeweils mit einem oder mehreren Resten R³ substituiert sein kann, wobei eine oder mehrere nicht-benachbarte CH₂-Gruppen durch R³C=CR³, C=C oder C=O ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, oder einer Kombination dieser Systeme; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein;
R³ ist ausgewählt aus der Gruppe bestehend aus H, D, F, CN, aliphatischem Kohlenwasserstoffrest mit 1 bis 20 C-Atomen, aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches, aromatisches oder heteroaromatisches Ringsystem bilden können;
n ist 1 bis 10, bevorzugt 1, 2, 3, 4, 5 oder 6;
m ist 1, wenn L eine Einfachbindung oder eine bivalente Gruppe ist, oder ist 2, wenn L eine trivalente Gruppe ist, oder ist 3, wenn L eine tetravalente Gruppe ist;
q ist bei jedem Auftreten gleich oder verschieden 0 oder 1;
dabei sind die folgenden Verbindungen von der Erfindung ausgeschlossen: 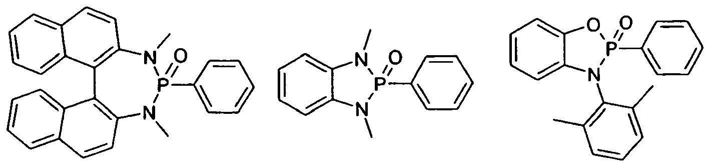 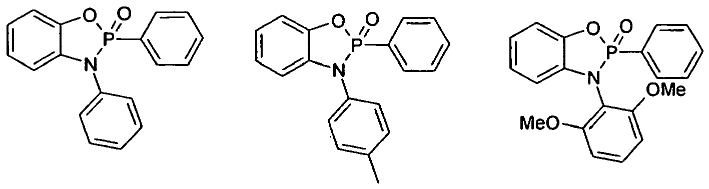 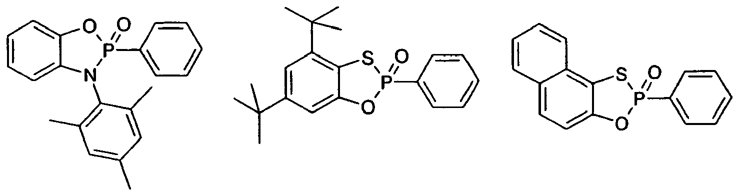 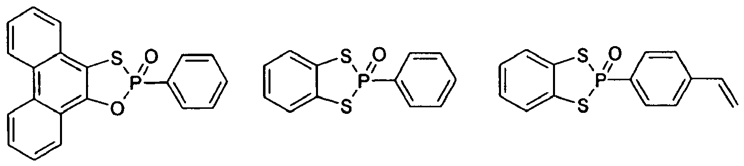 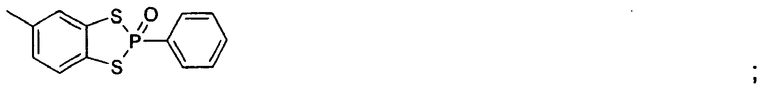 oder Verbindung gemäß Formel (39), 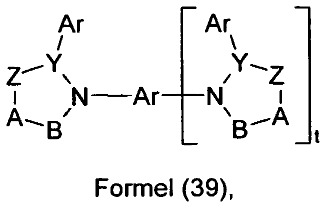 wobei die verwendeten Symbole und Indizes die in Anspruch 1 genannten Bedeutungen aufweisen.

13. Verwendung einer Verbindung nach Anspruch 12 in einer elektronischen Vorrichtung.

14. Verfahren zur Herstellung einer Verbindung nach Anspruch 12 durch Umsetzung eines ortho-Diamino-substituierten Aromaten, wobei die Aminogruppen unsubstituiert oder bevorzugt monosubstituiert sind, mit einem aromatischen Phosphonsäurechloridderivat bzw. einem aromatischen Oligophosphonsäurechloridderivat.

15. Oligomer, Polymer oder Dendrimer enthaltend eine oder mehrere Verbindungen nach Anspruch 12, wobei ein oder mehrere Bindungen der Verbindung zum Polymer, Oligomer oder Dendrimer vorhanden sind,
bzw. Oligomer oder Polymer gemäß Formel (37) bzw. Formel (38), 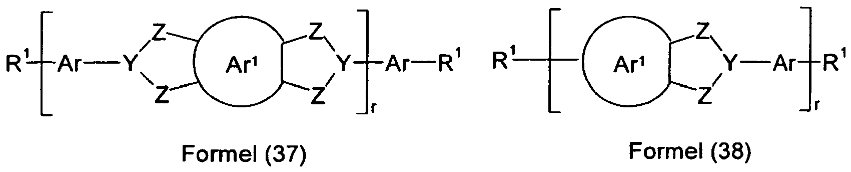 wobei die verwendeten Symbole die in Anspruch 11 genannten Bedeutungen aufweisen und r für eine ganze Zahl zwischen 2 und 1000000 steht.

## Claims

1. Electronic device comprising at least one compound of the formula (1) or formula (39) 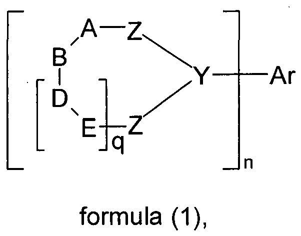 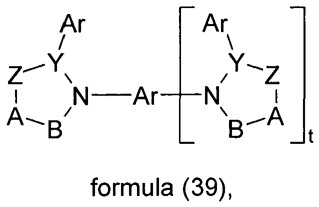 where the following applies to the symbols and indices used:
A-B and D-E are in each case, identically or differently on each occurrence, a unit of the following formula (2), (3), (4), (5) or (6), 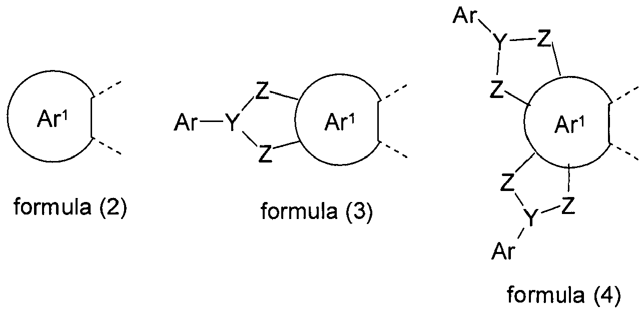 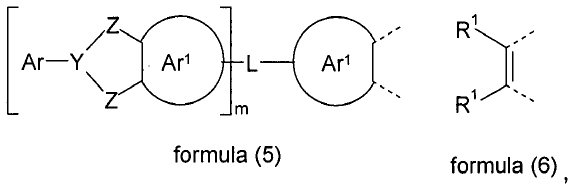 where the dashed bond in each case represents the link to Z;
and
Z is, identically or differently on each occurrence, N-R², O or S;
or
A-Z and B-Z are in each case, identically or differently on each occurrence, a unit of the following formula (7) and q = 0, 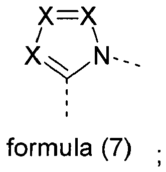 the dashed bond here represents the linking of this unit in the compound of the formula (1), where the nitrogen is linked to the group Y;
Y is on each occurrence, identically or differently, P(=O), P(=S), P, As(=O), As(=S), As, Sb(=O), Sb(=S), Sb, Bi(=O), Bi(=S) or Bi;
Ar is on each occurrence, identically or differently, an aromatic or heteroaromatic ring system having 5 to 60 aromatic ring atoms, which may be substituted by one or more radicals R¹;
Ar¹ is on each occurrence, identically or differently, an aryl or heteroaryl group having 5 to 18 aromatic ring atoms, which may be substituted by one or more radicals R¹;
X is on each occurrence, identically or differently, CR¹ or N;
L is a single bond or a divalent, trivalent or tetravalent group;
R¹ is selected on each occurrence, identically or differently, from the group consisting of H, D, F, Cl, Br, I, CN, NO₂, N(R³)₂, C(=O)R³, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, each of which may be substituted by one or more radicals R³, where one or more non-adjacent CH₂ groups may be replaced by R³C=CR³, C≡C, Si(R³)₂, Ge(R³)₂, Sn(R³)₂, C=O, C=S, C=Se, C=NR³, P(=O)(R³), SO, SO₂, NR³, O, S or CONR³ and where one or more H atoms may be replaced by D, F, Cl, Br, I, CN or NO₂, an aromatic or heteroaromatic ring system having 5 to 60 aromatic ring atoms, which may in each case be substituted by one or more radicals R³, an aryloxy or heteroaryloxy group having 5 to 60 aromatic ring atoms, which may be substituted by one or more radicals R³, or a combination of these systems, where two or more adjacent substituents R¹ may optionally form a monocyclic or polycyclic, aliphatic, aromatic or heteroaromatic ring system, which may be substituted by one or more radicals R³;
R² is selected on each occurrence, identically or differently, from the group consisting of a straight-chain alkyl group having 1 to 40 C atoms or a branched or cyclic alkyl group having 3 to 40 C atoms, each of which may be substituted by one or more radicals R³, where one or more non-adjacent CH₂ groups may be replaced by R³C=CR³, C≡C or C=O and where one or more H atoms may be replaced by D, F, Cl, Br, I, CN or NO₂, an aromatic or heteroaromatic ring system having 5 to 60 aromatic ring atoms, which may in each case be substituted by one or more radicals R³, or a combination of these systems; R¹ and R² which are adjacent to one another in the 1,2-position may optionally form a monocyclic or polycyclic, aliphatic, aromatic or heteroaromatic ring system, which may be substituted by one or more radicals R³;
R³ is selected from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN, where two or more adjacent substituents R³ may form a mono- or polycyclic, aliphatic, aromatic or heteroaromatic ring system with one another;
n, t are 1 to 10, preferably 1, 2, 3, 4, 5 or 6;
m is 1 if L is a single bond or a divalent group, or is 2 if L is a trivalent group, or is 3 if L is a tetravalent group;
q is on each occurrence, identically or differently, 0 or 1.

2. Electronic device according to Claim 1, **characterised in that** Ar¹ stands, identically or differently on each occurrence, for an aryl or heteroaryl group having 5 to 14 aromatic ring atoms, preferably having 5 to 10 aromatic ring atoms, particularly preferably for benzene, pyridine, pyrimidine, pyridazine, pyrazine, triazine, furan, thiophene, pyrrole, naphthalene, phenanthrene, quinoline, isoquinoline, quinoxaline, indole, benzofuran, benzothiophene or carbazole.

3. Electronic device according to Claim 1 or 2, **characterised in that** the compound of the formula (1) is selected from compounds of the formulae (8) to (18), 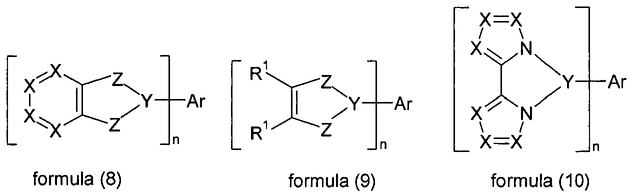 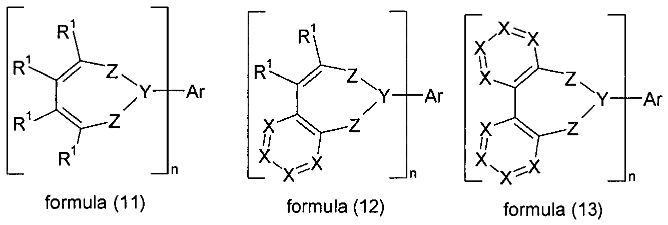 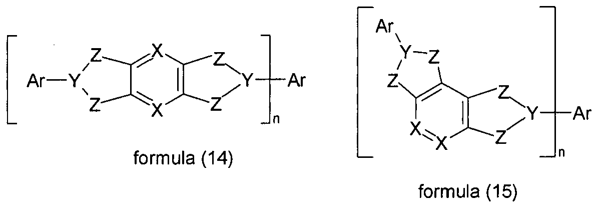 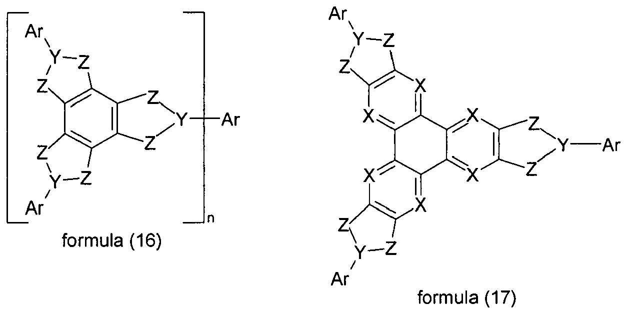 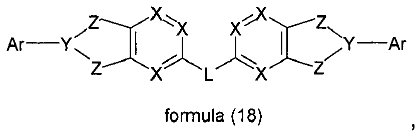 where the symbols and indices used have the meanings indicated in Claim 1.

4. Electronic device according to one or more of Claims 1 to 3, **characterised in that** the symbol Y stands for P(=O) and the symbol Z stands on each occurrence, identically or differently, for N-R².

5. Electronic device according to one or more of Claims 1 to 4, **characterised in that** the group Ar stands for an aromatic or heteroaromatic ring system having 5 to 30 aromatic ring atoms, preferably selected from the group consisting of the units of the following formulae (19) to (36), where the dashed bond in each case indicates a link to the group Y in formula (1) or to N in formula (39),:

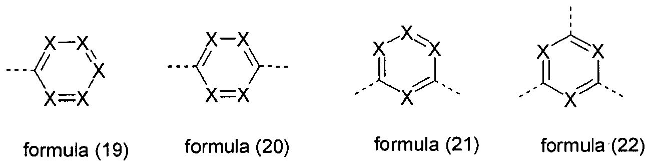

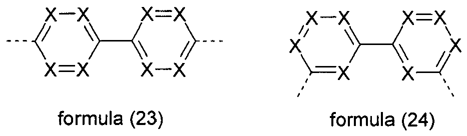

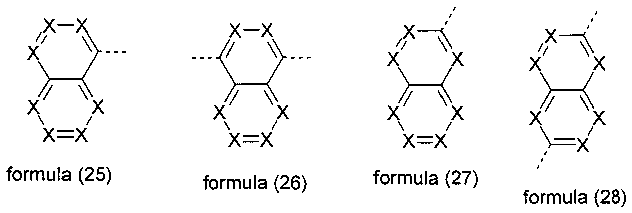

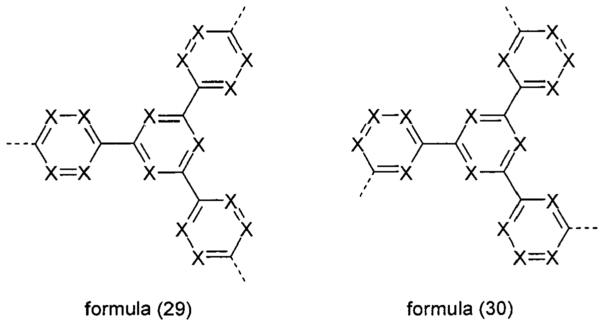

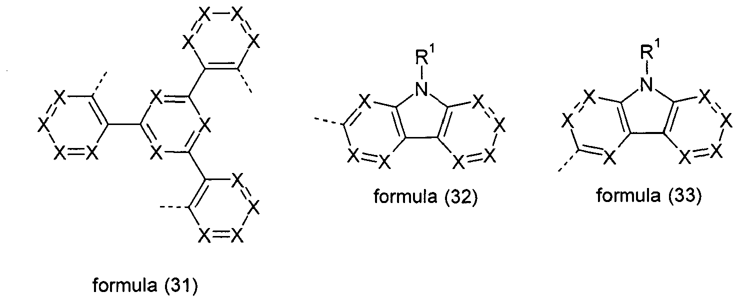

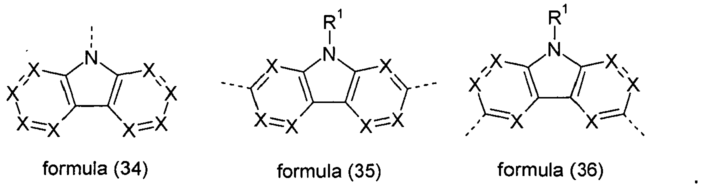

6. Electronic device according to one or more of Claims 1 to 5, **characterised in that** R² is selected, identically or differently on each occurrence, from the group consisting of an aromatic or heteroaromatic ring system having 5 to 24 aromatic ring atoms, which may in each case be substituted by one or more radicals R³, preferably phenyl, naphthyl, biphenyl or terphenyl, each of which may be substituted by one or more radicals R³

7. Electronic device according to one or more of Claims 1 to 6, **characterised in that** the following applies to the symbols and indices:
Ar¹ is, identically or differently on each occurrence, an aryl or heteroaryl group having 5 to 14 aromatic ring atoms, preferably having 5 to 10 aromatic ring atoms, particularly preferably having 6 aromatic ring atoms;
Y is on each occurrence, identically or differently, P(=O) or P(=S);
Z is, identically or differently on each occurrence, N-R² if it does not form a ring of the formula (7) with A or B;
X stands for CR¹ or N, where a maximum of two symbols X stand for N, preferably a maximum of one symbol X stands for N;
Ar is, identically or differently, an aromatic or heteroaromatic ring system having 5 to 30 aromatic ring atoms, preferably having 5 to 24 aromatic ring atoms; preferably, none of the aryl or heteroaryl groups of the aromatic or heteroaromatic ring system contains more than 10 aromatic ring atoms;
q is 0;
n is 1, 2, 3 or 4, preferably 1, 2 or 3;
R¹ is selected, identically or differently on each occurrence, from the group consisting of H, D, F, CN, N(R³)₂, a straight-chain alkyl or alkoxy group having 1 to 20 C atoms or a branched or cyclic alkyl or alkoxy group having 3 to 20 C atoms or an alkenyl group having 2 to 20 C atoms, each of which may be substituted by one or more radicals R³, where one or more non-adjacent CH₂ groups may be replaced by R³C=CR or O and where one or more H atoms may be replaced by D or F, an aromatic or heteroaromatic ring system having 5 to 30 aromatic ring atoms, which may in each case be substituted by one or more radicals R³, an aryloxy or heteroaryloxy group having 5 to 30 aromatic ring atoms, which may be substituted by one or more radicals R³ or a combination of these systems, where two or more adjacent substituents R¹ may optionally form a monocyclic or polycyclic, aliphatic, aromatic or heteroaromatic ring system, which may be substituted by one or more radicals R³;
R² is selected on each occurrence, identically or differently, from the group consisting of a straight-chain alkyl group having 1 to 10 C atoms or a branched or cyclic alkyl group having 3 to 10 C atoms, each of which may be substituted by one or more radicals R³, where one or more H atoms may be replaced by F or D, an aromatic or heteroaromatic ring system having 5 to 30 aromatic ring atoms, which may in each case be substituted by one or more radicals R³, or a combination of these systems; R¹ and R² which are adjacent to one another in the 1,2-position may optionally form a monocyclic or polycyclic, aliphatic, aromatic or heteroaromatic ring system, which may be substituted by one or more radicals R³;
the other symbols and indices have the meanings indicated in Claim 1.

8. Electronic device according to one or more of Claims 1 to 7, **characterised in that** the following applies to the symbols and indices used:
Ar¹ is selected, identically or differently on each occurrence, from the group consisting of benzene, pyridine, pyrimidine, pyridazine, pyrazine, triazine, furan, thiophene, pyrrole, naphthalene, quinoline, isoquinoline, quinoxaline, indole, benzofuran, benzothiophene and carbazole;
Y is P(=O);
Z is, identically or differently on each occurrence, N-R² if it does not form a ring of the formula (7) with A or B;
X is, identically or differently on each occurrence, CR¹;
Ar is an aromatic or heteroaromatic ring system having 5 to 24 aromatic ring atoms, which is built up from in each case one or more of the groups benzene, pyridine, pyrimidine, pyridazine, pyrazine, pyrrole, thiophene, furan, naphthalene, triphenylene, quinoline, isoquinoline, quinoxaline, indole, benzothiophene or benzofuran, preferably from in each case one or more of the groups benzene, pyridine, pyrimidine, pyridazine, pyrazine or triazine, in particular benzene;
q is 0;
n is 1 or 2;
R¹ is selected, identically or differently on each occurrence, from the group consisting of H, D, F, CN, a straight-chain alkyl group having 1 to 10 C atoms, preferably having 1 to 4 C atoms, or a branched or cyclic alkyl group having 3 to 10 C atoms, particularly preferably having 3 to 5 C atoms, or an alkenyl group having 2 to 10 C atoms, preferably having 2 to 4 C atoms, each of which may be substituted by one or more radicals R³, where one or more H atoms may be replaced by D, an aromatic or heteroaromatic ring system having 5 to 12 aromatic ring atoms, which may in each case be substituted by one or more radicals R³, or a combination of these systems, where two or more adjacent substituents R¹ may optionally form a monocyclic or polycyclic, aliphatic, aromatic or heteroaromatic ring system, which may be substituted by one or more radicals R³;
R² is selected on each occurrence, identically or differently, from the group consisting of an aromatic or heteroaromatic ring system having 5 to 24 aromatic ring atoms, which may in each case be substituted by one or more radicals R³;
the other symbols and indices used have the meanings indicated in Claim 1.

9. Electronic device according to one or more of Claims 1 to 8, selected from the group consisting of organic electroluminescent devices (OLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and "organic plasmon emitting devices".

10. Organic electroluminescent device according to one or more of Claims 1 to 9, **characterised in that** the compound of the formula (1) or formulae (8) to (18) is employed as matrix material for fluorescent or phosphorescent emitters, in particular for phosphorescent emitters, and/or as hole-blocking material in a hole-blocking layer and/or as electron-transport material in an electron-transport layer or an electron-injection layer and/or as electron-blocking material or exciton-blocking material in an electron-blocking or exciton-blocking layer and/or as hole-transport material in a hole-transport layer or hole-injection layer.

11. Organic electroluminescent device according to Claim 10, **characterised in that** the phosphorescent emitter employed is a compound which contains copper, molybdenum, tungsten, rhenium, ruthenium, osmium, rhodium, iridium, palladium, platinum, silver, gold or europium, in particular iridium or platinum.

12. Compound of the formula (1') 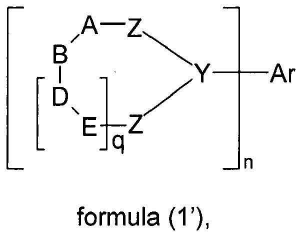 where the following applies to the symbols and indices used:
A-B and D-E are in each case, identically or differently on each occurrence, a unit of the following formula (2), (3), (4), (5) or (6), 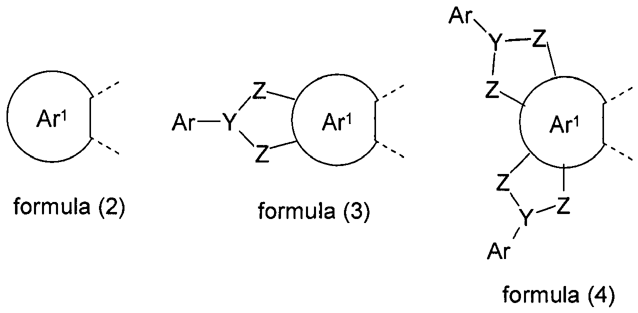 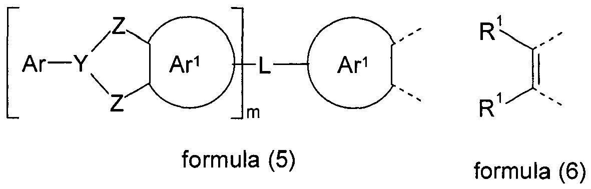 where the dashed bond in each case represents the bond to Z;
and
Z is, identically or differently on each occurrence, N-R², O or S, with the proviso that both groups Z bonded to the same group Y do not stand for O;
or
A-Z and B-Z are in each case, identically or differently on each occurrence, a unit of the following formula (7) and q = 0, 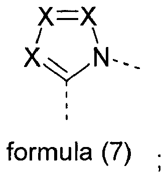 the dashed bond in formula (7) in each case represents the linking of this unit in the compound of the formula (1), where the nitrogen is linked to the group Y;
Y is on each occurrence, identically or differently, P(=O), As(=O), As(=S), Sb(=O), Sb(=S), Bi(=O) or Bi(=S);
Ar is on each occurrence, identically or differently, an aromatic or heteroaromatic ring system having 5 to 60 aromatic ring atoms, which may be substituted by one or more radicals R¹;
Ar¹ is on each occurrence, identically or differently, an aryl or heteroaryl group having 5 to 16 aromatic ring atoms, which may be substituted by one or more radicals R¹;
X is on each occurrence, identically or differently, CR¹ or N;
L is a single bond or a divalent, trivalent or tetravalent group;
R¹ is selected on each occurrence, identically or differently, from the group consisting of H, D, F, Cl, Br, I, CN, NO₂, N(R³)₂, C(=O)R³, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, each of which may be substituted by one or more radicals R³, where one or more non-adjacent CH₂ groups may be replaced by R³C=CR³, C≡C, Si(R³)₂, Ge(R³)₂, Sn(R³)₂, C=O, C=S, C=Se, C=NR³, P(=O)(R³), SO, SO₂, NR³, O, S or CONR³ and where one or more H atoms may be replaced by D, F, Cl, Br, I, CN or NO₂, an aromatic or heteroaromatic ring system having 5 to 60 aromatic ring atoms, which may in each case be substituted by one or more radicals R³, an aryloxy or heteroaryloxy group having 5 to 60 aromatic ring atoms, which may be substituted by one or more radicals R³, or a combination of these systems, where two or more adjacent substituents R¹ may optionally form a monocyclic or polycyclic, aliphatic, aromatic or heteroaromatic ring system, which may be substituted by one or more radicals R³;
R² is selected on each occurrence, identically or differently, from the group consisting of a straight-chain alkyl group having 1 to 40 C atoms or a branched or cyclic alkyl group having 3 to 40 C atoms, each of which may be substituted by one or more radicals R³, where one or more non-adjacent CH₂ groups may be replaced by R³C=CR³, C=C or C=O and where one or more H atoms may be replaced by D, F, Cl, Br, I, CN or NO₂, an aromatic or heteroaromatic ring system having 5 to 60 aromatic ring atoms, which may in each case be substituted by one or more radicals R³, or a combination of these systems; R¹ and R² which are adjacent to one another in the 1,2-position may optionally form a monocyclic or polycyclic, aliphatic, aromatic or heteroaromatic ring system, which may be substituted by one or more radicals R³;
R³ is selected from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN, where two or more adjacent substituents R³ may form a mono- or polycyclic, aliphatic, aromatic or heteroaromatic ring system with one another;
n is 1 to 10, preferably 1, 2, 3, 4, 5 or 6;
m is 1 if L is a single bond or a divalent group, or is 2 if L is a trivalent group, or is 3 if L is a tetravalent group;
q is on each occurrence, identically or differently, 0 or 1;
the following compounds are excluded from the invention: 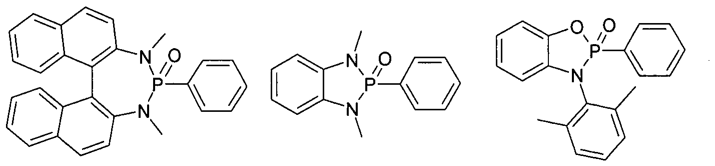 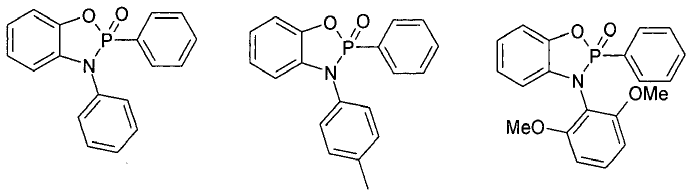 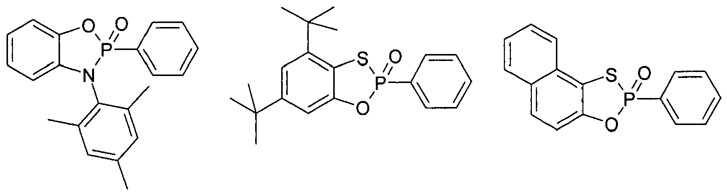 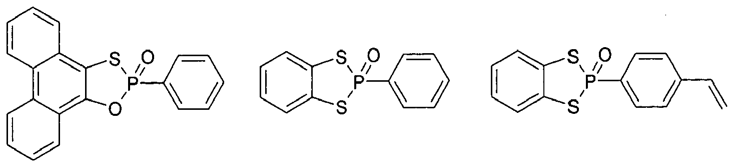 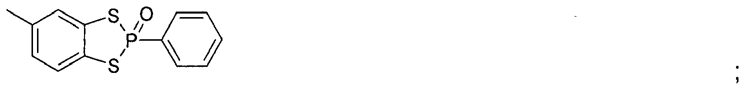 or compound of the formula (39), 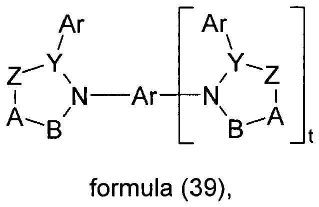 where the symbols and indices used have the meanings indicated in Claim 1.

13. Use of a compound according to Claim 12 in an electronic device.

14. Process for the preparation of a compound according to Claim 12 by reaction of an ortho-diamino-substituted aromatic compound, in which the amino groups are unsubstituted or preferably monosubstituted, with an aromatic phosphonyl chloride derivative or an aromatic oligo-phosphonyl chloride derivative.

15. Oligomer, polymer or dendrimer containing one or more compounds according to Claim 12, where one or more bonds are present from the compound to the polymer, oligomer or dendrimer,
or an oligomer or polymer of the formula (37) or formula (38), 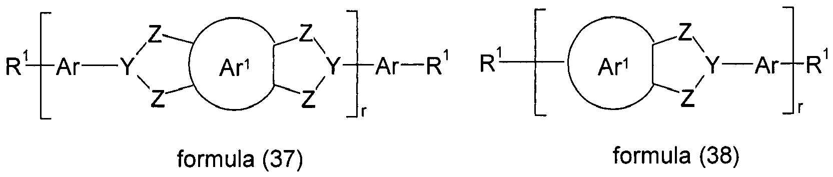 where the symbols used have the meanings indicated in Claim 11, and r stands for an integer between 2 and 1,000,000.

## Revendications

1. Dispositif électronique comprenant au moins un composé de la formule (1) ou de la formule (39) 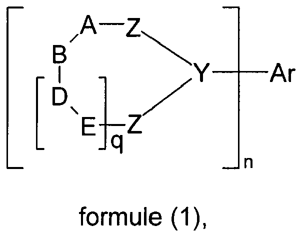 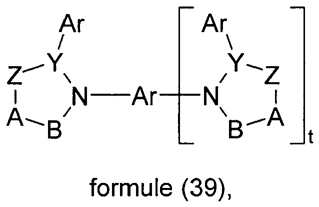 où ce qui suit s'applique aux symboles et indices utilisés :
A-B et D-E sont, dans chaque cas de manière identique ou différente pour chaque occurrence, une unité de la formule qui suit (2), (3), (4), (5) ou (6), 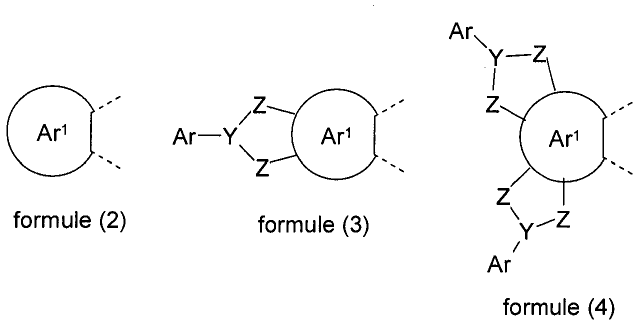 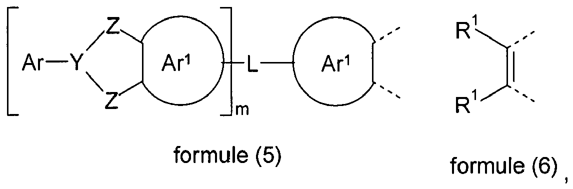 où le lien en pointillés représente dans chaque cas la liaison sur Z;
et
Z est, de manière identique ou différente pour chaque occurrence, N-R², O ou S ;
ou
A-Z et B-Z sont, dans chaque cas de manière identique ou différente pour chaque occurrence, une unité de la formule qui suit (7) et q = 0, 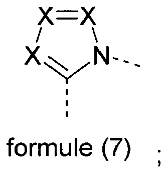 le lien en pointillés représente ici la liaison de cette unité dans le composé de la formule (1), où l'azote est lié au groupe Y ;
Y est, pour chaque occurrence de manière identique ou différente, P(=O), P(=S), P, As(=O), As(=S), As, Sb(=O), Sb(=S), Sb, Bi(=O), Bi(=S) ou Bi ;
Ar est, pour chaque occurrence de manière identique ou différente, un système de cycle aromatique ou hétéroaromatique comportant de 5 à 60 atomes de cycle aromatique, lequel peut être substitué par un ou plusieurs radicaux R¹ ;
Ar¹ est, pour chaque occurrence de manière identique ou différente, un groupe aryle ou hétéroaryle comportant de 5 à 18 atomes de cycle aromatique, lequel peut être substitué par un ou plusieurs radicaux R¹;
X est, pour chaque occurrence de manière identique ou différente, CR¹ ou N ;
L est une liaison simple ou un groupe divalent, trivalent ou tétravalent ;
R¹ est choisi, pour chaque occurrence de manière identique ou différente, parmi le groupe constitué par H, D, F, Cl, Br, I, CN, NO₂, N(R³)₂, C(=O)R³, un groupe alkyle, alcoxy ou thioalkyle en chaîne droite comportant de 1 à 40 atomes de C ou un groupe alkyle, alcoxy ou thioalkyle ramifié ou cyclique comportant de 3 à 40 atomes de C ou un groupe alkényle ou alkynyle comportant de 2 à 40 atomes de C, dont chacun peut être substitué par un ou plusieurs radicaux R³, où un ou plusieurs groupes CH₂ non adjacents peut/peuvent être remplacé(s) par R³C=CR³, C≡C, Si(R³)₂, Ge(R³)₂, Sn(R³)₂, C=O, C=S, C=Se, C=NR³, P(=O)(R³), SO, SO₂, NR³, O, S ou CONR³ et un ou plusieurs atomes de H peut/peuvent être remplacé(s) par D, F, Cl, Br, I, CN ou NO₂, un système de cycle aromatique ou hétéroaromatique comportant de 5 à 60 atomes de cycle aromatique, lequel peut dans chaque cas être substitué par un ou plusieurs radicaux R³, un groupe aryloxy ou hétéroaryloxy comportant de 5 à 60 atomes de cycle aromatique, lequel peut être substitué par un ou plusieurs radicaux R³, ou une combinaison de ces systèmes, où deux substituants R¹ adjacents ou plus peuvent en option former un système de cycle aliphatique, aromatique ou hétéroaromatique, monocyclique ou polycyclique, lequel peut être substitué par un ou plusieurs radicaux R³ ;
R² est choisi, pour chaque occurrence de manière identique ou différente, parmi le groupe constitué par un groupe alkyle en chaîne droite comportant de 1 à 40 atomes de C ou un groupe alkyle ramifié ou cyclique comportant de 3 à 40 atomes de C, dont chacun peut être substitué par un ou plusieurs radicaux R³, où un ou plusieurs groupes CH₂ non adjacents peut/peuvent être remplacé(s) par R³C=CR³, C=C ou C=O et où un ou plusieurs atomes de H peut/peuvent être remplacé(s) par D, F, Cl, Br, I, CN ou NO₂, un système de cycle aromatique ou hétéroaromatique comportant de 5 à 60 atomes de cycle aromatique, lequel peut dans chaque cas être substitué par un ou plusieurs radicaux R³, ou une combinaison de ces systèmes ; R¹ et R² qui sont adjacents l'un à l'autre à la position 1,2 peuvent en option former un système de cycle aliphatique, aromatique ou hétéroaromatique, monocyclique ou polycyclique, lequel peut être substitué par un ou plusieurs radicaux R³ ;
R³ est choisi parmi le groupe constitué par H, D, F, CN, un radical hydrocarbone aliphatique comportant de 1 à 20 atomes de C, un système de cycle aromatique ou hétéroaromatique comportant de 5 à 30 atomes de cycle aromatique, où un ou plusieurs atomes de H peut/peuvent être remplacé(s) par D, F, Cl, Br, I ou CN, où deux substituants R³ adjacents ou plus peuvent former un système de cycle aliphatique, aromatique ou hétéroaromatique, mono- ou polycyclique l'un avec l'autre ou les uns avec les autres ;
n, t sont 1 à 10, de préférence 1, 2, 3, 4, 5 ou 6 ;
m est 1 si L est une liaison simple ou un groupe divalent, ou est 2 si L est un groupe trivalent, ou est 3 si L est un groupe tétravalent ;
q est, pour chaque occurrence de manière identique ou différente, 0 ou 1.

2. Dispositif électronique selon la revendication 1, **caractérisé en ce que** Ar¹ représente, de manière identique ou différente pour chaque occurrence, un groupe aryle ou hétéroaryle comportant de 5 à 14 atomes de cycle aromatique, de préférence comportant de 5 à 10 atomes de cycle aromatique, de façon particulièrement préférable, benzène, pyridine, pyrimidine, pyridazine, pyrazine, triazine, furane, thiophène, pyrrole, naphtalène, phénanthrène, quinoline, isoquinoline, quinoxaline, indole, benzofurane, benzothiophène ou carbazole.

3. Dispositif électronique selon la revendication 1 ou 2, **caractérisé en ce que** le composé de la formule (1) est choisi parmi des composés des formules (8) à (18), 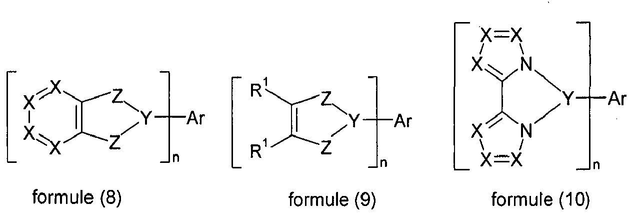 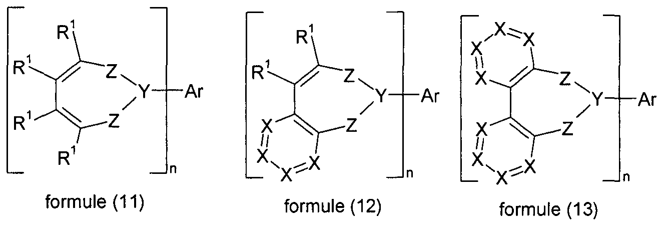 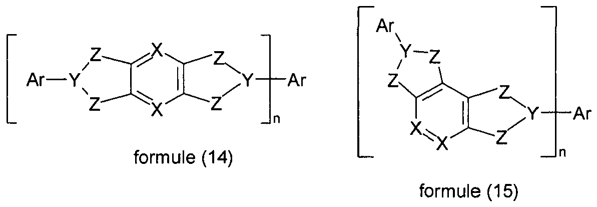 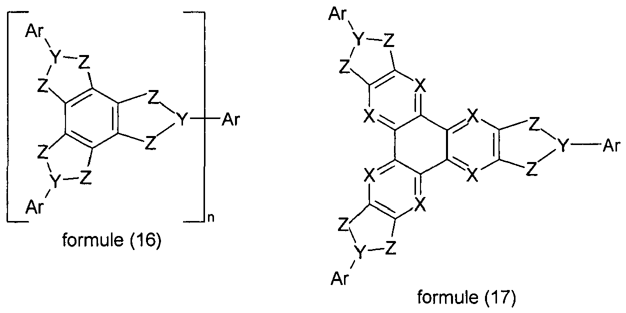 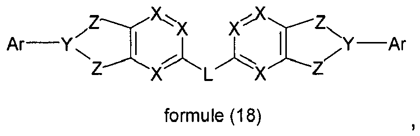 où les symboles et indices utilisés présentent les significations indiquées selon la revendication 1.

4. Dispositif électronique selon une ou plusieurs des revendications 1 à 3, **caractérisé en ce que** le symbole Y représente P(=O) et le symbole Z représente, pour chaque occurrence de manière identique ou différente, N-R².

5. Dispositif électronique selon une ou plusieurs des revendications 1 à 4, **caractérisé en ce que** le groupe Ar représente un système de cycle aromatique ou hétéroaromatique comportant de 5 à 30 atomes de cycle aromatique, de préférence choisi parmi le groupe constitué par les unités des formules qui suivent (19) à (36), où le lien en pointillés dans chaque cas indique une liaison sur le groupe Y dans la formule (1) ou N dans la formule (39) :

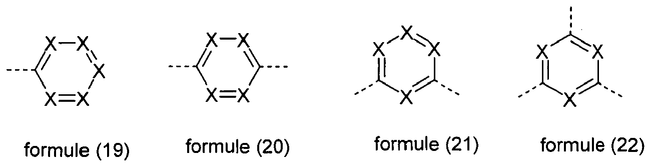

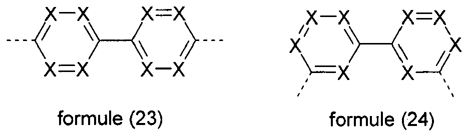

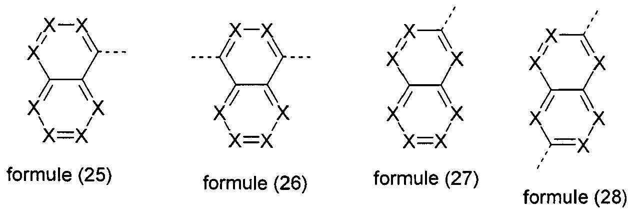

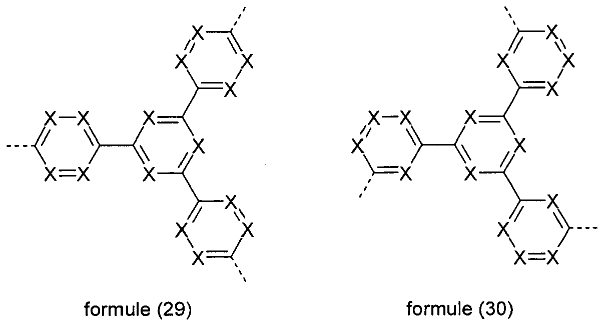

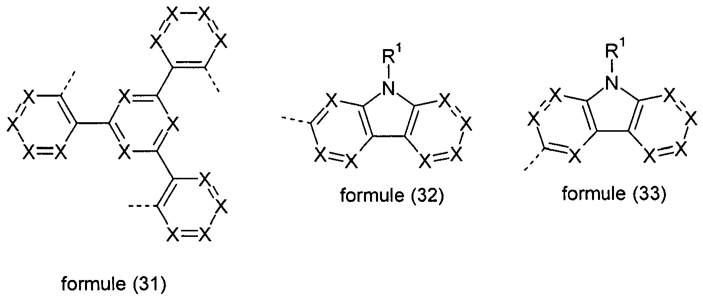

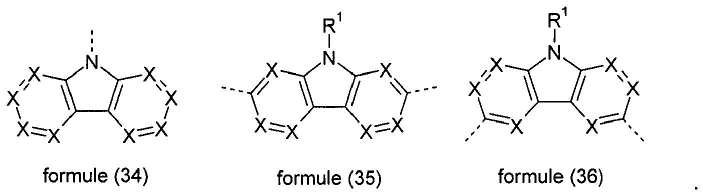

6. Dispositif électronique selon une ou plusieurs des revendications 1 à 5, **caractérisé en ce que** R² est choisi, de manière identique ou différente pour chaque occurrence, parmi le groupe constitué par un système de cycle aromatique ou hétéroaromatique comportant de 5 à 24 atomes de cycle aromatique, lequel peut dans chaque cas être substitué par un ou plusieurs radicaux R³, de préférence phényle, naphtyle, biphényle ou terphényle, dont chacun peut être substitué par un ou plusieurs radicaux R³.

7. Dispositif électronique selon une ou plusieurs des revendications 1 à 6, **caractérisé en ce que** ce qui suit s'applique aux symboles et indices :
Ar¹ est, de manière identique ou différente pour chaque occurrence, un groupe aryle ou hétéroaryle comportant de 5 à 14 atomes de cycle aromatique, de préférence comportant de 5 à 10 atomes de cycle aromatique, de façon particulièrement préférable comportant 6 atomes de cycle aromatique ;
Y est, pour chaque occurrence de manière identique ou différente, P(=O) ou P(=S) ;
Z est, de manière identique ou différente pour chaque occurrence, N-R² s'il ne forme pas un cycle de la formule (7) avec A ou B ;
X représente CR¹ ou N, où un maximum de deux symboles X représentent N, de préférence un maximum d'un symbole X représente N;
Ar est, de manière identique ou différente, un système de cycle aromatique ou hétéroaromatique comportant de 5 à 30 atomes de cycle aromatique, de préférence comportant de 5 à 24 atomes de cycle aromatique ; de préférence, aucun des groupes aryle ou hétéroaryle du système de cycle aromatique ou hétéroaromatique ne contient plus de 10 atomes de cycle aromatique ;
q est 0 ;
n est 1, 2, 3 ou 4, de préférence 1, 2 ou 3 ;
R¹ est choisi, de manière identique ou différente pour chaque occurrence, parmi le groupe constitué par H, D, F, CN, N(R³)₂, un groupe alkyle ou alcoxy en chaîne droite comportant de 1 à 20 atomes de C ou un groupe alkyle ou alcoxy ramifié ou cyclique comportant de 3 à 20 atomes de C ou un groupe alkényle comportant de 2 à 20 atomes de C, dont chacun peut être substitué par un ou plusieurs radicaux R³, où un ou plusieurs groupes CH₂ non adjacents peut/peuvent être remplacé(s) par R³C=CR³ ou O et où un ou plusieurs atomes de H peut/peuvent être remplacé(s) par D ou F, un système de cycle aromatique ou hétéroaromatique comportant de 5 à 30 atomes de cycle aromatique, lequel peut dans chaque cas être substitué par un ou plusieurs radicaux R³, un groupe aryloxy ou hétéroaryloxy comportant de 5 à 30 atomes de cycle aromatique, lequel peut être substitué par un ou plusieurs radicaux R³, ou une combinaison de ces systèmes, où deux substituants R¹ adjacents ou plus peuvent en option former un système de cycle aliphatique, aromatique ou hétéroaromatique, monocyclique ou polycyclique, lequel peut être substitué par un ou plusieurs radicaux R³ ;
R² est choisi, pour chaque occurrence de manière identique ou différente, parmi le groupe constitué par un groupe alkyle en chaîne droite comportant de 1 à 10 atomes de C ou un groupe alkyle ramifié ou cyclique comportant de 3 à 10 atomes de C, dont chacun peut être substitué par un ou plusieurs radicaux R³, où un ou plusieurs atomes de H peut/peuvent être remplacé(s) par F ou D, un système de cycle aromatique ou hétéroaromatique comportant de 5 à 30 atomes de cycle aromatique, lequel peut dans chaque cas être substitué par un ou plusieurs radicaux R³, ou une combinaison de ces systèmes ; R¹ et R² qui sont adjacents l'un à l'autre à la position 1,2 peuvent en option former un système de cycle aliphatique, aromatique ou hétéroaromatique, monocyclique ou polycyclique, lequel peut être substitué par un ou plusieurs radicaux R³ ;
les autres symboles et indices présentent les significations indiquées selon la revendication 1.

8. Dispositif électronique selon une ou plusieurs des revendications 1 à 7, **caractérisé en ce que** ce qui suit s'applique aux symboles et indices utilisés :
Ar¹ est choisi, de manière identique ou différente pour chaque occurrence, parmi le groupe constitué par benzène, pyridine, pyrimidine, pyridazine, pyrazine, triazine, furane, thiophène, pyrrole, naphtalène, quinoline, isoquinoline, quinoxaline, indole, benzofurane, benzothiophène et carbazole ;
Y est P(=O) ;
Z est, de manière identique ou différente pour chaque occurrence, N-R² s'il ne forme pas un cycle de la formule (7) avec A ou B ;
X est, de manière identique ou différente pour chaque occurrence, CR¹;
Ar est un système de cycle aromatique ou hétéroaromatique comportant de 5 à 24 atomes de cycle aromatique, lequel est construit à partir, dans chaque cas, d'un ou de plusieurs des groupes benzène, pyridine, pyrimidine, pyridazine, pyrazine, pyrrole, thiophène, furane, naphtalène, triphenylène, quinoline, isoquinoline, quinoxaline, indole, benzothiophène ou benzofurane, de préférence à partir, dans chaque cas, d'un ou de plusieurs des groupes benzène, pyridine, pyrimidine, pyridazine, pyrazine ou triazine, en particulier benzène ;
q est 0 ;
n est 1 ou 2;
R¹ est choisi, de manière identique ou différente pour chaque occurrence, parmi le groupe constitué par H, D, F, CN, un groupe alkyle en chaîne droite comportant de 1 à 10 atomes de C, de préférence comportant de 1 à 4 atomes de C, ou un groupe alkyle ramifié ou cyclique comportant de 3 à 10 atomes de C, de façon particulièrement préférable, comportant de 3 à 5 atomes de C, ou un groupe alkényle comportant de 2 à 10 atomes de C, de préférence, comportant de 2 à 4 atomes de C, dont chacun peut être substitué par un ou plusieurs radicaux R³, où un ou plusieurs atomes de H peut/peuvent être remplacé(s) par D, un système de cycle aromatique ou hétéroaromatique comportant de 5 à 12 atomes de cycle aromatique, lequel peut, dans chaque cas, être substitué par un ou plusieurs radicaux R³, ou une combinaison de ces systèmes, où deux substituants R¹ adjacents ou plus peuvent en option former un système de cycle aliphatique, aromatique ou hétéroaromatique, monocyclique ou polycyclique, lequel peut être substitué par un ou plusieurs radicaux R³ ;
R² est choisi, pour chaque occurrence de manière identique ou différente, parmi le groupe constitué par un système de cycle aromatique ou hétéroaromatique comportant de 5 à 24 atomes de cycle aromatique, lequel peut, dans chaque cas, être substitué par un ou plusieurs radicaux R³ ;
les autres symboles et indices utilisés présentent les significations indiquées selon la revendication 1.

9. Dispositif électronique selon une ou plusieurs des revendications 1 à 8, choisi parmi le groupe constitué par des dispositifs électroluminescents organiques (OLED), des circuits intégrés organiques (O-IC), des transistors à effet de champ organiques (O-FET), des transistors à film mince organiques (O-TFT), des transistors émetteurs de lumière organiques (O-LET), des cellules solaires organiques (O-SC), des détecteurs optiques organiques, des photorécepteurs organiques, des dispositifs à extinction de champ organiques (O-FQD), des cellules électrochimiques émettrices de lumière (LEC), des diodes laser organiques (O-laser) et des "dispositifs émetteurs de plasmon organiques".

10. Dispositif électroluminescent organique selon une ou plusieurs des revendications 1 à 9, **caractérisé en ce que** le composé de la formule (1) ou des formules (8) à (18) est utilisé en tant que matériau de matrice pour des émetteurs fluorescents ou phosphorescents, en particulier pour des émetteurs phosphorescents, et/ou en tant que matériau de blocage de trous dans une couche de blocage de trous et/ou en tant que matériau de transport d'électrons dans une couche de transport d'électrons ou une couche d'injection d'électrons et/ou en tant que matériau de blocage d'électrons ou matériau de blocage d'excitons dans une couche de blocage d'électrons ou une couche de blocage d'excitons et/ou en tant que matériau de transport de trous dans une couche de transport de trous ou une couche d'injection de trous.

11. Dispositif électroluminescent organique selon la revendication 10, **caractérisé en ce que** l'émetteur phosphorescent utilisé est un composé qui contient du cuivre, du molybdène, du tungstène, du rhénium, du ruthénium, de l'osmium, du rhodium, de l'iridium, du palladium, du platine, de l'argent, de l'or ou de l'europium, en particulier de l'iridium ou du platine.

12. Composé de la formule (1') 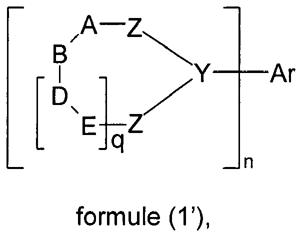 où ce qui suit s'applique aux symboles et indices utilisés :
A-B et D-E sont, dans chaque cas de manière identique ou différente pour chaque occurrence, une unité de la formule qui suit (2), (3), (4), (5) ou (6), 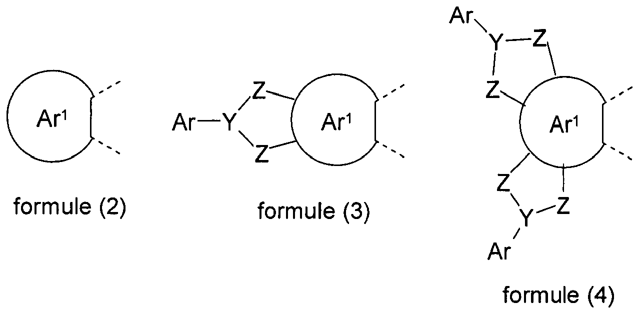 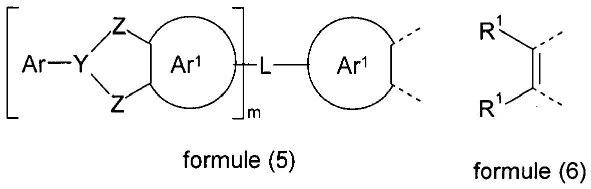 où le lien en pointillés représente dans chaque cas la liaison sur Z;
et
Z est, de manière identique ou différente pour chaque occurrence, N-R², O ou S, étant entendu que les deux groupes Z liés au même groupe Y ne représentent pas O ;
ou
A-Z et B-Z sont, dans chaque cas de manière identique ou différente pour chaque occurrence, une unité de la formule (7) qui suit et q = 0, 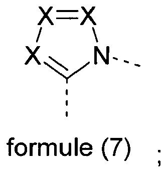 le lien en pointillés dans la formule (7) représente dans chaque cas la liaison de cette unité dans le composé de la formule (1), où l'azote est lié au groupe Y ;
Y est, pour chaque occurrence de manière identique ou différente, P(=O), As(=O), As(=S), Sb(=O), Sb(=S), Bi(=O) ou Bi(=S) ;
Ar est, pour chaque occurrence de manière identique ou différente, un système de cycle aromatique ou hétéroaromatique comportant de 5 à 60 atomes de cycle aromatique, lequel peut être substitué par un ou plusieurs radicaux R¹ ;
Ar¹ est, pour chaque occurrence de manière identique ou différente, un groupe aryle ou hétéroaryle comportant de 5 à 16 atomes de cycle aromatique, lequel peut être substitué par un ou plusieurs radicaux R¹ ;
X est, pour chaque occurrence de manière identique ou différente, CR¹ ou N ;
L est une liaison simple ou un groupe divalent, trivalent ou tétravalent ;
R¹ est choisi, pour chaque occurrence de manière identique ou différente, parmi le groupe constitué par H, D, F, Cl, Br, I, CN, NO₂, N(R³)₂, C(=O)R³, un groupe alkyle, alcoxy ou thioalkyle en chaîne droite comportant de 1 à 40 atomes de C ou un groupe alkyle, alcoxy ou thioalkyle ramifié ou cyclique comportant de 3 à 40 atomes de C ou un groupe alkényle ou alkynyle comportant de 2 à 40 atomes de C, dont chacun peut être substitué par un ou plusieurs radicaux R³, où un ou plusieurs groupes CH₂ non adjacents peut/peuvent être remplacé(s) par R³C=CR³, C≡C, Si(R³)₂, Ge(R³)₂, Sn(R³)₂, C=O, C=S, C=Se, C=NR³, P(=O)(R³), SO, SO₂, NR³, O, S ou CONR³ et où un ou plusieurs atomes de H peut/peuvent être remplacé(s) par D, F, Cl, Br, I, CN ou NO₂, un système de cycle aromatique ou hétéroaromatique comportant de 5 à 60 atomes de cycle aromatique, lequel peut, dans chaque cas, être substitué par un ou plusieurs radicaux R³, un groupe aryloxy ou hétéroaryloxy comportant de 5 à 60 atomes de cycle aromatique, lequel peut être substitué par un ou plusieurs radicaux R³, ou une combinaison de ces systèmes, où deux substituants R¹ adjacents ou plus peuvent en option former un système de cycle aliphatique, aromatique ou hétéroaromatique, monocyclique ou polycyclique, lequel peut être substitué par un ou plusieurs radicaux R³ ;
R² est choisi, pour chaque occurrence de manière identique ou différente, parmi le groupe constitué par un groupe alkyle en chaîne droite comportant de 1 à 40 atomes de C ou un groupe alkyle ramifié ou cyclique comportant de 3 à 40 atomes de C, dont chacun peut être substitué par un ou plusieurs radicaux R³, où un ou plusieurs groupes CH₂ non adjacents peut/peuvent être remplacé(s) par R³C=CR³, C≡C ou C=O et où un ou plusieurs atomes de H peut/peuvent être remplacé(s) par D, F, Cl, Br, I, CN ou NO₂, un système de cycle aromatique ou hétéroaromatique comportant de 5 à 60 atomes de cycle aromatique, lequel peut, dans chaque cas, être substitué par un ou plusieurs radicaux R³, ou une combinaison de ces systèmes ; R¹ et R² qui sont adjacents l'un à l'autre à la position 1,2 peuvent en option former un système de cycle aliphatique, aromatique ou hétéroaromatique, monocyclique ou polycyclique, lequel peut être substitué par un ou plusieurs radicaux R³ ;
R³ est choisi parmi le groupe constitué par H, D, F, CN, un radical hydrocarbone aliphatique comportant de 1 à 20 atomes de C, un système de cycle aromatique ou hétéroaromatique comportant de 5 à 30 atomes de cycle aromatique, où un ou plusieurs atomes de H peut/peuvent être remplacé(s) par D, F, Cl, Br, I ou CN, où deux substituants R³ adjacents ou plus peuvent former un système de cycle aliphatique, aromatique ou hétéroaromatique, mono- ou polycyclique l'un avec l'autre ou les uns avec les autres ;
n est 1 à 10, de préférence 1, 2, 3, 4, 5 ou 6 ;
m est 1 si L est une liaison simple ou un groupe divalent, ou est 2 si L est un groupe trivalent, ou est 3 si L est un groupe tétravalent ;
q est, pour chaque occurrence de manière identique ou différente, 0 ou 1;
les composés qui suivent sont exclus de l'invention : 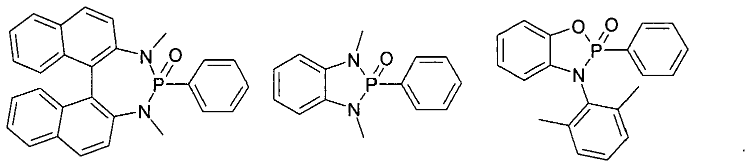 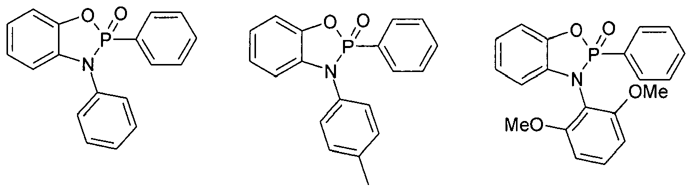 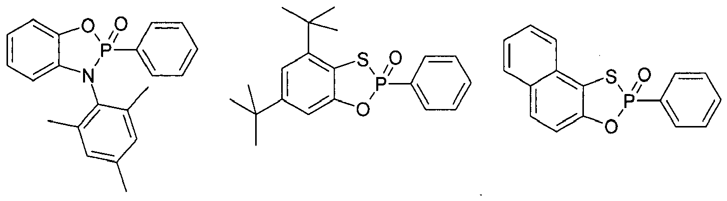 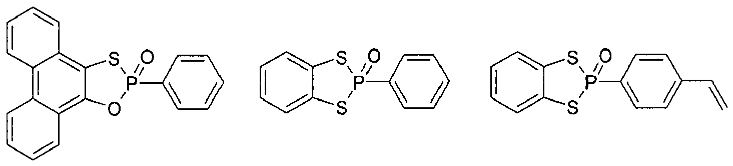 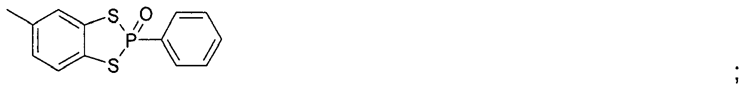 ou un composé de la formule (39), 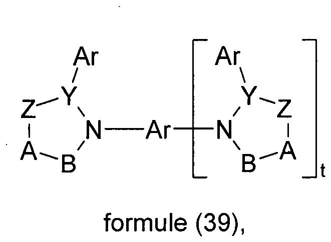 où les symboles et indices utilisés présentent les significations indiquées selon la revendication 1.

13. Utilisation d'un composé selon la revendication 12 dans un dispositif électronique.

14. Procédé pour la préparation d'un composé selon la revendication 12 par réaction d'un composé aromatique ortho-diamino-substitué, où les groupes amino sont non substitués ou de préférence monosubstitués, avec un dérivé de chlorure de phosphonyle aromatique ou un dérivé de chlorure d'oligophosphonyle aromatique.

15. Oligomère, polymère ou dendrimère contenant un ou plusieurs composés selon la revendication 12, où une ou plusieurs liaisons est/sont présente(s) depuis le composé jusqu'au polymère, oligomère ou dendrimère,
ou un oligomère ou polymère de la formule (37) ou de la formule (38), 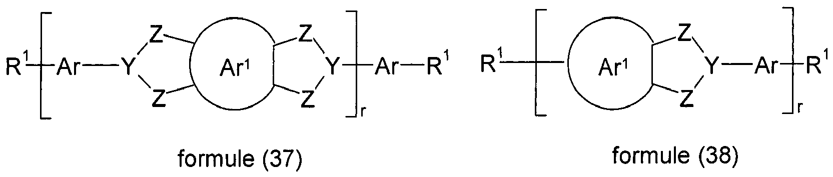 où les symboles utilisés présentent les significations indiquées selon la revendication 11, et r représente un entier entre 2 et 1 000 000.
